# EUROPÄISCHE PATENTSCHRIFT

(11) **EP 2 857 842 B1**
(45) Veröffentlichungstag und Bekanntmachung des Hinweises auf die Patenterteilung: **21.09.2022**
(21) Anmeldenummer: 13004812.7
(22) Anmeldetag: 07.10.2013
(51) Int. Cl.: G01N 35/00

(54) **Zugriffssteuerung für ein Laborgerät, Laborgerät mit Zugriffssteuerung und Verfahren zur gerätegesteuerten Behandlung von Laborproben**
Access control for a laboratory device, laboratory device with access control, and method for treatment of laboratory specimens controlled by devices
Commande d'accès pour un appareil de laboratoire, appareil de laboratoire avec commande d'accès et procédé de traitement d'échantillons de laboratoire commandé par un appareil

(43) Veröffentlichungstag der Anmeldung: 08.04.2015
(73) Patentinhaber: Eppendorf SE, 22339 Hamburg (DE)
(72) Erfinder: Goemann-Thoss, Wolfgang Dr., 22339 Hamburg (DE); Wente, Wolf Dr., 22339 Hamburg (DE); Thieme, Andreas, 22339 Hamburg (DE); Frerichs, Jan-Gerd Dr., 22339 Hamburg (DE); Markau, Christiane, 22339 Hamburg (DE); Hacker, Jan-Hendrik, 22339 Hamburg (DE)
(74) Vertreter: Ricker, Mathias

(56) Entgegenhaltungen:
- EP-A1- 1 981 245
- EP-A2- 2 182 364
- WO-A1-2009/085534
- US-A1- 2003 141 116
- US-A1- 2005 192 908
- US-A1- 2006 242 276
- US-A1- 2007 143 465
- US-A1- 2009 305 392
- US-A1- 2011 246 215
- Iris Poggendorf: "Einsatz eines Serviceroboters zur Automatisierung der Probenentnahme und des Probenmanagements während Kultivierungen tierischer Zellen in einer Technikumsumgebung", , 28 June 2004 (2004-06-28), XP055652656, Retrieved from the Internet: URL:https://pub.uni-bielefeld.de/download/ 2305348/2305351/ipoggendorf_diss.pdf [retrieved on 2019-12-13]

## Beschreibung

Die Erfindung bezieht sich auf ein Laborgerät mit Zugriffssteuerung zur gerätegesteuerten Behandlung mindestens einer Laborprobe, und ein Verfahren zur Steuerung des Zugriffs mittels der Zugriffssteuerung.

Solche Laborgeräte werden verwendet, um in chemischen, biologischen, biochemischen, medizinischen oder forensischen Laboratorien Laborproben, insbesondere flüssige Laborproben, mit hoher Effizienz zu bearbeiten. Solche Laborgeräte automatisieren Behandlungsschritte zumindest teilweise, die sonst manuell durchgeführt werden müssten, und steigern auf diese Weise die Geschwindigkeit, Präzision und Zuverlässigkeit dieser Behandlungen. Eine Behandlung von zumeist flüssigen Laborproben kann darauf gerichtet sein, diese Laborproben, insbesondere deren Zusammensetzung, physikalisch, chemisch, biochemisch oder auf andere Weise zu ändern oder zu untersuchen.

Die genannten Laborgeräte weisen eine oder mehrere Behandlungseinrichtung(en) zur gerätegesteuerten Behandlung der mindestens einen Laborprobe auf. Sie weisen oft eine Programmsteuerung auf, mittels der ein Benutzer des Laborgeräts die durchzuführende Behandlung durch Einstellen der gewünschten Programmparameter festlegen kann. Die Einstellung der Programmparameter erfolgt über eine Bedieneinheit des Laborgeräts, welche die Ein- und Ausgabe von Informationen, insbesondere von Werten der Programmparameter ermöglicht. Wenn das Laborgerät keine Behandlung durchführt, ist die Bedieneinheit für den Zugriff durch den Benutzer freigegeben. Läuft dagegen eine gerätegesteuerte Behandlung im Laborgerät ab, so wird die Bedieneinheit aus Sicherheitsgründen nicht ohne Rückfrage bzw. Bestätigung für den Benutzerzugriff freigegeben, um den sicheren Ablauf der Behandlung nicht zu gefährden. Die Behandlung wird zumindest teilweise automatisiert durchgeführt. Nach Ablauf der Behandlung kann der Benutzer die behandelte Probe weiterverwenden und das Laborgerät steht für eine weitere Verwendung zur Verfügung. Der folgende Benutzer des bekannten Laborgeräts wird erneut die gewünschte Einstellung der für die folgende Behandlung einstellbaren Programmparameter vornehmen, die Behandlung starten und nach Ablauf dieser Behandlung die behandelte Probe entnehmen und das Laborgerät für die weitere Verwendung zur Verfügung stellen. Um die Produktivität eines Labors zu steigern, besteht die Möglichkeit ein Labor mit mehreren Laborgeräten derselben Art auszustatten. Das Dokument US 2006/242276 A1 beschreibt ein System zur Fernkonfiguration von Vorrichtungen zur Anpassung eines Labornetzwerksystems. US 2007/143465 A1 beschreibt Systeme und Verfahren zur Verwaltung von Netzwerkgeräten und/oder Geräten eines physikalischen Levels. US 2005/192908 A1 beschreibt ein Verfahren zur benutzerabhängigen Kontrolle elektronischer Aufzeichnungen, die von einem Anwendungssoftwareprogramm für ein analytisches Laborgerät erzeugt werden. EP 1 981 245 A1 beschreibt ein System zur Steuerung von Laborgeräten über einen Host-Rechner mit einem Software-Dienst, der auf eingehende Nachrichten an einem TCP/IP-Port (2) hört. US 2003/141116 A1 beschreibt ein Messgerät, das mit Parameterwerten zur benutzerabhängigen Durchführung von Messaufgaben arbeitet. WO 2009/085534 A1 beschreibt ein Netzwerk von Steuergeräten zur Steuerung von Assay-Testsystemen, die mit einer Fernüberwachungseinheit zur Überwachung der Steuergeräte verbunden sind. Das Dokument "Poggendorf, Iris; Einsatz eines Serviceroboters zur Automatisierung der Probenentnahme und des Probenmanagements während Kultivierungen tierischer Zellen in einer Technikumsumgebung; 28.06.2004; XP055652656" beschreibt eine vollautomatisierte Laborumgebung zur 10 Probenbearbeitung, bei der ein Zellzählgerät via Fernsteuerung eingebunden wird, die durch Ausführung einer "Login"-Funktion ermöglicht wird, wobei die manuelle Nutzung des Geräts während der Fernsteuerung nicht möglich ist. US 2011/246215 A1 beschreibt ein Nachschubzentrum, das mit Analysesystemen verbunden ist, die jeweils nachfüllbare Artikel beobachten, mit einer Datenbank, einem Bestandsrechner, und einem Generator für Bestellvorschläge. US 2009/305392 A1 beschreibt eine Vorrichtung zur Probenaufbereitung mit vernetzten Modulen, davon eines zur Aufnahme oder Abgabe mindestens eines Probengefäßes, eines zum Transport eines Prozessgefäßes, eines zur Probenkonditionierung und eines zur Initiierung eines sequentiellen Prozesses für einen Analyten. EP 2 182 364 A2 beschreibt einen Probenanalysator mit erster und zweiter Messeinheit zum Messen einer Probe und einer Informationsverarbeitungseinheit zum Auswerten der Messungen und Erhalten von Analyseergebnisses, die mittels Korrekturwerten korrigiert werden.

Über derartige bekannte Ansätze hinaus verwendet die vorliegende Erfindung eine neue Ausgestaltung von Laborgeräten, um die Produktivität eines Labors weiter zu steigern.

Aufgabe der vorliegenden Erfindung ist es, ein Laborgerät mit Zugriffssteuerung und ein Verfahren zur gerätegesteuerten Behandlung mindestens einer Laborprobe zur Verfügung zu stellen, mit dem sich die Produktivität in einem Labor verbessern lässt.

Die Erfindung löst diese Aufgabe durch das Laborgerät gemäß Anspruch 1 und das Verfahren nach Anspruch 13. Bevorzugte Ausgestaltungen sind insbesondere Gegenstände der Unteransprüche.

Die Zugriffssteuerung erlaubt es bei einem erfindungsgemäßen Laborgerät, den Zugriff eines oder mehrerer weiterer Benutzer am Laborgerät zu steuern, während ein erster Benutzer bereits angemeldet ist und dessen Sitzung am Laborgerät noch läuft, also während des Zugriffversuchs des/der weiteren Benutzer. Durch diese Ausgestaltung lässt sich das Laborgerät effizienter nutzen und die Produktivität des Labors verbessern.

Bevorzugte Ausgestaltungen der Zugriffssteuerung und des Laborgeräts mit dieser Zugriffssteuerung werden im Rahmen der Beschreibung der vorliegenden Erfindung genannt oder lassen sich dort entnehmen.

Die Zugriffssteuerung ist dazu eingerichtet, während der Sitzung des ersten Benutzers die Anfrage des mindestens einen weiteren Benutzers nach einer Anmeldung an der Zugriffssteuerung, insbesondere den Zugriff auf mindestens eine Funktion des Laborgeräts zu steuern, insbesondere der Anfrage stattzugeben (Zugriff erlaubt) oder die Anfrage abzulehnen (Zugriff verweigert).

Die Zugriffssteuerung ist eine zur Datenverarbeitung ausgebildete Einrichtung, die vorliegend auch als "access control device" bezeichnet wird. Sie dient der Zugriffskontrolle (englisch: "access control"). Die Zugriffssteuerung weist eine Steuereinrichtung auf. Die Steuereinrichtung ist zur Datenverarbeitung ausgebildet. Die Steuereinrichtung ist insbesondere eine elektronische Steuereinrichtung. Sie weist vorzugsweise eine Datenverarbeitungseinrichtung auf, die insbesondere elektronisch ist.

Eine Steuereinrichtung weist im Rahmen der vorliegenden Erfindung generell insbesondere eine Datenverarbeitungseinrichtung, insbesondere eine Recheneinheit (CPU) zum Verarbeiten von Daten und/oder einen Mikroprozessor auf oder ist eine Datenverarbeitungseinrichtung. Eine Recheneinheit der Steuereinrichtung eines Laborgeräts ist vorzugsweise auch zum Steuern des Behandlungsprozesses und/oder der individuellen Behandlungen eingerichtet.

Der Begriff "Behandlung" bedeutet insbesondere, dass eine Laborprobe, die zumeist flüssig ist, bewegt, und/oder transportiert und/oder untersucht und/oder verändert wird, insbesondere in ihrer Zusammensetzung, physikalisch, chemisch, biochemisch oder auf andere Weise verändert wird.

Die Steuereinrichtung des Laborgeräts und/oder die Zugriffssteuerung und/oder die optionale Benutzerschnittstelleneinrichtung können - insbesondere alle - in einer physikalischen Geräteinheit integriert sein, können aber jeweils auch eine eigene physikalischen Geräteinheit sein. Eine physikalische Geräteinheit kann insbesondere ein Modul sein, das mit dem Laborgerät verbunden ist oder verbindbar ist. Die Steuereinrichtung des Laborgeräts und/oder die Zugriffssteuerung und/oder die optionale Benutzerschnittstelleneinrichtung oder Bestandteile dieser Komponenten können auch durch Softwarefunktionen implementiert sein oder können insbesondere als Programmcode vorliegen. Ein Laborgerät kann z.B. einen Computer aufweisen, der in Kombination mit Softwarefunktionen die Steuereinrichtung des Laborgeräts und/oder die optionale Zugriffssteuerung und/oder die optionale Benutzerschnittstelleneinrichtung jeweils zumindest teilweise implementiert. Ist z.B. die Zugriffssteuerung in das Laborgerät integriert, kann die Zugriffssteuerung selber Teil der Steuereinrichtung des Laborgeräts sein bzw. mittels der Steuereinrichtung implementiert sein, insbesondere durch Softwarefunktionen, insbesondere zumindest teilweise als ausführbarer Programmcode.

Ein Modul kann insbesondere die Zugriffssteuerung und/oder eine Benutzerschnittstelleneinrichtung aufweisen. Ein Modul ist ein von anderen Geräten getrenntes, und/oder ein vom anderen Gerät, insbesondere Laborgerät, abtrennbares Gerät. Ein Laborgerät kann eine Verbindungseinrichtung aufweisen, durch die das Modul mit dem Laborgerät verbindbar ist, insbesondere mittels einer vom Benutzer lösbaren Verbindung. Ein Modul kann portabel sein, also von einem Benutzer transportierbar. Das Modul kann auch fest mit dem Laborgerät verbunden sein. Die modulare Bauweise bietet Vorteile bei der Herstellung von Laborgeräten. Ein portables Modul bietet eine größere Flexibilität bei der Benutzung eines Laborgeräts.

Eine Kommunikationseinrichtung ist vorzugsweise eingerichtet für das Senden und/oder Empfangen von Daten, insbesondere für den Datenaustausch über eine durch die Kommunikationseinrichtung bereitgestellte Datenverbindung, insbesondere für eine Datenfernverbindung mit einem entfernten Gerät. Insbesondere wird das bezüglich eines Laborgeräts entfernt angeordnete Gerät auch bezeichnet als "Remote-Gerät" oder externes Gerät. Insbesondere wird eine Datenverarbeitungseinrichtung, die nicht Bestandteil eines Laborgeräts ist, auch als externe Datenverarbeitungseinrichtung bezeichnet. Die Datenverbindung, insbesondere Datenfernverbindung, kann über ein beschränktes (insbesondere ein Intranet) oder weltweites (insbesondere das Internet) Netzwerk aus Computern aufgebaut werden. Die Datenverbindung, insbesondere Datenfernverbindung, kann auch über eine Funkverbindung aufgebaut werden. Die Datenverbindung, insbesondere Datenfernverbindung, kann insbesondere über eine Mobilfunkverbindung aufgebaut werden.

Eine Datenverbindung verbindet insbesondere zwei datenverarbeitende Einheiten, insbesondere zwei Datenverarbeitungseinrichtungen, in der Weise, dass Daten zwischen den Einheiten ausgetauscht werden können, entweder unidirektional oder bidirektional. Die Datenverbindung kann leitungsgebunden realisiert sein oder drahtlos, insbesondere als Funkverbindung. Eine Datenfernverbindung verbindet insbesondere zwei datenverarbeitende Einheiten, insbesondere zwei Datenverarbeitungseinrichtungen, die entfernt voneinander angeordnet sind, die also insbesondere nicht Bestandteil desselben Geräts, insbesondere derselben Zugriffssteuerung, Benutzerschnittstelleneinrichtung oder desselben Laborgeräts sind, falls die genannten Geräte als separate Geräte ausgeführt sind. Eine Datenverbindung, insbesondere eine Datenfernverbindung, eines Geräts mit einem anderen Gerät wird vorzugsweise über eine direkte Verbindung der beiden Geräte realisiert, oder eine mittelbare Verbindung der beiden Geräte, so dass ein drittes Gerät zwischen die beiden Geräte geschaltet ist, um die Daten weiterzuvermitteln. Eine Datenfernverbindung kann insbesondere über ein Netzwerk aus Computern realisiert sein, bei denen die über die Datenfernverbindung verbundenen Geräte über das Netzwerk verbunden sind. Das Netzwerk kann ein beschränktes Netzwerk sein, z.B. ein Intranet, oder kann ein weltweites Netzwerk sein, insbesondere das Internet.

Die Datenverarbeitungseinrichtung weist vorzugsweise eine Recheneinheit auf, insbesondere eine CPU, ferner vorzugsweise mindestens eine Datenspeichereinrichtung, insbesondere zur flüchtigen und/oder dauerhaften Speicherung von Daten. Die Datenverarbeitungseinrichtung ist vorzugsweise dazu ausgebildet, über die erste Schnittstelleneinrichtung eine oder mehrere erste Datenverbindungen mit einer oder mehreren Benutzerschnittstelleneinrichtungen herzustellen, die insbesondere Bestandteile der Zugriffssteuerung oder des Laborgerätes sein können; vorzugsweise über die zweite Schnittstelleneinrichtung eine zweite Datenverbindung mit dem Laborgerät herzustellen; und vorzugsweise Zugriffsrechte für den Zugriff von Benutzern über die Benutzerschnittstelleneinrichtungen und die ersten und zweiten Datenverbindungen auf Funktionen des Laborgerätes zu steuern; wobei vorzugsweise die Zugriffsrechte so gesteuert werden können, dass ein gleichzeitiger Zugriff (eingeloggt sein) eines ersten und wenigstens eines weiteren Benutzers mit jeweils getrennt zugewiesenen Zugriffsrechten auf Funktionen des Laborgerätes, erfolgt.

Eine Schnittstelleneinrichtung dient der Verbindung von zwei Einrichtungen, die jeweils Signale, insbesondere Informationen, insbesondere Daten, verarbeiten können, insbesondere senden und/oder empfangen können. Eine Schnittstelleneinrichtung kann mindestens eine Hardwareschnittstelle beinhalten und/oder mindestens eine Softwareschnittstelle.

Hardwareschnittstellen sind insbesondere Schnittstellen zwischen elektrisch arbeitenden Einheiten, gemäß dem üblichen Verständnis in der Elektrotechnik und Elektronik. Vorliegend bezeichnet der Begriff "Hardwareschnittstelle" insbesondere auch die Verbindungskomponenten zwischen wenigstens zwei elektrisch arbeitenden Einheiten selbst, also insbesondere alle Bestandteile, die diese Verbindung ermöglichen, z.B. integrierte Schaltkreise, Elektronik und Leitungen, über die zwischen den wenigstens zwei elektrisch arbeitenden Einheiten elektrische Signale versandt werden. Diese zwei elektrisch arbeitenden Einheiten können insbesondere ein Laborgerät und eine externe Datenverarbeitungseinrichtung sein oder zwei Laborgeräte oder zwei elektrisch arbeitenden Einheiten innerhalb eines Laborgeräts. Eine Hardwareschnittstelle muss nicht, aber kann eine lösbare Verbindungseinrichtung zum Lösen und/oder Wiederherstellen dieser Verbindung aufweisen, insbesondere mindestens einen Stecker.

Softwareschnittstellen, insbesondere softwareseitige Datenschnittstellen, sind insbesondere logische Berührungspunkte in einem Informationsverwaltungssystem, insbesondere Softwaresystem: Sie ermöglichen und regeln den Austausch von Kommandos und Daten zwischen verschiedenen Prozessen und Komponenten. Softwareschnittstellen können nur zur Kommunikation benutzte, datenorientierte Schnittstellen sein. In diesem Fall enthält die Softwareschnittstelle lediglich die Informationen, die zwischen beteiligten Systemteilen ausgetauscht werden.

Die Zugriffssteuerung ist vorzugsweise dazu eingerichtet, die Zugriffsrechte zu steuern, indem die Steuereinrichtung eine Datenverbindung mit einer Datenbank für Zugriffsrechte verwendet. Die Datenbank für Zugriffsrechte ist vorzugsweise in mindestens einer, vorzugsweise in genau einer, Speichereinrichtung für Zugriffsrechte gespeichert. Die mindestens eine Speichereinrichtung für Zugriffsrechte kann in der Zugriffssteuerung angeordnet sein, und/oder kann in einer externen Datenverarbeitungseinrichtung angeordnet sein. Extern bedeutet, dass das Gerät, hier die Datenverarbeitungseinrichtung, nicht Bestandteil der in Rede stehenden Vorrichtung, hier der Zugriffssteuerung, ist. Die Datenbank für Zugriffsrechte kann zentral gespeichert sein, kann aber auch in mehreren Speichereinrichtungen gespeichert sein, die jeweils Teile der Daten der Datenbank aufweisen können oder eine Kopie der Daten der Datenbank aufweisen können.

Eine - insbesondere externe - Datenverarbeitungseinrichtung kann ein Computer sein, insbesondere ein Server, der insbesondere zur Herstellung einer Datenverbindung zu mehr als einer Zugriffssteuerung und/oder mehr als einem Laborgerät eingerichtet ist. Eine - insbesondere externe - Datenverarbeitungseinrichtung kann einen Computer oder Mikroprozessor aufweisen oder kann ein Computer oder Mikroprozessor sein. Ein Server ist insbesondere ein Computer, dessen Hardware vorzugsweise auf Serveranwendungen abgestimmt ist. Eine externe Datenverarbeitungseinrichtung kann eine mobile Datenverarbeitungseinrichtung sein, die zur Herstellung einer drahtlosen Datenverbindung eingerichtet ist, insbesondere einer Datenverbindung über ein begrenztes, insbesondere ein Intranet, oder weltweites Rechnernetz, insbesondere das Internet. Ein Rechnernetz ist ein Zusammenschluss verschiedener technischer, primär selbstständiger, elektronischer Systeme (insbesondere Computer, aber auch Sensoren, Aktoren, Agenten und/oder sonstige funktechnische Komponenten usw.), der die Kommunikation der einzelnen Systeme untereinander ermöglicht.

Die Zugriffssteuerung kann eine Kommunikationseinrichtung zur Herstellung einer Datenverbindung mit einer externen Datenverarbeitungseinrichtung, insbesondere über die erste, zweite oder eine andere Schnittstelleneinrichtung der Zugriffssteuerung aufweisen. Die Zugriffssteuerung ist vorzugsweise dazu ausgebildet, die Zugriffsrechte unter Verwendung der Datenverbindung zu der externen Datenverarbeitungseinrichtung herzustellen, insbesondere über die erste, zweite oder eine andere Schnittstelleneinrichtung der Zugriffssteuerung. Die externe Datenverarbeitungseinrichtung weist vorzugsweise die Datenbank für Zugriffsrechte zumindest teilweise oder vollständig auf.

Die Steuereinrichtung der Zugriffssteuerung ist dazu eingerichtet, Berechtigungen und/oder Zugriffsrechte für den Zugriff von Benutzern über die Benutzerschnittstelleneinrichtungen und die ersten und zweiten Datenverbindungen auf Funktionen des Laborgerätes zu steuern. Dadurch wird eine benutzerabhängige Nutzung des Laborgeräts möglich, die in Abhängigkeit von den jeweils vergebenen Zugriffsrechten gesteuert wird. Insbesondere wird eine gleichzeitige Nutzung des Laborgeräts durch mindestens einen ersten und mindestens einen zweiten Benutzer möglich.

Die Zugriffssteuerung ("access control device") führt die Zugriffskontrolle ("access control") aus. Der Begriff "Zugriffskontrolle" bezeichnet insbesondere Verfahren zur Verwaltung der Anfrage (englisch: "requests") nach Ressourcen und/oder Daten, die durch ein Informationsverwaltungssystem verwaltet werden, und zur Verwaltung der Entscheidungen, wie die Anfrage gehandhabt wird, insbesondere ob Zugriff gewährt wird oder nicht, und/oder in welcher Weise Zugriff gewährt wird oder nicht. Das Informationsverwaltungssystem kann insbesondere ein Betriebssystem sein, das auf der Zugriffssteuerung ausgeführt wird. Wenn der Benutzer eines Informationsverwaltungssystems eine bestimmte Operation auf einer bestimmten Ressource und/oder bestimmten Daten durchführen möchte, entscheidet die Zugriffskontrolle, ob diese Anfrage tatsächlich zugelassen oder abgelehnt werden soll. Eine Zugriffskontrollentscheidung (ja/nein) bezieht sich insbesondere auf ein Zugangssteuerungstriplett ("access control triple"), bestehend aus "Subjekt", "Objekt" und "Operation".

Als Subjekt wird insbesondere eine aktive Entität eines Systems bezeichnet, die eine bestimmte Operation auf einem bestimmten Objekt durchführen möchte. Eine Entität bezeichnet in diesem Zusammenhang eine eindeutig bestimmbare Einheit, über die Informationen gespeichert und/oder verarbeitet werden sollen. Die Einheit kann materiell oder immateriell, konkret oder abstrakt sein. Subjekte sind insbesondere menschliche Benutzer eines Informationsverwaltungssystems oder Computerprogramme, die von menschlichen Nutzern zur Erfüllung von Aufgaben eingesetzt werden. Ein Subjekt kann auch eine Gruppe von Nutzern sein, z.B. Laborarbeiter, Servicetechniker, Administrator. Die Gruppe fasst demnach mehrere individuelle Subjekte zusammen.

Ein Benutzer kann ein Individuum repräsentieren oder eine Gruppe von mehreren Individuen oder eine Klasse von Individuen, die gemäß einer Klassenregel oder Rollenregel ausgewählt wurden.

Die Zugriffssteuerung kann vorzugsweise den mindestens einen ersten Benutzer und den mindestens einen zweiten Benutzer voneinander unterscheiden. Ein Benutzer wird von der Zugriffssteuerung vorzugsweise eindeutig identifiziert. Dazu verarbeitet die Zugriffssteuerung vorzugsweise Identifizierungsdaten. Vorzugsweise ist die Zugriffssteuerung dazu ausgebildet, den anfragenden Benutzer zu authentifizieren, das heißt ein Nachweisverfahren durchzuführen, mit dem die Authentizität des anfragenden Benutzers überprüft wird und der Benutzer authentisiert wird, falls der Nachweis positiv ist. Authentifizierungsdaten enthalten zum Beispiel einen Login-Text und einen Passwort-Text oder einen Datensatz zur Gesichtserkennung oder zum Iris-Scan oder zum Fingerabdruckscan etc.. Die Authentifizierung kann ferner mittels RFID Chips oder NFC Chip oder über Gestenerkennung erfolgen. Eine Authentifizierung kann insbesondere per Direktzugriff auf das Laborgerät bzw. dessen Zugriffssteuerung vor Ort erfolgen oder per Fernzugriff.

Die Zugriffssteuerung weist vorzugsweise ein Informationsverwaltungssystem auf, mit dem die Zugriffskontrolle realisiert wird. Das Informationsverwaltungssystem ist vorzugsweise ein Betriebssystem eines Laborgeräts und/oder ein Betriebssystem der Zugriffssteuerung eines Laborgeräts, mit dem die Zugriffssteuerung und/oder das Laborgerät betrieben wird.

Die Zugriffssteuerung ist vorzugsweise dazu ausgebildet den anfragenden Benutzer, insbesondere mehrere anfragende Benutzer, insbesondere den mindestens einen ersten Benutzer und den mindestens einen zweiten Benutzer, an der Zugriffssteuerung, insbesondere am Informationsverwaltungssystem der Zugriffssteuerung anzumelden. Der Anmeldevorgang wird auch als Einloggen bezeichnet. Vorzugsweise erhält der erfolgreich angemeldete Benutzer vorbestimmte Berechtigungen und/oder Zugriffsrechte. Die Anmeldung kann vom Benutzer selber aufgehoben werden oder aufgrund anderer Bedingungen aufgehoben werden, z.B. durch das gerätegesteuerte Abmelden des Benutzers, insbesondere bei Überschreiten einer maximalen Anmeldezeit, in der der Benutzer ohne Unterbrechung über die Zugriffssteuerung eingeloggt war oder nach einer vorbestimmten Zeit der Inaktivität oder in Abhängigkeit vom Zeitpunkt des Endes einer vom Benutzer ausgeführten Behandlung oder aufgrund einer individuellen Methodenprogrammierung. Das Aufheben der Anmeldung bedeutet vorzugsweise, dass die bei der Anmeldung gewährte Autorisierung wieder entzogen wird.

Das Einloggen in das Informationsverwaltungssystem erfolgt vorzugsweise dadurch, dass der Benutzer authentifiziert wird. Nach erfolgter Authentifizierung erhält der Benutzer zum Einloggen einen personalisierten Zugang zu dem Informationsverwaltungssystem, mit Berechtigungen und/oder Zugriffsrechten, die mittels der Datenbank für Zugriffsrechte ermittelt werden. Mit dem Login beginnt eine Sitzung, die durch ein Logout, auch bezeichnet als Abmelden, beendet wird.

Die Zugriffssteuerung ist vorzugsweise dazu ausgebildet die Verwendung der Berechtigungen, Operationen und Objekte an dem Laborgerät, bzw. die Funktionen und Dienste des Laborgeräts, das die Zugriffssteuerung aufweist, in Abhängigkeit von den vorbestimmten Zugriffsrechten freizugeben, d.h. den authentifizierten Benutzer zu autorisieren. Die Zugriffssteuerung ist vorzugsweise Software-gesteuert, insbesondere programmgesteuert. Vorzugsweise wird als Anwendungsprotokoll bei der Implementierung der Softwarefunktionen LDAP eingesetzt (Lightweight Directory Access Protocol).

Als Objekt wird bei einem Zugriff bzw. bei einem Zugriffsversuch insbesondere eine passive Entität bezeichnet, auf der eine Operation durchgeführt werden soll. Objekte werden auch als "Ressourcen" bezeichnet. Objekte können z.B. sein: Daten oder Datensammlungen, d.h. Dateien, Datenobjekte in Datenbanken, z.B. Tabellen oder Spalten), Dienste oder Funktionen, insbesondere solche Dienste oder Funktionen, die mit der Zugriffssteuerung und/oder dem Laborgerät durchführbar sind. Solche Dienste können z.B. bezeichnen das Verfügbarmachen einer Kalenderdatenbank, wobei diese Verwendung die Anzeige von Kalenderdaten, die Lese- und/oder Schreibrechte an der Kalenderdatenbank vorsehen kann. Solche Dienste und Funktionen können z.B. bezeichnen eine Nachrichtenfunktion, mittels der Benutzern Nachrichten übersandt werden können, die insbesondere Informationen über die Verfügbarkeit des Laborgeräts in einem bestimmten Kalenderzeitraum enthalten können. Eine solche Funktion wäre insbesondere auch das Verfügbarmachen der Ausführung einer Behandlung, was insbesondere das Erteilen der dafür notwendigen Zugriffsrechte beinhalten kann. Eine Funktion kann z.B. das Einschalten einer UV-Beleuchtung des Laborgeräts sein oder das Öffnen einer Gehäusetüre eines Laborgerätegehäuses.

Als Operationen werden Vorgänge bezeichnet, die auf einem Objekt ausgeführt werden. Operationen können insbesondere Funktionen sein, insbesondere Funktionen der Zugriffssteuerung oder des Laborgeräts. Mehrere Funktionen können auf einem Objekt ausgeführt werden. Ist das Objekt eine Datei, sind mögliche Operationen das Schreiben, Lesen, Hinzufügen, Verändern, Kopieren oder Löschen von Daten. Ist das Objekt ein Dienst oder eine Funktion, so kann Ausführen die einzige mögliche Operation sein. Die Menge der möglichen Operationen hängt von der Art des Objekts ab. Die Mengen der Operationen, die von einzelnen Subjekten auf dem gleichen Objekt durchgeführt werden können, können unterschiedlich sein.

Ein bestimmtes Objekt in Kombination mit einer bestimmten Operation wird insbesondere als Berechtigung bezeichnet. Als "Leseberechtigung" lässt sich z.B. die Kombination aus der Operation "Lesen" mit dem Objekt "Datei" verstehen, als "Ausführungsberechtigung" lässt sich z.B. die Operation "Ausführen" mit dem Objekt "Funktion" verstehen.

Die Zugriffskontrolle kann insbesondere als Erlaubnisfunktion formuliert werden, formal beschrieben durch
*Erlaubnis_für(Subjekt, Objekt, Operation) → (ja, nein).*

Wird dieser Funktion das Triple an Parametern (Subjekt, Objekt, Operation) übergeben, so liefert die Erlaubnisfunktion entweder "ja" (Zugriff gewährt) oder "nein" (Zugriff verweigert) zurück.

Es ist auch möglich bei dieser Erlaubnisfunktion einen weiteren Eingabeparameter vorzusehen, der eine weitere Bedingung für die Zugriffsentscheidung liefert. Diese Bedingung kann z.B. den Zweck bezeichnen, zu dem ein bestimmter Zugriff erfolgen soll.

Ferner ist es möglich, dass die Erlaubnisfunktion nicht - oder nicht nur - die ja/nein-Entscheidung über die Zugriffserlaubnis zurückliefert, sondern eine Auflage (auch "Obligation") zurückliefert, in Abhängigkeit von der über die Zugriffserlaubnis entschieden wird. Dadurch kann insbesondere eine "Erlaubnis unter Vorbehalt" definiert werden. Eine solche Obligation ist insbesondere bereits vor dem Zugriff bzw. Zugriffsversuch erfüllt, kann aber auch während - oder nach - dem Zugriff oder der zu erlaubenden Operation erfüllt werden.

Die Zugriffskontrolle kann gemäß einem oder mehrerer spezieller Datenmodelle erfolgen. Ein solches spezielles Datenmodell ist insbesondere das Zugriffskontrollmodell (ZKM, Access Control Model (ACM)). Die Zugriffskontrolle kann insbesondere einen sogenannten Referenz-Monitor aufweisen. Diese Komponente ist insbesondere als funktioneller Kern der Zugriffssteuerung zu verstehen. Der Referenz-Monitor erfüllt die Funktion der Entscheidung, ob der von einem Subjekt gewünschte Zugriff auf ein Objekt gewährt wird. Vorzugsweise kann von der Zugriffssteuerung kein Zugriff auf eine Ressource des Laborgeräts freigegeben werden, ohne dass der Referenz-Monitor benutzt wird. Der Referenz-Monitor erfüllt vorzugsweise auch die Funktion, die erfolgten Zugriffsversuche aufzuzeichnen.

Die Datenbank über Zugriffsrechte enthält vorzugsweise Informationen in Form von Daten darüber, welche Operationen, insbesondere abhängig von einem bestimmten Zeitpunkt oder Zeitraum, für ein Objekt verfügbar sind. Dadurch kann insbesondere festgelegt werden, ob für einen Benutzer zu einem bestimmten Zeitpunkt und/oder in einem bestimmten Zeitraum der Zugriff auf die mindestens eine Behandlungseinrichtung erlaubt wird, insbesondere ob zu einem bestimmten Zeitpunkt und/oder in einem bestimmten Zeitraum das Recht vergeben wird eine Behandlung am Laborgerät zu starten oder zu ändern, wobei das Laborgerät mit der Zugriffssteuerung über die zweite Datenverbindung verbindbar ist und/oder verbunden ist.

Die Datenbank über Zugriffsrechte enthält vorzugsweise Informationen in Form von Daten darüber, welche Berechtigungen an den anfragenden Benutzer vergeben werden können, insbesondere in Abhängigkeit von möglichen Rechten aufgrund einer Gruppenzugehörigkeit und/oder Rollenzugehörigkeit.

Die Zugriffskontrolle ist vorzugsweise gemäß einem oder auch gemäß mehrerer der bekannten Grundformen DAC ("Discretionary Access Control"), MAC ("Mandatory Access Control") oder RBAC ("Role-Based Access Control") ausgestaltet, wobei RBAC besonders bevorzugt ist.

Das DAC-Modell wird auch als identitätsbasierte Zugriffskontrolle ("Identity Based Access Control") bezeichnet. Es wird die Identität des anfragenden Benutzers ausgewertet, um festzustellen welche Rechte der Benutzer erhalten darf und/oder welche Rechte ihm zugewiesen werden. Wenn die Zugriffskontrolle zumindest teilweise gemäß dem DAC-Modell ausgebildet ist, dann ist es möglich, dass ein vom Benutzer erstelltes Objekt immer mit bestimmten Rechten versehen wird, insbesondere Lese- und/oder Schreibrechte. Der erstellende Benutzer ist dann, wenigstens nach dem Erstellen des Objekts, dessen Eigentümer. Ein solches, zu erstellendes Objekt kann zum Beispiel eine Datei sein, die ein Methodenprogramm oder einen Satz von Parametern, insbesondere Programmparametern, repräsentiert. Es ist möglich und bevorzugt, dass die Zugriffskontrolle dazu ausgebildet ist, dass der Eigentümer eines Objektes Rechte an mindestens einen weiteren Benutzer vergeben kann, insbesondere Rechte über eine Operation auf dem Objekt, z.B. das Recht ein Methodenprogramm auszuführen. Es ist möglich und bevorzugt, dass die Zugriffskontrolle dazu ausgebildet ist, dass der Eigentümer eines Objektes die Eigentümerschaft an einen neuen Eigentümer oder einen Miteigentümerweitergeben kann. Ein erster Benutzer könnte z.B. einem zweiten Benutzer das Recht einräumen über ein vom ersten Benutzer erstelltes Methodenprogramm mit denselben Rechten zu verfügen wie der erste Benutzer. DAC kann insbesondere als sogenanntes Liberal DAC (Eigentümer darf die Eigentümerrechte weitergeben) oder Strict DAC (Eigentümer darf die Eigentümerrechte nicht weitergeben) ausgebildet sein. Beim Liberal DAC kann insbesondere eine Begrenzung der Anzahl der Rechteübertragungen vorgesehen sein, z.B. als One-Level-Grant, Two-Level-Grant oder Multi-Level-Grant.

Die Zugriffskontrolle kann, insbesondere gemäß einem DAC-ZKM Modell, eine Zugriffsmatrix aufweisen. Die Zugriffsmatrix ist eine Datentabelle, in der jede Zeile ein Subjekt repräsentiert und jede Spalte ein Objekt. Diese Datentabelle ist vorzugsweise in der Datenbank für Zugriffsrechte enthalten. Die Zugriffsmatrix kann pro Feld mindestens einen weiteren Datensatz enthalten, mittels dem die Rechte in diesem Feld anhand einer weiteren Bedingung differenziert werden, insbesondere gemäß einer Rolle des Benutzers. Jedes Subjekt-Objekt-Paar, also Element, in der Zugriffsmatrix enthält Informationen über die zulässigen Operationen, die das Subjekt am Objekt vornehmen darf. Das Element kann auch die Information über die Eigentümerschaft am Objekt anzeigen. Eine Zugriffsmatrix kann insbesondere als Zugriffskontroll-Liste ("access control list") gespeichert sein, in der für jedes Objekt aufgelistet ist, welches Subjekt welche Rechte am Objekt hat. Eine Zugriffsmatrix kann auch Capabilities-Listen aufweisen, in der für Subjekte der Zugriffsmatrix gespeichert ist, welche Operationen es auf welchen Objekten durchführen darf. Eine Capability-Liste kann ein Zertifikat für einen Benutzer bilden. Das ist insbesondere sinnvoll, wenn dem Benutzer ein Zertifikat verliehen werden soll, das ihm bestimmte Rechte am Laborgerät vorübergehend oder permanent zuweist. Vorzugsweise wird das Zertifikat verwendet, um den Benutzer zu qualifizieren, insbesondere nachdem der Benutzer am Laborgerät oder an einem externer Datenverarbeitungseinrichtung ein Qualifizierungsverfahren durchlaufen hat. Die Zugriffsmatrix kann ferner Autorisierungstabellen aufweisen, in denen Tupel aus Subjekt, Objekt und Operation enthalten sind. Die Zugriffsmatrix ist vorzugsweise durch die Zugriffssteuerung veränderbar.

Das RBAC-Modell sieht vor, dass einzelnen Subjekten nicht direkt Rechte zugewiesen werden, sondern indirekt über sogenannte "Rollen". Ein möglicher, im Rahmen der Gestaltung der Zugriffssteuerung anwendbarer, Standard des RBAC Modell ist detailliert beschrieben, im US Standard ANSI INCITS 359-2004. Die Zugriffssteuerung kann zumindest teilweise als RBAC-Modell ausgebildet sein, insbesondere zumindest teilweise gemäß dem genannten US Standard.

Vorzugsweise sieht die Zugriffskontrolle den Einsatz mindestens einer Rolle vor, vorzugsweise mehrerer Rollen, wobei insbesondere in der Rolle jeweils Rechte zusammengefasst sind. Die mindestens eine Rolle ist vorzugsweise in der Datenbank für Zugriffsrechte gespeichert. Eine Rolle ist insbesondere geeignet an eine Zuständigkeit oder eine Aufgabenbeschreibung im Rahmen des Einsatzes eines Laborgeräts angepasst, insbesondere innerhalb des Unternehmens, welches das Laborgerät einsetzt, und/oder beim Unternehmen, das einen Wartungsvertrag über das Laborgerät erfüllt, indem er z.B. Diagnosefunktionen am Laborgerät ausführt, und/oder beim Hersteller des Laborgeräts, der z.B. Firmware-Aktualisierungen, Kalibrierungen oder Informationen über das Laborgerät und/oder dessen Zubehör über die Zugriffssteuerung direkt an das Laborgerät übersendet. Solche Rollen können insbesondere Rechte zusammenfassen. Anstatt für jeden Benutzer einen Satz an Einzelrechten zu speichern, kann ihm mindestens eine Rolle zugeordnet werden. Die Rollenzuweisung ist in der Umsetzung besonders zuverlässig und erfordert relativ wenig Aufwand, insbesondere Verwaltungsaufwand beim Ermitteln und Speichern der Rechte.

Vorzugsweise sieht die Zugriffskontrolle mindestens zwei, vorzugsweise mehrere Rollen vor. Mögliche Rollen sind insbesondere Administrator ("Admin"), Wartungsdienst (Maintenance), normaler Labor-Benutzer ("LabUser"), unerfahrener Labor-Benutzer ("Inexperienced"), Manager. Durch solche Rollen wird eine sichere und effiziente Zugriffskontrolle ermöglicht. Die Benutzung eines mit der Zugriffssteuerung versehenen Laborgeräts ist sicher und effizient. Es wird auf einfache Weise verhindert, dass ein Benutzer z.B. wegen mangelnder Qualifikation bestimmte Operationen am Laborgerät durchführt, die gegebenenfalls zu Beschädigungen oder ineffizienter Nutzung des Laborgeräts bzw. zu Mehrkosten im Betrieb führen, z.B. durch überflüssigen Verbrauch an Verbrauchsmaterial, das für eine Behandlung verwendet wird.

Vorzugsweise sieht die Zugriffskontrolle mindestens eine Rolle oder mehr als eine Rolle vor, denen ein Benutzer gleichzeitig zugeordnet sein kann. Ein Individuum kann somit z.B. als Administrator oder als normaler Labor-Benutzer Zugriff erhalten, in Abhängigkeit von einer weiteren Bedingung. Vorzugsweise kann der Benutzer selber entscheiden, in welcher Rolle er am Laborgerät Zugriff erhält. Es ist aber auch möglich, dass der Benutzer dies nicht selber entscheidet, sondern dies die Zugriffssteuerung entscheidet. Diese Bedingung kann der verwendete Datensatz zur Authentifizierung sein, insbesondere das verwendete Passwort, oder kann von einem Parameter des Laborgeräts abhängen, insbesondere von einem Betriebsparameter des Laborgeräts, z.B. einem Betriebsparameter, der den Fehlerstatus des Laborgeräts kennzeichnet.

Das RBAC-Modell weist insbesondere vier Bestandteile auf: "Core RBAC" beinhaltet die grundlegende Rechtestruktur, auf der die andere Bestandteile aufbauen. "Core RBAC" besteht insbesondere aus den fünf Datenelementen Benutzer, Rollen, Objekte, Operationen und Berechtigungen. "Hierarchical RBAC" erweitert die in "Core RBAC" beschriebenen Rollen um eine Vererbungshierarchie. Vererbung bedeutet, dass einem Manager zusätzlich z.B. alle Rechte eines normalen Labor-Benutzers übertragen werden können. "Static Separation of Duty" kann Informationen darüber enthalten, dass einem Benutzer nie bestimmte Rollen gleichzeitig zugewiesen werden dürfen. "Dynamic Separation of Duty" kann Informationen darüber enthalten, dass ein Benutzer innerhalb einer Sitzung niemals bestimmte Rollen gleichzeitig verwenden darf.

Die vorliegend genannten bevorzugten Gestaltungen eines erfindungsgemäßen Laborgeräts gelten auch für ein Laborgerät, das Bestandteil eines Systems ist.

Die Steuereinrichtung der Zugriffssteuerung ist vorzugsweise dazu eingerichtet, dass mehr als ein Benutzer zeitgleich an der Zugriffssteuerung angemeldet sein kann, um Berechtigungen und/oder Zugriffsrechte zugewiesen zu bekommen. Eine solche Zugriffssteuerung zur gleichzeitigen Nutzung eines Laborgeräts stellt eine effiziente Lösung zur Steigerung der Produktivität in einem Labor dar.

Die Steuereinrichtung der Zugriffssteuerung ist dazu eingerichtet, während der Sitzung des ersten Benutzers mindestens einen weiteren, insbesondere zweiten, anfragenden Benutzer ebenfalls an der Zugriffssteuerung anzumelden und für diesen weiteren Benutzer eine Sitzung zu starten, die zumindest zeitweise parallel zu der Sitzung des ersten Benutzers stattfindet. Jedenfalls im Falle der Anmeldung des Benutzers an der Zugriffssteuerung, d.h. erfolgreichen Authentifizierung, ist es vorzugsweise vorgesehen, dem mindestens einen weiteren, insbesondere zweiten, anfragenden Benutzer während der Sitzung des (ersten) Benutzers Berechtigungen und/oder Zugriffsrechte zuzuweisen.

Die Begriffe "erster Benutzer" und "zweiter Benutzer" bezeichnen den Umstand, dass der erste Benutzer zeitlich vor dem Anmeldeversuch des zweiten Benutzers angemeldet wurde. Jedenfalls bei erfolgreichem Anmeldeversuch des zweiten Benutzers wird dieser ebenfalls angemeldet, und zwar noch während der Sitzung des ersten Benutzers. Es kann weitere an der Zugriffssteuerung angemeldete Benutzer geben, die zeitlich vor dem ersten oder dem zweiten Benutzer angemeldet wurden, oder nach deren Anmeldungen. Dem ersten Benutzer werden nach der Anmeldung vorzugsweise erste Berechtigungen und/oder erste Zugriffsrechte zugeordnet, vorzugsweise gemäß einem ersten Rechteprofil oder ersten Zertifikat. Dem zweiten Benutzer werden bei der Anmeldung vorzugsweise zweite Berechtigungen und/oder zweite Zugriffsrechte zugeordnet, vorzugsweise gemäß einem zweiten Rechteprofil oder zweiten Zertifikat. Vorzugsweise unterscheiden sich die ersten und zweiten Zugriffsrechte voneinander, vorzugsweise unterscheiden sich die ersten und zweiten Berechtigungen voneinander, vorzugsweise unterscheiden sich die ersten und zweiten Rechteprofile oder Zertifikate voneinander. Die Zugriffsrechte und Berechtigungen unterschiedlicher Benutzer können aber zumindest teilweise auch identisch sein.

Jeder Benutzer kann über dieselbe Benutzerschnittstelleneinrichtung in eine erste Datenverbindung mit der Zugriffssteuerung treten, oder mehrere Benutzer können über unterschiedliche Benutzerschnittstelleneinrichtungen in eine erste Datenverbindung mit der Zugriffssteuerung treten. Eine Benutzerschnittstelleneinrichtung kann Bestandteil der Zugriffssteuerung sein. Eine Zugriffssteuerung kann Bestandteil der Benutzerschnittstelleneinrichtung sein. Eine Benutzerschnittstelleneinrichtung kann Bestandteil eines Laborgeräts sein. Eine Benutzerschnittstelleneinrichtung weist jeweils vorzugsweise auf: eine Steuereinrichtung für eine Benutzerschnittstelleneinrichtung; eine Kommunikationseinrichtung zur Herstellung einer Datenverbindung mit einem Laborgerät über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer. Dabei ist die Steuereinrichtung der Benutzerschnittstelleneinrichtung vorzugsweise dazu eingerichtet, über die Datenverbindung Daten mit dem Laborgerät auszutauschen, die aus den Benutzereingaben gewonnen wurden, und die im erfindungsgemäßen Laborgerät bewirken, dass dem zweiten Benutzer am erfindungsgemäßen Laborgerät Berechtigungen und/oder Zugriffsrechte erteilt werden, so dass ein gleichzeitiges angemeldet-sein und/oder der gleichzeitige Zugriff eines ersten und wenigstens eines zweiten Benutzers am erfindungsgemäßen Laborgerät über die Schnittstelleneinrichtung mit jeweils zugewiesenen Zugriffsrechten auf Funktionen des Laborgerätes steuerbar ist.

Die Zugriffssteuerung kann Bestandteil des Laborgeräts sein, für das die Zugriffssteuerung die Berechtigungen und/oder Zugriffsrechte steuert. Die Zugriffssteuerung kann insbesondere Teil der Steuereinrichtung des Laborgeräts sein, indem sie z.B. zumindest teilweise als Programmcode von einer mikroprozessorbasierten Steuereinrichtung des Laborgeräts ausgeführt wird. Sie kann aber auch separat zu diesem Laborgerät vorgesehen sein und kann insbesondere ein Modul sein, das mit diesem Laborgerät verbindbar ist. Diese Zugriffssteuerung kann insbesondere ein mobiles Gerät sein, d.h. sie kann insbesondere von einem Menschen transportierbar sein.

Vorzugsweise ist in einer bevorzugten Gestaltung der Zugriffssteuerung, die als zweite Gestaltung bezeichnet wird, die Zugriffssteuerung oder die Steuereinrichtung der Zugriffssteuerung dazu eingerichtet, dem mindestens einen weiteren, anfragenden Benutzer jedenfalls nach dessen Anmeldung während der Sitzung des ersten Benutzers Berechtigungen und/oder Zugriffsrechte zuzuweisen.

Vorzugsweise ist in einer dritten bevorzugten Gestaltung der Zugriffssteuerung, die insbesondere die zweite Gestaltung aufweisen kann, die Zugriffssteuerung oder die Steuereinrichtung der Zugriffssteuerung dazu eingerichtet, dass die Zugriffsrechte und/oder Berechtigungen so gesteuert werden, d.h. insbesondere erteilt oder verweigert werden, dass ein gleichzeitiger Zugriff eines ersten und wenigstens eines weiteren Benutzers mit jeweils getrennt zugewiesenen Zugriffsrechten auf Funktionen des Laborgeräts, erfolgen kann. Die Steuereinrichtung der Zugriffssteuerung ist dazu eingerichtet Zugriffsrechte so zu steuern, dass mindestens ein erster Benutzer mindestens erste Zugriffsrechte erhält und mindestens ein zweiter Benutzer mindestens zweite Zugriffsrechte erhält, wobei sich insbesondere die mindestens ersten und die mindestens zweiten Zugriffsrechte unterscheiden können. Insbesondere ist die Steuereinrichtung der Zugriffssteuerung dazu eingerichtet, dass benutzerabhängige Zugriffsrechte gleichzeitig an eine Mehrzahl (N=2-10) oder Vielzahl (N>10) von Benutzern gleichzeitig vergeben sein können. Dadurch wird die Verwendung eines mit dieser Zugriffssteuerung versehenen Laborgeräts besonders effizient.

Die Zugriffssteuerung, insbesondere die Steuereinrichtung der Zugriffssteuerung, ist vorzugsweise dazu eingerichtet, dass die Zugriffsrechte so gesteuert werden, dass ein gleichzeitiger Zugriff eines ersten und wenigstens eines weiteren Benutzers mit jeweils getrennt zugewiesenen Zugriffsrechten auf Funktionen des Laborgeräts, erfolgen kann und insbesondere im Falle der zeitgleichen Anmeldung der Benutzer an der Zugriffssteuerung auch erfolgt.

Vorzugsweise ist in einer vierten bevorzugten Gestaltung der Zugriffssteuerung, die insbesondere die zweite oder dritte Gestaltung aufweisen kann, die Zugriffssteuerung oder die Steuereinrichtung der Zugriffssteuerung so eingerichtet, dass, wenn ein erster Benutzer eine oder mehrere Funktionen des Laborgeräts aktiviert hat, die Berechtigungen und/oder Zugriffsrechte jedes weiteren eingeloggten Benutzers so festgelegt werden, dass durch eine Aktivierung einer gemäß den Berechtigungen und/oder Zugriffsrechten des weiteren Benutzers zugelassenen Funktion die Ausführung einer bereits aktivierten Funktion des Laborgeräts nicht beeinflusst werden kann. Die vom ersten Benutzer aktivierte Funktion kann z.B. sein, dass eine Behandlung gestartet wurde, die insbesondere zu dem Zeitpunkt abläuft, zu dem der zweite Benutzer eine zweite Funktion aktiviert. Die Aktivierung der zweiten Funktion ist nur dann erlaubt, wenn diese zweite Funktion die erste Funktion nicht stört. Auf diese Weise kann verhindert werden, dass die Tätigkeit des ersten Benutzers durch Eingaben des zweiten Benutzers gestört werden.

Vorzugsweise ist die Zugriffssteuerung, insbesondere die Steuereinrichtung der Zugriffssteuerung, so eingerichtet, dass, wenn ein erster Benutzer angemeldet ist, die Zugriffsrechte jedes weiteren angemeldeten Benutzers so in Abhängigkeit von den Berechtigungen und/oder Zugriffsrechten des ersten Benutzers festgelegt werden, dass durch eine Aktivierung einer gemäß den Berechtigungen und/oder Zugriffsrechten des weiteren Benutzers zugelassenen Funktion des Laborgeräts eine potentielle Ausführung der gemäß den Berechtigungen und/oder Zugriffsrechten des ersten Benutzers zugelassenen Funktion des Laborgeräts nicht beeinflusst werden kann. Dadurch wird bereits anhand der Anmeldedaten und/oder des Zeitpunkts der Anmeldungen festgelegt und sichergestellt, dass die den beiden Benutzern jeweils zugewiesenen Berechtigungen und/oder Zugriffsrechte nicht zu einer einseitigen oder beidseitigen Störung der von den Benutzern aktivierten Funktionen des Laborgeräts führen, und insbesondere nicht zu einer Störung einer aktiven, d.h. ablaufenden, Behandlung führen.

Vorzugsweise ist in einer fünften bevorzugten Gestaltung der Zugriffssteuerung, die insbesondere die zweite, dritte oder vierte Gestaltung aufweisen kann, die Zugriffssteuerung oder die Steuereinrichtung der Zugriffssteuerung so eingerichtet, dass beim Vorliegen wenigstens einer Bedingung eine Umstellung der Berechtigungen und/oder Zugriffsrechte derart erfolgen kann, dass ein weiterer Benutzer anstelle des ersten Benutzers zumindest teilweise dessen Berechtigungen und/oder Zugriffsrechte erhält. Diese Bedingung kann z.B. sein, dass der weitere Benutzer einen höheren Rang aufweist, insbesondere wenn ein Administrator aus administrativen Gründen den Zugriff auf das Laborgerät benötigt. Die Bedingung kann auch sein, dass der erste Benutzer dem zweiten Benutzer erlaubt hat, z.B. durch Ändern eines Parameters der Zugriffssteuerung oder des Laborgeräts, die Berechtigungen und/oder Zugriffsrechte in der genannten Weise zumindest teilweise oder vollständig übergehen. Die Bedingung kann insbesondere voraussetzen, dass dem weiteren Benutzer mittels einer Kommunikationseinrichtung eine Nachricht übersandt wurde, mit der dem weiteren Benutzer das zumindest teilweise Erhalten der genannten Berechtigungen und/oder Zugriffsrechte ermöglicht wird. Dazu weist insbesondere die Zugriffssteuerung, und/oder das Laborgerät, und insbesondere die Benutzerschnittstelleneinrichtung oder die externe Datenverarbeitungsanlage des weiteren Benutzers eine Kommunikationseinrichtung auf. In dieser Weise kann der Zugriff auf die Funktionen des Laborgeräts weiter flexibel gestaltet werden.

Vorzugsweise ist in einer sechsten bevorzugten Gestaltung der Zugriffssteuerung, die insbesondere die fünfte Gestaltung aufweist, die Zugriffssteuerung oder die Steuereinrichtung der Zugriffssteuerung so eingerichtet, dass die Berechtigungen und/oder Zugriffsrechte, die der weitere Benutzer anstelle des ersten Benutzers erhält, das Recht zum Steuern der Behandlungseinrichtung beinhaltet. Dadurch wird die Verteilung der Ressource "Behandlungseinrichtung" flexibel gestaltet.

Vorzugsweise ist die Zugriffssteuerung, insbesondere die Steuereinrichtung der Zugriffssteuerung, so eingerichtet, dass die Berechtigung zur Steuerung der Behandlungseinrichtung, insbesondere in einem vorbestimmten Zeitraum, nur an einen einzigen Benutzer vergeben wird. Diese Berechtigung bezeichnet insbesondere das Starten, Ändern oder Stoppen einer Behandlung. Auf diese Weise ist weiter dafür gesorgt, dass keine Situation auftreten kann, in der eine laufende Behandlung durch einen weiteren Benutzer unbeabsichtigt geändert oder gestoppt wird.

Vorzugsweise ist in einer siebten bevorzugten Gestaltung der Zugriffssteuerung, die insbesondere die sechste Gestaltung aufweist, die Zugriffssteuerung oder die Steuereinrichtung der Zugriffssteuerung so eingerichtet, dass die gemäß den Berechtigungen und/oder Zugriffsrechten des ersten Benutzers ausgeführte Funktion die Behandlung der wenigstens einen Probe durch die Behandlungseinrichtung ist. Auf diese Weise ist weiter dafür gesorgt, dass keine Situation auftreten kann, in der eine laufende Behandlung durch einen weiteren Benutzer unbeabsichtigt geändert oder gestoppt wird.

Vorzugsweise ist in einer achten bevorzugten Gestaltung der Zugriffssteuerung, die insbesondere die zweite, dritte, vierte, fünfte, sechste oder siebte Gestaltung aufweisen kann, die Zugriffssteuerung oder die Steuereinrichtung der Zugriffssteuerung so eingerichtet, dass die Berechtigungen und/oder Zugriffsrechte des ersten Benutzers oder jedes weiteren Benutzers in Abhängigkeit vom Betriebszustand des Laborgeräts festgelegt werden. Dadurch wird eine noch flexiblere Nutzung des Laborgeräts ermöglicht.

Vorzugsweise ermöglicht die erste Schnittstelleneinrichtung das Anmelden und den Zugriff auf Funktionen des Laborgeräts über wenigstens zwei verschiedene Benutzerschnittstelleneinrichtungen.

Vorzugsweise ist in einer neunten bevorzugten Gestaltung der Zugriffssteuerung, die insbesondere die zweite, dritte, vierte, fünfte, sechste, siebte oder achte Gestaltung aufweisen kann, die Zugriffssteuerung oder die Steuereinrichtung der Zugriffssteuerung so eingerichtet, dass beim Anmelden über eine zweite Benutzerschnittstelleneinrichtung geprüft wird, ob der anmeldende Benutzer vorausgehend bereits über eine erste Benutzerschnittstelleneinrichtung
a) eine oder mehrere gerade ausgeführte Funktionen des Laborgeräts aktiviert hatte oder
b) schon angemeldet ist, und
wenn Bedingung a) oder b) erfüllt ist, dann werden die dem Benutzer beim vorausgehenden Anmelden über die erste Benutzerschnittstelleneinrichtung durch die Zugriffsteuerung zugewiesenen Berechtigungen und/oder Zugriffsrechte für den Zugriff auf das Laborgerät über die zweite Benutzerschnittstelleneinrichtung zugewiesen. Auf diese Weise kann der Benutzer ein Laborgerät mit mehreren Benutzerschnittstelleneinrichtungen verwenden, insbesondere kann er die Benutzerschnittstelleneinrichtung wechseln ohne die Verwendung des Laborgeräts unterbrechen zu müssen. Dies kann insbesondere hilfreich sein, um eine ablaufende Behandlung von unterschiedlichen Orten aus zu steuern oder zu beobachten oder um die Programmierung eines Methodenprogramms von unterschiedlichen Orten aus fortzusetzen. Ein Benutzer könnte insbesondere das Labor verlassen und die Verwendung des Laborgeräts von einer mobilen Benutzerschnittstelleneinrichtung aus weiter steuern und/oder beobachten.

Vorzugsweise ist in einer zehnten bevorzugten Gestaltung der Zugriffssteuerung, die insbesondere die neunte Gestaltung aufweisen kann, die Zugriffssteuerung oder die Steuereinrichtung der Zugriffssteuerung so eingerichtet, dass, insbesondere zusätzlich zu den Bedingungen a) oder b), geprüft wird, ob wenigstens eine weitere vorbestimmte Bedingung beim Anmelden an der zweiten Benutzerschnittstelleneinrichtung erfüllt ist, und die Zugriffsrechte für den Zugriff auf das Laborgerät über die zweite Benutzerschnittstelleneinrichtung nur dann zugewiesen werden, wenn auch die wenigstens eine weitere vorbestimmte Bedingung erfüllt ist. Diese weitere(n) Bedingung(en) kann (können) von der Verwendungssituation ("use case") abhängig sein.

Ähnliche Bedingungen können von der Zugriffssteuerung generell berücksichtigt werden, wenn über die Vergabe von Berechtigungen und/oder Zugriffsrechten eines anmeldenden oder angemeldeten Benutzers entschieden wird.

Mögliche Verwendungssituationen sind z.B., jeweils vorzugsweise, die Beobachtung des Laborgeräts über eine Datenfernverbindung ("remote monitoring"), die Steuerung des Laborgeräts über eine Datenfernverbindung ("remote control"), die Einbeziehung eines Buchungszeitplans zur zeitabhängigen Planung der Benutzung des Laborgeräts durch mehrere Nutzer ("booking schedule"), die Vorprogrammierung einer Behandlung, insbesondere einer programmgesteuerten Behandlung, insbesondere durch eine Methodenprogrammierung ("preprogramming"), oder den Fernzugriff eines Servicetechnikers ("remote service access" bzw. "Remote Service Zugriff"). Die Bedingung kann ferner die Rolle des Benutzers und/oder den Betriebszustand des Laborgeräts berücksichtigen. Der Betriebszustand des Laborgeräts kann insbesondere ein Zustand des Leerlaufs sein, also ein Zustand insbesondere ohne laufende Behandlung, in dem aber das Laborgerät insbesondere für die Anmeldung eines Benutzers und/oder die Durchführung einer Behandlung bereit sein kann. Der Betriebszustand des Laborgeräts kann insbesondere ein Zustand sein, in dem eine Behandlung programmiert wird oder wurde und/oder die Behandlung vorbereitet wurde und unmittelbar vor der Ausführung steht. Der Betriebszustand des Laborgeräts kann insbesondere ein Zustand sein, in dem eine Behandlung bereits gestartet wurde und läuft oder ein Zustand, in dem eine Behandlung gestoppt wurde oder ein Zustand, in dem der Buchungszeitplan einen Reservierungseintrag für die Behandlung durch einen Benutzer aufweist, wobei unterschieden werden kann, ob dieser Benutzer entweder angemeldet ist oder nicht. Der Betriebszustand des Laborgeräts kann insbesondere ein Energiesparzustand ("standby" Modus) des Laborgeräts sein. Weitere Beispiele der möglichen bzw. bevorzugten Ausführung solcher Berechtigungen in Abhängigkeit von den genannten Bedingungen finden sich im "Anhang 1" der Beschreibung.

Vorzugsweise ist die Zugriffssteuerung, insbesondere die Steuereinrichtung der Zugriffssteuerung, so eingerichtet, dass, insbesondere wenn eine weitere Bedingung erfüllt ist, über die Schnittstelleneinrichtung Informationen über den Betriebszustand des Laborgeräts, Messwerte oder durch Benutzer beeinflussbare Einstellungen oder Programmierungen des Laborgeräts an die zweite Benutzerschnittstelleneinrichtung übermittelt werden. Diese Bedingung kann insbesondere sein, dass ein Benutzer bei der Zugriffssteuerung diese Informationsübermittlung beantragt hat.

Vorzugsweise ist in einer elften bevorzugten Gestaltung der Zugriffssteuerung, die insbesondere die neunte oder zehnte Gestaltung aufweisen kann, die Zugriffssteuerung oder die Steuereinrichtung der Zugriffssteuerung so eingerichtet, dass, wenn a) oder b) erfüllt ist, über die Schnittstelleneinrichtung Informationen über den Betriebszustand des Laborgeräts, Messwerte oder durch Benutzer beeinflussbare Einstellungen oder Programmierungen des Laborgeräts an die zweite Benutzerschnittstelleneinrichtung übermittelt werden. Durch die Informationsübermittlung kann das Laborgerät, insbesondere eine dort laufende Behandlung, mittels der zweiten Benutzerschnittstelleneinrichtung weiter beobachtet und/oder gesteuert werden. Insbesondere kann der Verwendungszustand der ersten Benutzerschnittstelleneinrichtung teilweise oder vollständig in die zweite Benutzerschnittstelleneinrichtung kopiert bzw. geklont werden. Die Informationsübermittlung kann insbesondere ein Synchronisierungsvorgang sein. Die erste und zweite Benutzerschnittstelle können insbesondere auf diese Weise synchronisiert werden.

Die genannten Gestaltungen des erfindungsgemäßen Laborgeräts und/oder der erfindungsgemäßen Zugriffssteuerung können mit anderen Merkmalen kombiniert werden, die im Rahmen der Beschreibung der vorliegenden Erfindung genannt sind.

Vorzugsweise ist die Zugriffssteuerung, insbesondere die Steuereinrichtung der Zugriffssteuerung, so eingerichtet, dass die durch Benutzer beeinflussbaren Einstellungen mindestens einen Programmparameter für die programmgesteuerte Behandlung einer Laborprobe beinhalten, die insbesondere über ein Methodenprogramm gesteuert wird.

Vorzugsweise weist die Steuereinrichtung der Zugriffssteuerung und/oder die Steuereinrichtung des Laborgeräts eine Speichereinrichtung auf, in der Benutzerqualifikationsdaten gespeichert sind, in denen für jeden Benutzer des Laborgeräts eine Qualifikation in Form mindestens eines Qualifikationswertes oder Zertifikats zugeordnet werden. Vorzugsweise ist die Zugriffssteuerung, insbesondere die Steuereinrichtung der Zugriffssteuerung, so eingerichtet, dass einem Benutzer in Abhängigkeit von dessen Qualifikation die Berechtigungen und/oder Zugriffsrechte erteilt werden, insbesondere eine Rolle zugewiesen wird. Dadurch können Benutzer das Laborgerät entsprechend ihrer Qualifikation verwenden, wodurch insbesondere unerfahrene Benutzer nicht überfordert werden. Dadurch wird die Produktivität und Betriebssicherheit bei der Verwendung des Laborgeräts gesteigert.

Vorzugsweise ist die Steuereinrichtung der Zugriffssteuerung und/oder die Steuereinrichtung des Laborgeräts dazu ausgebildet ein Qualifizierungsverfahren für mindestens einen Benutzer durchzuführen, bei dem der mindestens eine Benutzer eine von der Steuereinrichtung durchgeführte und ausgewertete Qualifizierungsprüfung durchläuft und wobei das Qualifizierungsverfahren insbesondere vorsieht, die vom mindestens einem Benutzer in Antwort auf bestimmte Fragen eingegebenen Daten auszuwerten und insbesondere vorsieht dem mindestens einen Benutzer, insbesondere gemäß einer Vergleichstabelle oder Rechenvorschrift, in Abhängigkeit vom Ergebnis dieser Auswertung eine Qualifikation zuzuordnen. Ein derartiges, an der Zugriffssteuerung bzw. am Laborgerät durchgeführtes Qualifizierungsverfahren ist besonders praxisnah und deshalb effizient.

Vorzugsweise ist die Steuereinrichtung der Zugriffssteuerung und/oder die Steuereinrichtung des Laborgeräts dazu ausgebildet dem Benutzer in Abhängigkeit von seiner Qualifikation mittels der Zugriffssteuerung bestimmte Zugriffsrechte auf Funktionen des Laborautomaten zu erteilen und/oder zu entziehen.

Vorzugsweise ist die Steuereinrichtung der Zugriffssteuerung und/oder die Steuereinrichtung des Laborgeräts dazu ausgebildet dem Benutzer in Abhängigkeit von seiner Qualifikation mindestens eine, der Qualifikation entsprechende, grafische Benutzeroberfläche auf der Anzeige der Benutzerschnittstelleneinrichtung anzuzeigen und/oder insbesondere bestimmte Hilfsassistenzprogramme und/oder Hilfsinformationen zu Verfügung zu stellen oder nicht zur Verfügung zu stellen.

Vorzugsweise weist die Steuereinrichtung der Zugriffssteuerung und/oder die Steuereinrichtung des Laborgeräts einen Zeitgeber, insbesondere eine Uhr, und/oder insbesondere eine Reservierungseinrichtung auf, die eine Speichereinrichtung aufweist, in der Reservierungsdaten gespeichert sind, die insbesondere mindestens einen Reservierungsdatensatz oder eine Vielzahl von Reservierungsdatensätzen enthalten, die zumindest einen Reservierungszeitplan ("booking schedule"), insbesondere für jede Behandlungseinrichtung einzeln, beschreiben.

Ein Reservierungsdatensatz enthält insbesondere mindestens eine der Informationen, insbesondere welcher Benutzer, insbesondere zu welchem Zeitpunkt, insbesondere welche Behandlung von Proben, insbesondere mittels welches Laborgerätes, durchführt bzw. durchgeführt hat bzw. durchführen wird. Die Reservierungsdaten enthalten vorzugsweise Informationen über die von der Reservierungseinrichtung akzeptierten Reservierungen, die nach einem Vergleich mit den im Reservierungszeitplan vorhandenen freien Kapazitäten tatsächlich bestätigt und in den Reservierungszeitplan aufgenommen wurden. Die Reservierungsdaten können aber auch Reservierungsanträge ("booking requests") enthalten, die die Reservierungseinrichtung insbesondere auch zu einem späteren Zeitpunkt nach der Antragstellung erneut prüfen kann und gegebenenfalls später akzeptieren kann, falls z.B. ein früherer Eintrag in der Reservierungszeitplan nachträglich storniert wurde. Der Reservierungsdatensatz enthält vorzugsweise auch die Information darüber, welche Art der Behandlung an einem Laborgerät jeweils geplant ist, welcher konkrete Zeitraum oder welche Dauer der Belegung des Laborgeräts dabei vorgesehen ist, und/oder Informationen über das zu verwendende Methodenprogramm, und enthält vorzugsweise insbesondere mindestens einen Programmparameter oder Steuerparameter.

Vorzugsweise ist die Zugriffssteuerung dazu ausgebildet, einem Benutzer auf Anfrage mindestens eine Information über den Reservierungszeitplan zu übersenden, insbesondere den Reservierungszeitplan teilweise oder vollständig zu übersenden oder mindestens eine Änderung des Reservierungszeitplans zu übersenden. Vorzugsweise ist die Zugriffssteuerung dazu ausgebildet, einem Benutzer automatisch eine Nachricht in Abhängigkeit von mindestens einer Bedingung zu übersenden. Diese Bedingung könnte die Änderung des Reservierungszeitplans eines Laborgeräts sein, insbesondere betreffend die Verfügbarkeit eines Termins zur Durchführung einer Behandlung, insbesondere das Freiwerden oder Stornieren eines Termins.

Die "Art der Behandlung" ist insbesondere durch die Programmparameter vorgegeben, die eine Behandlung kennzeichnen. Solche Programmparameter werden insbesondere von der Steuereinrichtung dazu verwendet ein Methodenprogramm zu erzeugen. Ein Methodenprogramm ist insbesondere ein Steuerungscode zur Steuerung der Behandlung mittels Steuerparameter. Die Steuerparameter werden insbesondere von der Steuereinrichtung, insbesondere von einem in der Steuereinrichtung ablaufenden Steuerprogramm, z.B. einem Betriebssystem, unter Verwendung der Programmparameter erzeugt. Die Behandlung einer Probe wird insbesondere durchgeführt, indem von der Steuereinrichtung ein Methodenprogramm ausgeführt wird.

Eine "Art der Behandlung" meint eine Methode, nämlich eine Anwendungsart (z.B. "MagSep Blood gDNA", "Compose Mastermix" etc.). Der Benutzer wählt bei einer bevorzugten Ausgestaltung des Laborgeräts als Laborautomat zunächst eine gewünschte Anwendung, also eine "Art der Behandlung", indem er insbesondere am Touchscreen eines Geräts eine Anwendung auswählt. Diese Anwendung, auch bezeichnet als "Methode", ist insbesondere einem Programmmodul zugeordnet, das insbesondere ein Bestandteil des Steuerprogramms sein kann. Insbesondere mittels des Programmoduls wird vom Benutzer mindestens ein Programmparameter abgefragt. Ein Programmmodul erzeugt insbesondere ein Methodenprogramm auf Basis des mindestens einen vom Benutzer gewählten Programmparameters.

Vorzugsweise ist die Steuereinrichtung der Zugriffssteuerung und/oder die Steuereinrichtung des Laborgeräts dazu ausgebildet Reservierungsdaten in der Speichereinrichtung der Reservierungseinrichtung abzuspeichern.

Vorzugsweise ist die Steuereinrichtung der Zugriffssteuerung und/oder die Steuereinrichtung des Laborgeräts dazu ausgebildet den von einem Benutzer in das Laborgerät, insbesondere mittels der Benutzerschnittstelleneinrichtung oder einer portablen bzw. mobilen Benutzerschnittstelleneinrichtung eingegebenen Reservierungsdatensatz aufzunehmen.

Vorzugsweise ist die Steuereinrichtung der Zugriffssteuerung und/oder die Steuereinrichtung des Laborgeräts dazu ausgebildet den von einem Benutzer eingegebenen Reservierungsdatensatz mit bereits in der Speichereinrichtung der Benutzerschnittstelleneinrichtung gespeicherten Reservierungsdaten zu vergleichen.

Vorzugsweise ist die Steuereinrichtung der Zugriffssteuerung und/oder die Steuereinrichtung des Laborgeräts dazu ausgebildet mindestens einen, einige oder alle von mindestens einem Benutzer eingegebenen Reservierungsdatensätze in der Speichereinrichtung zu speichern.

Vorzugsweise ist die Steuereinrichtung der Zugriffssteuerung und/oder die Steuereinrichtung des Laborgeräts dazu ausgebildet einige oder alle von mindestens einem Benutzer eingegebenen und in der Speichereinrichtung gespeicherten Reservierungsdatensätze gemäß einem in der Steuereinrichtung abgelegten Auswerteverfahren auszuwerten und nach mindestens einem Kriterium den Zeitplan zu erstellen, indem der/die Reservierungsdatensätze gemäß dem mindestens einen Kriterium eines in der Steuereinrichtung abgelegten Sortierverfahrens sortiert werden.

Vorzugsweise ist die Steuereinrichtung der Zugriffssteuerung und/oder die Steuereinrichtung des Laborgeräts dazu ausgebildet, mittels eines Auswerteverfahrens dem mindestens einen Reservierungsdatensatz eine Priorität zuzuordnen, die gemäß mindestens eines Kriteriums ermittelt wird.

Das Kriterium kann insbesondere durch eine in der Steuereinrichtung gespeicherten Datentabelle repräsentiert sein, in der z.B. die Priorität zu mindestens einem anderen Parameter in Bezug gesetzt wird, wobei dieser andere Parameter z.B. den Benutzer oder eine Benutzergruppe, oder die Klassifizierung einer Behandlung gemäß einer Relevanzliste (z.B. von wichtig bis unwichtig, kostspielig bis kostengünstig, etc.) charakterisieren kann.

Vorzugsweise ist die Steuereinrichtung der Zugriffssteuerung und/oder die Steuereinrichtung des Laborgeräts dazu ausgebildet, dass das Sortierverfahren mindestens zwei Reservierungsdatensätze gemäß mindestens eines Kriteriums sortiert, um insbesondere einen Zeitplan zu erstellen, der andere Zeitdaten verwendet als dies in den Reservierungsdatensätzen der Benutzer vorgesehen ist.

Das Kriterium kann entsprechend der Definitionen beim Auswerteverfahren gewählt sein. Vorzugsweise ist die Steuereinrichtung zur Realisierung eines bevorzugten Kriteriums dazu ausgebildet die Reservierungsdatensätze unter dem Aspekt zu sortieren, dass eine Ressource optimiert wird.

Diese Ressource kann z.B. die Zeit sein, insbesondere kann eine Minimierung der Wartezeiten angestrebt werden, die ein Benutzer jeweils als Differenz zwischen der von ihm gewünschten Startzeit und der vom Laborgerät nach Auswertung und Sortierung zugewiesenen Startzeit für sein Experiment, also die von ihm gewünschte Behandlung, erhält. Es kann auch die Minimierung der Passivitätszeit angestrebt werden, während der ein Laborgerät nicht genutzt wird. Insbesondere können auch zwischenzeitliche Wartungs-, Reinigungs- und/oder Sterilisierungsvorgänge mit eingeplant werden, während der z.B. mindestens ein Arbeitsbereich mindestens eines Laborgeräts bzw. Laborautomaten vorbereitet wird, insbesondere manuell und/oder automatisch vorbereitet und/oder gereinigt und/oder sterilisiert wird.

Diese Ressource kann auch die Energie sein, die möglicherweise über verschiedene und nacheinander ablaufende Behandlungen in Abhängigkeit von deren Reihenfolge unterschiedlich stark verbraucht wird.

Die Ressource kann ein Verbrauchsmaterial sein, insbesondere eine Substanz, z.B. ein Reinigungsmittel, oder bestimmte Transportbehälter, z.B. Pipettenspitzen, oder Lagerbehälter, z.B. Mikrotiterplatten, die über verschieden und nacheinander ablaufende Behandlungen in Abhängigkeit von deren Reihenfolge unterschiedlich stark verbraucht werden. Möglicherweise werden bei von unterschiedlichen Benutzern geplanten Behandlungen die gleichen Methoden verwendet, so dass es effizient sein kann die Reservierungen anhand der Methoden zu sortieren. Es ist z.B. denkbar, dass eine bestimmte Substanz und/oder ein bestimmtes Verbrauchsmaterial und/oder ein bestimmtes Werkzeug bei mehreren von unterschiedlichen (oder denselben Benutzern) geplanten Methoden eingesetzt werden. Dann kann es insbesondere effizient sein diese Substanz bzw. dieses Verbrauchsmaterial bzw. dieses Werkzeug im Laborgerät zu lagern, so dass einige Transportvorgänge überflüssig werden, wodurch Zeit gespart wird und ggf. auch die Ressource selbst, die oftmals unter sterilen Bedingungen gehalten werden muss. Es wäre z.B. auch möglich, dass zwei im Reservierungszeitplan zeitlich nacheinander vorgesehene Behandlungen sich bestimmte Verbrauchsmaterialien teilen können. Beispielsweise könnte ein und derselbe Lagerbehälter in beiden Behandlungen verwendbar sein, so dass es effizient ist, den Lagerbehälter nach Beendigung der ersten Behandlung für die zweite Behandlung zu verwenden, anstatt zum Ende der ersten Behandlung den ersten Lagerbehälter zu entsorgen und zu Beginn der zweiten Behandlung einen weiteren Lagerbehälter einzusetzen. Darüber hinaus wäre es z.B. auch möglich zwei separate Reservierungen für eine identische Behandlung zusammen zu legen und in einer Behandlung in einem einzigen Verbrauchsmaterial gemeinsam (Mikrotiterplatte) abzuarbeiten. Dadurch kann in vielen Situationen Material und Zeit gespart werden.

Die Ressource kann auch die Mehrzahl von Laborgeräten sein, auf die die in einem Reservierungszeitraum anfallenden Reservierungen gemäß der mehreren Reservierungsdatensätze mehrerer Benutzer automatisch verteilt werden sollen, um eine optimale Ausnutzung des in einem Labor verfügbaren Parks von Laborgeräten zu erhalten. Insbesondere kann es Experimente geben, die den zeitlich abgestimmten Einsatz von mehr als einem Laborgerät erfordern. Die Ressource kann deshalb darin bestehen eine Mehrzahl von Laborgeräten zeitlich optimal zu nutzen, insbesondere unter Berücksichtigung mindestens eines Experiments oder mehrerer Experimente, die jeweils unterschiedliche Laborgeräte benötigen können.

Beispielsweise ist es möglich, dass eine höherrangige Rolle, z.B. ein "Administrator", bereits vorbelegte Reservierungseinträge im Reservierungszeitplan löschen oder verschieben kann, beispielsweise weil ein (externer) Servicetechniker die Behandlungseinrichtung(en) an dem Termin warten muss/will oder aus anderen oben geschilderten Gründen. Insbesondere aus Kundensicht ist dabei ein ungefragtes Agieren nicht bevorzugt, sondern ein Hinweis an den/die Benutzer der vorbelegten einen oder mehreren Behandlungseinrichtungen, dass die Nutzung der Behandlungseinrichtung auf einen späteren Zeitpunkt verschoben werden muss. Dabei könnte auch das Vorschlagen eines geeigneten alternativen Zeitpunktes sinnvoll sein. Die Steuereinrichtung ist vorzugsweise dazu ausgebildet, einen solchen Hinweis über die Benutzerschnittstelleneinrichtung des betreffenden Benutzers auszugeben, insbesondere unter Verwendung einer Datenfernverbindung.

Sofern eine Behandlungseinrichtung sehr stark nachgefragt ist, eignet sich insbesondere ein Reservierungsmechanismus, der als eine FIFO-Liste (FIFO -first in first out) aufgebaut ist und die wiederum dafür genutzt wird den an oberster Stelle stehenden Benutzer in dem Moment zu informieren, wenn die Behandlungseinrichtung nicht belegt ist. Diese Information umfasst dann vorzugsweise auch den Zeitrahmen, den die Behandlungseinrichtung zur Verfügung steht. Der an oberster Stelle stehende Benutzer bekäme dann für einen definierten Zeitraum die Priorität die Behandlungseinrichtung zu belegen. Tut er dies nicht, wird der Benutzer von der Liste gestrichen und die Belegungsoption geht an den nächsten Benutzer auf der Liste usw.

Der Begriff "gerätegesteuerte Behandlung" bedeutet, dass die Behandlung der mindestens einen Laborprobe zumindest teilweise vom Laborgerät gesteuert wird, insbesondere durchgeführt wird. Soweit die Behandlung vom Laborgerät gesteuert und/oder durchgeführt wird, wird diese insbesondere insofern nicht vom Benutzer gesteuert und/oder durchgeführt, insbesondere nicht manuell vom Benutzer gesteuert und/oder durchgeführt.

Unter einer gerätegesteuerten Behandlung wird ferner vorzugsweise verstanden, dass die Behandlung in Abhängigkeit von mindestens einer Benutzereingabe zumindest teilweise vom Laborgerät gesteuert wird, insbesondere durchgeführt wird. Die Benutzereingabe kann vor dem Start der Behandlung erfolgen und/oder während der Behandlung. Die Benutzereingabe erfolgt vorzugsweise über eine Benutzerschnittstelleneinrichtung, die vorzugsweise ein Bestandteil des Laborgeräts ist, oder die separat vom Laborgerät vorgesehen ist und mit der Steuereinrichtung des Laborgeräts und/oder der Steuereinrichtung der Zugriffssteuerung signalverbunden ist. Die Benutzereingabe dient insbesondere der Eingabe mindestens eines Parameters, dessen Wert die Behandlung beeinflusst und/oder steuert. Dieser Parameter kann insbesondere ein Programmparameter sein.

Die "gerätegesteuerte Behandlung" bezeichnet insbesondere die zumindest teilautomatisierte Behandlung. Bei einer teilautomatisierten Behandlung ist es insbesondere möglich, dass die Behandlung so durchgeführt wird, dass nach dem Starten der Behandlung und vor dem Beenden der Behandlung mindestens eine Benutzereingabe erfolgt, mit der der Benutzer die laufende Behandlung beeinflussen kann, insbesondere indem er z.B. eine über eine Benutzerschnittstelleneinrichtung des Laborgeräts erfolgende automatische Abfrage beantwortet, insbesondere eine Eingabe bestätigt oder verneint oder andere Eingaben vornimmt. Bei der teilautomatisierten Behandlung ist es insbesondere möglich, dass die Behandlung mehrere Behandlungsschritte aufweist, die insbesondere zeitlich nacheinander automatisch durchgeführt werden und mindestens einen Behandlungsschritt aufweist, der eine, insbesondere über eine Benutzerschnittstelleneinrichtung erfolgende, Benutzereingabe erfordert.

Eine gerätegesteuerte Behandlung ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mithilfe einer digitalen Datenverarbeitungseinrichtung, die insbesondere Bestandteil der Steuereinrichtung des Laborgeräts sein kann. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Ein Programmparameter kann ein benutzerseitig erforderlicher Programmparameter sein. Ein benutzerseitig erforderlicher Programmparameter zeichnet sich dadurch aus, dass er für die Ausführung einer Behandlung, insbesondere zur Ausführung eines Methodenprogramms, erforderlich ist. Andere Programmparameter, die nicht benutzerseitig erforderlich sind, können aus den benutzerseitig erforderlichen Programmparametern abgeleitet werden oder anderweitig verfügbar gemacht werden, insbesondere wahlweise vom Benutzer eingestellt werden. Die Einstellung eines Programmparameters durch einen Benutzer erfolgt insbesondere durch Anzeige einer Auswahl von möglichen vorgegebenen Werten aus einer im Laborgerät gespeicherten Liste von vorgegebenen Werten, wobei der Benutzer aus dieser Liste den gewünschten Parameter auswählt und somit einstellt. Es ist auch möglich, dass dieser Programmparameter eingestellt wird, indem der Benutzer den Wert eingibt, indem er z.B. über einen Ziffernblock eine Zahl eingibt, die dem gewünschten Wert entspricht oder indem der Benutzer einen Wert kontinuierlich oder in Inkrementen erhöht bzw. erniedrigt, bis dieser dem gewünschten Wert entspricht und den Wert so einstellt. Andere Formen der Eingabe, z.B. durch Sprachsteuerung und/oder Gestensteuerung, sind denkbar.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungsanlage eine bestimmte Funktionalität, Aufgaben- oder Problemstellung bearbeiten und/oder lösen zu können. Ein Programm liegt in der Regel als Software vor, die mit einer digitalen Datenverarbeitungsanlage verwendet wird. Das Programm kann insbesondere als Firmware vorliegen, im Fall der vorliegenden Erfindung insbesondere als Firmware der Steuereinrichtung des Laborgeräts und/oder der Zugriffssteuerung. Das Programm liegt meist auf einem Datenträger als ausführbare Programmdatei, häufig im sogenannten Maschinencode vor, die zur Ausführung in den Arbeitsspeicher des Rechners der digitalen Datenverarbeitungsanlage geladen wird. Das Programm wird als Abfolge von Maschinen-, d. h. Prozessorbefehlen von dem/den Prozessoren des Computers verarbeitet und damit ausgeführt. Unter ,Computerprogramm' wird insbesondere auch der Quelltext des Programms verstanden, aus dem im Verlauf der Steuerung des Laborgerätes der ausführbare Code entstehen kann.

Als Anweisung wird in üblicher Weise ein zentrales Element einer Programmiersprache bezeichnet. Die Programme derartiger Sprachen setzen sich primär aus einer oder mehreren Anweisungen zusammen. Eine Anweisung stellt eine in der Syntax einer Programmiersprache formulierte einzelne Vorschrift dar, die im Rahmen der Abarbeitung des Programms auszuführen ist. Wie eine Anweisung syntaktisch auszusehen hat, wird durch die jeweilige Programmiersprache bzw. deren Spezifikation festgelegt. In der maschinennahen Programmierung werden Anweisungen häufig auch als Befehl bezeichnet. Anweisungen sind üblicherweise Zuweisungen, Kontrollanweisungen (wie Sprünge, Schleifen und bedingte Anweisungen) und Prozeduraufrufe. Abhängig von der Programmiersprache sind teilweise auch Zusicherungen, Deklarationen, Klassen- und Funktionsdefinitionen Anweisungen. Die Anweisungen des Steuerprogramms können so in üblicher Weise ausgestaltet sein.

Unter einem Programmmodul wird in üblicher Weise eine abgeschlossene funktionale Einheit einer Software verstanden, bestehend aus einer Folge von Verarbeitungsschritten und Datenstrukturen. Dabei können insbesondere folgende Definitionen zutreffen: Der Inhalt eines Moduls ist häufig eine wiederkehrende Berechnung oder Bearbeitung von Daten, die mehrfach durchgeführt werden muss. Module bieten eine Kapselung durch die Trennung von Schnittstelle und Implementierung: Die Schnittstelle eines Moduls definiert die Datenelemente, die als Eingabe und Ergebnis der Verarbeitung durch das Modul benötigt werden. Die Implementierung enthält den tatsächlichen Programmcode. Ein Modul wird zum Beispiel als Funktion oder Unterprogramm aufgerufen, führt eine Reihe von Verarbeitungsschritten durch und liefert als Ergebnis Daten zurück an das aufrufende Programm. Ein Modul kann selbst weitere Module aufrufen - so ist eine Hierarchie von Programmaufrufen möglich. Die in Modulen festgelegten Datenstrukturen und Methoden können gegebenenfalls vererbt und von anderen Modulen geerbt werden. Module sind daher ein wesentliches Element in der strukturierten und objektorientierten Programmierung.

Unter einem Steuerprogramm wird ein ausführbares Computerprogramm verstanden, das vorzugsweise die gewünschte Behandlung der mindestens einen Probe steuert und/oder durchführt, insbesondere in Abhängigkeit von mindestens einem Programmparameter. Dieser Programmparameter kann ein vom Benutzer beeinflusster und/oder eingestellter Programmparameter sein. Die Behandlung kann insbesondere gesteuert werden, indem die Steuereinrichtung in Abhängigkeit von den Programmparametern einen oder mehrere Steuerparameter erzeugt, mittels derer die mindestens eine Behandlungseinrichtung gesteuert wird. Vorzugsweise weist das Laborgerät ein Betriebssystem auf, das ein Steuerprogramm sein kann oder aufweisen kann. Das Steuerprogramm kann insbesondere ein Betriebssystem des Laborgeräts bezeichnen oder einen Bestandteil des Betriebssystems. Das Betriebssystem steuert die Behandlung und weitere Betriebsfunktionen des Laborgeräts.

Das Steuerprogramm kann insbesondere mit der Zugriffssteuerung signalverbunden sein, und/oder kann die Zugriffssteuerung steuern. Die Steuereinrichtung der Zugriffssteuerung kann in die Steuereinrichtung des Laborgeräts integriert sein oder kann getrennt von dieser Steuereinrichtung ausgebildet sein. Die Zugriffssteuerung kann in die Steuereinrichtung des Laborgeräts integriert sein. Die Steuerung der Zugriffssteuerung kann in die Steuerung des Laborgeräts integriert sein, kann vom Steuerprogramm steuerbar sein und/oder kann insbesondere in das Steuerungsprogramm integriert sein. Das Steuerprogramm kann weitere vorzugsweise vorgesehene Funktionen des Laborgeräts steuern, zum Beispiel eine Energiesparfunktion des Laborgeräts oder eine Kommunikationsfunktion zur Kommunikation mit externen Datenverarbeitungseinrichtungen, die insbesondere separat zum Laborgerät vorgesehen sind und insbesondere nicht ein Bestandteil des Laborgeräts sind.

Unter einem Methodenprogramm wird ein Programm verstanden, das den konkreten Ablauf einer Behandlung bestimmt, insbesondere gemäß einer vorbestimmten Behandlungsart und/oder gemäß einer benutzerseitig festgelegten Weise.

Die Erfindung betrifft ferner ein Laborgerät zur gerätegesteuerten Behandlung mindestens einer Laborprobe, das mindestens eine Behandlungseinrichtung zur Durchführung der Behandlung der mindestens einen Laborprobe und eine erfindungsgemäße Zugriffssteuerung aufweist.

Vorzugsweise weist das Laborgerät eine Kommunikationseinrichtung zur Herstellung einer Datenfernverbindung für den Datenaustausch mit einem externen Gerät auf, das ebenfalls eine geeignete Kommunikationseinrichtung zur Herstellung einer Fernverbindung für den Datenaustausch mit dem Laborautomat aufweist. Eine solche Kommunikationseinrichtung kann zur Ausbildung einer Funkverbindung ausgebildet sein, insbesondere einer Mobilfunkverbindung. Vorzugsweise ist die Kommunikationseinrichtung dazu eingerichtet den Fernzugriff eines Benutzers auf das Laborgerät, insbesondere die Auswahl oder Einstellung mindestens eines Parameters, zu ermöglichen, insbesondere eines Parameters, der eine Funktion des Laborgeräts steuert, insbesondere die Funktion der Ausführung einer Behandlung.

Vorzugsweise ist die Steuereinrichtung der Zugriffssteuerung oder des Laborgeräts dazu ausgebildet Synchronisationsdaten bereitzustellen. Vorzugsweise ist die Zugriffssteuerung, insbesondere die Steuereinrichtung der Zugriffssteuerung, so eingerichtet, dass, wenn mindestens eine Bedingung erfüllt ist, über die Schnittstelleneinrichtung Informationen über den Betriebszustand des Laborgeräts, Messwerte oder durch Benutzer beeinflussbare Einstellungen oder Programmierungen des Laborgeräts an die zweite Benutzerschnittstelleneinrichtung übermittelt werden. Durch die Informationsübermittlung kann das Laborgerät, insbesondere durch eine dort laufende Behandlung, mittels der zweiten Benutzerschnittstelleneinrichtung weiter beobachtet und/oder gesteuert werden. Insbesondere kann der Verwendungszustand der ersten Benutzerschnittstelleneinrichtung teilweise oder vollständig in die zweite Benutzerschnittstelleneinrichtung kopiert bzw. geklont werden. Die Informationsübermittlung kann insbesondere ein Synchronisierungsvorgang sein. Die erste und zweite Benutzerschnittstelle können insbesondere auf diese Weise synchronisiert werden. Die mindestens eine Bedingung kann sein, dass der Zugriff des zugreifenden Benutzers über eine Datenfernverbindung via eine (mobilen) Benutzerschnittstelleneinrichtung erfolgt und der Antrag des Benutzers nach einer Synchronisierung erfolgt. Die mindestens eine Bedingung kann zudem die Bedingung a) oder b) sein, nämlich die Antwort auf die Prüfung, ob der anmeldende Benutzer vorausgehend bereits über eine erste Benutzerschnittstelleneinrichtung a) eine oder mehrere gerade ausgeführte Funktionen des Laborgeräts aktiviert hatte oder b) schon angemeldet ist. In den Fällen a) und b) würde die Synchronisierung nur einem Benutzer mit aktiver Sitzung und/oder gerade am Laborgerät aktivierten Funktionen, insbesondere laufende Behandlungen, die vom Benutzer initiiert wurden, erlaubt werden. Es ist aber auch möglich und bevorzugt, dass es einem weiteren Benutzer erlaubt werden kann eine Synchronisierung durchzuführen, z.B. um eine Fernsteuerung zum Zwecke der Assistenz bei einer laufenden Sitzung oder Behandlung durchzuführen oder Wartungsarbeiten etc. durchzuführen.

Vorzugsweise ist die Steuereinrichtung der Zugriffseinrichtung dazu eingerichtet diese Synchronisationsdaten an eine - insbesondere mobile - Benutzerschnittstelleneinrichtung zu übertragen. Vorzugsweise sind diese Synchronisationsdaten dazu geeignet, die in der Anzeige der Benutzerschnittstelleneinrichtung angezeigten Informationen zumindest teilweise identisch auf der Anzeige der - insbesondere mobilen - Benutzerschnittstelleneinrichtung anzuzeigen.

Der Begriff Laborgerät bezeichnet insbesondere ein Gerät, das zur gerätegesteuerten Behandlung mindestens einer Laborprobe ausgebildet ist und das zur Verwendung in einem Labor ausgebildet ist. Bei diesem Labor kann es sich insbesondere um ein chemisches, biologisches, biochemisches, medizinisches oder forensisches Laboratorium handeln. Solche Labors dienen der Forschung und/oder der Analyse von Laborproben, können aber auch zur Herstellung von Produkten mittels Laborproben oder der Herstellung von Laborproben dienen.

Ein Laborgerät ist vorzugsweise eines der folgenden Laborgeräte und/oder ist vorzugsweise als mindestens eines der folgenden Laborgeräte ausgebildet: Laborzentrifuge, im Rahmen der Beschreibung der vorliegenden Erfindung auch bezeichnet als "Zentrifuge" oder "centrifuge"; Thermocycler, im Rahmen der Beschreibung der vorliegenden Erfindung auch bezeichnet als "Cycler"; Labor-Spektralphotometer, im Rahmen der Beschreibung der vorliegenden Erfindung auch bezeichnet als "Biospektrometer"; Zellenzählgerät, im Rahmen der Beschreibung der vorliegenden Erfindung auch bezeichnet als "cellcounter", insbesondere optische Zählgeräte; Labor-Inkubator, im Rahmen der Beschreibung der vorliegenden Erfindung auch bezeichnet als "incubator"; Labor-Schüttelgerät, im Rahmen der Beschreibung der vorliegenden Erfindung auch bezeichnet als "shaker"; Labor-Mischer, auch bezeichnet als "mixing device"; Labor-Gefriergerät, im Rahmen der Beschreibung der vorliegenden Erfindung auch bezeichnet als "freezer"; Bioreaktor, im Rahmen der Beschreibung der vorliegenden Erfindung auch bezeichnet als Fermenter; Sicherheitswerkbank, insbesondere Biosicherheitswerkbank, im Rahmen der Beschreibung der vorliegenden Erfindung auch bezeichnet als "biosafety cabinet"; Probenplatten-Lesegerät, im Rahmen der Beschreibung der vorliegenden Erfindung auch bezeichnet als "plate reader", insbesondere "microplate reader"; Laborautomat zur Behandlung von fluiden Proben, insbesondere Pipettierautomat;.

Eine Laborzentrifuge ist ein Gerät, das unter Ausnutzung der Massenträgheit arbeitet. Die Laborzentrifuge, insbesondere die Behandlungseinrichtung der Laborzentrifuge, weist insbesondere mindestens einen Rotor auf, in dem die mindestens eine Laborprobe anordenbar ist. Der mindestens eine Rotor ist rotierbar in mindestens einem Zentrifugenkessel angeordnet. Die Laborzentrifuge, insbesondere die Behandlungseinrichtung der Laborzentrifuge, weist mindestens eine Antriebseinrichtung auf, mittels der die Rotation angetrieben und/oder gebremst wird. Die Proben sind in dem mindestens einen Rotor anordenbar, vorzugsweise in Laborbehältern, z.B. Probenröhrchen, die in geeigneten Halterungen im Rotor angeordnet werden. Vorzugsweise weist die Laborzentrifuge, insbesondere die Behandlungseinrichtung der Laborzentrifuge, mindestens eine Heiz-/Kühleinrichtung auf, mit der die Temperatur der mindestens einen im Rotor angeordneten Probe gesteuert und/oder geregelt werden kann. Vorzugsweise weist die Laborzentrifuge, insbesondere die Behandlungseinrichtung der Laborzentrifuge, eine Zeitgebereinrichtung auf, mit der zeitliche Parameter der Rotation oder Temperatureinstellung steuerbar sind. Die Funktionsweise beruht auf der Zentrifugalkraft, die aufgrund einer gleichförmigen Kreisbewegung der zu zentrifugierenden Proben zustande kommt. Die Zentrifugalkraft wird zur Stofftrennung von Stoffen unterschiedlicher Dichte genutzt, die in einer Probe enthalten sind. Eine Zentrifuge kann ein Trennverfahren durchführen, bei dem insbesondere die Bestandteile von Suspensionen, Emulsionen und/oder Gasgemischen getrennt werden. Die gerätegesteuerte Behandlung der mindestens einen Laborprobe entspricht bei einer Laborzentrifuge einer Rotationsbehandlung, der die mindestens eine Probe unterzogen wird. Mögliche Parameter, insbesondere Programmparameter, insbesondere Nutzerparameter, die zur Beeinflussung einer Rotationsbehandlung verwendet werden, definieren insbesondere eine Temperatur der Laborzentrifuge, eine Rotationsgeschwindigkeit der Laborzentrifuge, einen zeitlichen Parameter der Rotation oder Temperatureinstellung, und/oder mindestens einen Ablaufparameter, der den Ablauf, insbesondere die Reihenfolge, eines aus mehreren Rotationsschritten bestehenden Rotationsprogramms beeinflusst oder definiert. Die Temperatur der Laborzentrifuge kann insbesondere mindestens eine Temperatur im Inneren des mindestens einen Rotors sein, insbesondere mindestens eine Temperatur mindestens einer Probe.

Ein Thermocycler ist ein Gerät, das in der Lage ist, die Temperatur mindestens einer Probe zeitlich nacheinander auf eine vorbestimmte Temperatur einzustellen und für eine vorgegebene Dauer auf dieser Temperaturstufe zu halten. Der Ablauf dieser Temperatursteuerung ist zyklisch. Das heißt ein vorbestimmter Temperaturzyklus, also eine Abfolge von mindestens zwei Temperaturstufen, wird wiederholt durchgeführt. Dieses Verfahren dient insbesondere der Durchführung einer Polymerase-Kettenreaktion (PCR). In diesem Zusammenhang bezeichnet man einen Thermocycler auch manchmal als PCR-Block. Ein Thermocycler, insbesondere die Behandlungseinrichtung des Thermocyclers, weist vorzugsweise einen Thermoblock auf. Ein Thermoblock ist ein Probenhalter aus einem wärmeleitenden Material, meistens ein metallhaltiges Material oder ein Metall, insbesondere Aluminium oder Silber. Der Probenhalter weist eine Kontaktierseite auf, die durch mindestens eine Heiz-/Kühleinrichtung des Thermocyclers, insbesondere ein Peltierelement, kontaktiert wird. Der Thermocycler, insbesondere die Behandlungseinrichtung des Thermocyclers, weist eine Regeleinrichtung mit mindestens einem Regelkreis auf, dem als Stellglied die mindestens eine Heiz-/Kühleinrichtung und als Messglied mindestens eine Temperaturmesseinrichtung zugeordnet sind. Mittels der Regeleinrichtung wird die Temperatur einer Temperaturstufe geregelt. Ein Kühlkörper des Thermocyclers, insbesondere der Behandlungseinrichtung des Thermocyclers, dient zur Kühlung von Abschnitten des Thermocyclers, insbesondere der Kühlung der Peltierelemente. Der Thermocycler, insbesondere die Behandlungseinrichtung des Thermocyclers, kann weitere Heiz- und/oder Kühlelemente aufweisen. Vorzugsweise weist der Thermocycler, insbesondere die Behandlungseinrichtung des Thermocyclers, eine Zeitgebereinrichtung auf, mit der zeitliche Parameter der Einstellung des Temperaturzyklus steuerbar sind. Die gerätegesteuerte Behandlung der mindestens einen Laborprobe entspricht bei einem Thermocycler einer Temperaturzyklusbehandlung, der die mindestens eine Probe unterzogen wird. Mögliche Parameter, insbesondere Programmparameter, insbesondere Nutzerparameter, die zur Beeinflussung einer Temperaturzyklusbehandlung verwendet werden, definieren insbesondere die Temperatur einer Temperaturstufe, die Dauer einer Temperaturstufe, die Steuerung weiterer Heiz- und/oder Kühlelemente und/oder die Anzahl von Temperaturstufen oder Zyklen und/oder mindestens einen Ablaufparameter, der den Ablauf, insbesondere die Reihenfolge, eines aus mehreren Schritten bestehenden Temperaturkontrollprogramms beeinflusst oder definiert.

Ein Labor-Spektralphotometer ist ein Gerät, das durch das Beleuchten mindestens eines Messvolumens mindestens einer Laborprobe meist über das gesamte Spektrum von infrarot bis ultraviolett des sichtbaren Lichtes die Remissionswerte ermittelt. Die Remission bezeichnet die Situation, dass ein Messvolumen einen Teil des Lichtspektrums absorbiert und einen Teil des Spektrums transmittiert (transparente Medien) bzw. reflektiert (undurchsichtige Medien). Mit dem Labor-Spektralphotometer wird insbesondere das Absorptionsvermögen einer Probe in Abhängigkeit von der Lichtwellenlänge gemessen. Darüber hinaus besteht insbesondere die Möglichkeit den Anwendungsbereich des Labor-Spektralphotometers durch verschiedene Module zu erweitern. So ist z.B. die Anordnung eines Fluoreszenz-Moduls zur Messung von Fluoreszenz oder eines Temperiermoduls zur Temperierung der Probe im Spektrometer denkbar. Das gemessene Absorptionsspektrum enthält insbesondere die bei bestimmten Wellenlängen gemessenen Lichtintensitäten. Das Absorptionsspektrum ist charakteristisch für die Laborprobe bzw. den darin enthaltenen Stoff oder die darin enthaltenen Stoffe. Dies kann zur qualitativen Analyse der Laborprobe genutzt werden. Ist die flüssige Probe bzw. der darin gelöste Stoff bekannt, kann durch Messung der Absorption die Konzentration des gelösten Stoffes ermittelt werden. Dies kann zur quantitativen Analyse der Laborprobe genutzt werden. Das Labor-Spektralphotometer, insbesondere die Behandlungseinrichtung des Labor-Spektralphotometers, weist vorzugsweise mindestens eine Lichtquelle auf, vorzugsweise mindestens einen Zeitgeber, vorzugsweise mindestens einen Photodetektor. Die gerätegesteuerte Behandlung der mindestens einen Laborprobe entspricht bei einem Labor-Spektralphotometer einer Licht- und Messbehandlung, der die mindestens eine Probe unterzogen wird. Mögliche Parameter, insbesondere Programmparameter, insbesondere Nutzerparameter, die zur Beeinflussung einer Licht- und Messbehandlung verwendet werden, definieren insbesondere das optische Lichtspektrum, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms beeinflusst oder definiert.

Ein Zellenzählgerät dient dem Zählen von biologischen Zellen bzw. Partikeln, die in einer Laborprobe enthalten sind. Es gibt verschiedene physikalische Prinzipien, die zum Zählen von Zellen in Frage kommen, insbesondere optische Verfahren, bei denen die zu messende Laborprobe in einer Zählkammer angeordnet wird, und insbesondere bei automatisch arbeitenden zusätzlich beleuchtet wird und ein Bild der in der Zählkammer angeordneten Zellen bzw. Partikel erfasst wird und ausgewertet wird. Ein weiteres, etabliertes Verfahren ist die Impedanzmessung: ein als Coulter Counter ausgeführtes Zellenzählgerät leitet die die Zellen enthaltende Laborprobe durch eine Apertur ("Messschleuse"). Jeder Durchtritt einer Zelle durch die Apertur wird als zählbares Ereignis elektrisch detektiert. Optische Zellenzählgeräte, insbesondere die Behandlungseinrichtung des Zellenzählgeräts, weisen je nach Ausführung vorzugsweise mindestens eine Lichtquelle auf, mindestens eine Bilderfassungseinrichtung und mindestens eine Bildauswerteeinrichtung*, zusätzlich u.U. eine Positioniereinrichtung. Die gerätegesteuerte Behandlung der mindestens einen Laborprobe entspricht bei einem optischen Zellenzählgerät z.B. einer Licht- und Messbehandlung, bei einem nach Coulter Prinzip arbeitenden Gerät einer Pump- und Messbehandlung, der die mindestens eine Probe unterzogen wird. Mögliche Parameter, insbesondere Programmparameter, insbesondere Nutzerparameter, die zur Beeinflussung einer Licht- und Messbehandlung oder der Pump- und Messbehandlung verwendet werden, definieren insbesondere die Lichtintensität der Lichtquelle, mit dem die mindestens eine Probe bestrahlt wird und/oder mindestens einen Ablaufparameter, der den Ablauf, insbesondere die Reihenfolge, eines aus mehreren Schritten bestehenden Licht- und Messbehandlungsprogramms oder Pump- und Messbehandlungsprogramms beeinflusst oder definiert. Bei optischen Zählgeräten sind außerdem die für die Bildauswertung notwendigen Algorithmen, deren Reihenfolge und Parametrierung entscheidend für die Aussagekraft des Messergebnisses. Optische Messgeräte, aber auch Coulter Counter, nutzen oft Zählkammern zum einmaligen Gebrauch ("Consumables)", dies sind den herkömmlichen Neubauerzählkammern nachempfundene Kunststoffartikel, bzw., bei den Coulter Countern, "Lab-on-a-Chip"- ähnliche Einmalzählkammern. Es gibt aber auch Geräte, die ohne diese Verbrauchsmaterialien arbeiten (z.B. "CASY").

Ein Labor-Inkubator ist ein Gerät, mit dem kontrollierte Klimabedingungen für verschiedene biologische Entwicklungs- und Wachstumsprozesse geschaffen und erhalten werden können. Er dient der Schaffung und Erhaltung eines Mikroklimas mit geregelten Gas-, und/oder Luftfeuchtigkeits- und/oder Temperatur-Bedingungen in einem Inkubatorraum, wobei diese Behandlung zeitabhängig sein kann. Der Labor-Inkubator, insbesondere die Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines dem Inkubatorraum zugeführten Austauschgases, insbesondere Frischluft, eine Einstelleinrichtung für die Zusammensetzung des Gases im Inkubatorraum des Labor-Inkubators, insbesondere zur Einstellung des CO₂ und/oder des O₂ Gehalts des Gases und/oder eine Einstelleinrichtung zur Einstellung der Luftfeuchtigkeit im Inkubatorraum des Labor-Inkubators. Der Labor-Inkubator, insbesondere die Behandlungseinrichtung des Labor-Inkubators, weist insbesondere den Inkubatorraum auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. CO₂₋Inkubatoren dienen insbesondere der Kultivierung tierischer bzw. humaner Zellen. Inkubatoren können Wendevorrichtungen zum Wenden der mindestens einen Laborprobe und/oder eine Schütteleinrichtung zum Schütteln bzw. Bewegen der der mindestens einen Laborprobe aufweisen. Die gerätegesteuerte Behandlung der mindestens einen Laborprobe entspricht bei einem Labor-Inkubator einer Klimabehandlung, der die mindestens eine Probe unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, den O₂- und/oder CO₂-Partialdruck im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Ein Labor-Schüttelgerät dient der Bewegung einer Laborprobe, insbesondere zum Mischen einer mehrere Bestandteile aufweisenden Laborprobe. Labor-Schüttelgeräte gibt es in verschiedenen Ausführungen, insbesondere als Überkopf-Schüttelgeräte oder als Flachbett-Schüttelgeräte. Labor-Schüttelgeräte können eine Temperierfunktion zum Temperieren der mindestens einen Laborprobe aufweisen und können insbesondere eine Inkubatorfunktion zum Inkubieren der mindestens einen Laborprobe bei kontrollierten Klimabedingungen aufweisen. Labor-Schüttelgeräte, insbesondere deren Behandlungseinrichtung, können insbesondere zur Durchführung einer oszillierenden Bewegung eingerichtet sein. Labor-Schüttelgeräte, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Schüttelbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Schüttelgerät einer Schüttelbehandlung, der die mindestens eine Probe unterzogen wird. Mögliche Parameter, insbesondere Programmparameter, insbesondere Nutzerparameter, die zur Beeinflussung einer Schüttelbehandlung verwendet werden, definieren insbesondere die Bewegungsintensität, insbesondere die Bewegungsfrequenz bei einem oszillierenden Antrieb, einer Zeitperiode bei der Schüttelbehandlung und/oder mindestens einen Ablaufparameter, der den Ablauf, insbesondere die Reihenfolge, eines aus mehreren Schritten bestehenden Schüttelbehandlungsprogramms beeinflusst oder definiert.

Ein Labor-Mischer, auch bezeichnet als "mixing device", dient wie das Labor-Schüttelgerät der Bewegung einer Laborprobe, insbesondere zum Mischen einer mehrere Bestandteile aufweisenden Laborprobe. Im Vergleich zu einem Labor-Schüttelgerät ermöglicht ein Labor-Mischer Bewegungen mit höheren Frequenzen, insbesondere höheren Drehzahlen. Labor-Mischer, insbesondere deren Behandlungseinrichtung, können insbesondere zur Durchführung einer oszillierenden Bewegung eingerichtet sein. Labor-Mischer, insbesondere deren Behandlungseinrichtung, weisen insbesondere einen Antrieb zum Antreiben der Bewegung auf, weisen insbesondere eine Zeitgebereinrichtung auf, mit der zeitliche Parameter der Einstellung der Mischbehandlung steuerbar sind und weisen insbesondere mindestens eine Heiz-/Kühleinrichtung und mindestens eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Die gerätegesteuerte Behandlung der mindestens einen Laborprobe entspricht bei einem Labor-Mischer einer Mischbehandlung, der die mindestens eine Probe unterzogen wird. Mögliche Parameter, insbesondere Programmparameter, insbesondere Nutzerparameter, die zur Beeinflussung einer Mischbehandlung verwendet werden, definieren insbesondere die Bewegungsintensität, insbesondere die Bewegungsfrequenz bei einem oszillierenden Antrieb, einer Zeitperiode bei der Mischbehandlung und/oder mindestens einen Ablaufparameter, der den Ablauf, insbesondere die Reihenfolge, eines aus mehreren Schritten bestehenden Mischbehandlungsprogramms beeinflusst oder definiert.

Ein Labor-Gefriergerät dient der Lagerung mindestens einer Laborprobe in einem Gefrierraum bei geregelten Temperaturen insbesondere im Tiefkühlbereich von -18° C bis -50 °C oder im Ultratiefkühlbereich von -50° C bis - 90° C. Ein Labor-Gefriergerät ist insbesondere kein Kühlschrank, der zum Kühlen bei Temperaturen insbesondere im Bereich von 0° C bis 10° C oder von - 10° C bis 10 °C verwendbar ist. Ein Labor-Gefriergerät, insbesondere die Behandlungseinrichtung des Labor-Gefriergeräts, weist insbesondere mindestens eine Kühleinrichtung und mindestens eine Regeleinrichtung mit mindestens einem Regelkreis auf, dem als Stellglied die mindestens eine Kühleinrichtung und als Messglied mindestens eine Temperaturmesseinrichtung zugeordnet ist. Ein Labor-Gefriergerät, insbesondere die Behandlungseinrichtung des Labor-Gefriergeräts, weist insbesondere ein Kontrollmessgerät zur Temperaturmessung und/oder insbesondere eine Alarmeinrichtung auf, mit der ein Alarmsignal ausgegeben wird, wenn die im Gefrierraum gemessene Temperatur einen erlaubten Temperaturbereich verlässt. Ein Labor-Gefriergerät, insbesondere die Behandlungseinrichtung des Labor-Gefriergeräts, kann insbesondere eine Informationsleseeinrichtung zum Lesen der Information aufweisen. Diese Information kann auf einem Informationsträger enthalten sein, der mit einem Artikel verbunden sein kann. Dieser Artikel kann insbesondere ein Probenbehälter sein, der mindestens eine Laborprobe enthalten kann. Der Informationsträger kann insbesondere einen RFID-Chip oder andere Identifikationsmerkmale aufweisen, wie z.B. einen Barcode, einen Datamatrix Code, einen QR-Code, die mit geeigneten Verfahren lesbar sind. Die gerätegesteuerte Behandlung der mindestens einen Laborprobe entspricht bei einem Labor-Gefriergerät einer Tieftemperaturbehandlung, der die mindestens eine Probe unterzogen wird. Mögliche Parameter, insbesondere Programmparameter, insbesondere Nutzerparameter, die zur Beeinflussung einer Tieftemperaturbehandlung verwendet werden, definieren insbesondere die Temperatur des Gefrierraums, in dem die mindestens eine Probe tiefgekühlt wird und/oder den Informationslesevorgang, der vorzugsweise durchgeführt wird, wenn ein mit einem Informationsträger versehener Artikel von einem Benutzer in das Labor-Gefriergerät überführt wird.

Ein Bioreaktorweist einen Behälter auf, in dem bestimmte Mikroorganismen, Zellen, Algen oder Pflanzen (z.B. Moose) unter möglichst optimalen Bedingungen kultiviert (auch: fermentiert) werden. Der Betrieb eines Bioreaktors ist somit eine Anwendung der Biotechnologie, die biologische Prozesse, insbesondere Biokonversion oder Biokatalyse, in technischen Einrichtungen nutzt bzw. nutzbar macht. Faktoren, die in den meisten Bioreaktoren insbesondere durch Einstellung entsprechender Parameter steuerbar oder kontrollierbar sind, sind die Zusammensetzung des Nährmediums, die Sauerstoffzufuhr, Temperatur, pH-Wert, Sterilität und/oder andere Faktoren. Zweck der Kultivierung in einem Bioreaktor kann die Gewinnung der Zellen oder von Bestandteilen der Zellen oder die Gewinnung von Stoffwechselprodukten sein. Diese können z. B. als Wirkstoff in der pharmazeutischen oder als Grundchemikalie in der chemischen Industrie verwendet werden. Auch der Abbau von chemischen Verbindungen kann in Bioreaktoren stattfinden, wie z. B. bei der Abwasserreinigung in Kläranlagen. Die Herstellung von Bier, Wein und anderen derartigen Produkten findet ebenfalls in Bioreaktoren statt. In Bioreaktoren werden unterschiedlichste Organismen für verschiedene Zwecke kultiviert. Ein Bioreaktor kann deshalb unterschiedlich ausgeführt sein. Er kann als Rührkesselreaktor ausgeführt sein, der ein Volumen von wenigen Millilitern bis hunderten Litern haben kann und mit Nährlösung gefüllt werden kann. Er kann auch als Festbettreaktor, Photobioreaktor verwendet werden bzw. ausgebildet sein. Ein Bioreaktor kann Teil eines Bioreaktor-Systems sein, vorzugsweise eines parallelen Bioreaktor-Systems. In einem solchen parallelen Bioreaktor-System wird eine Vielzahl von Bioreaktoren parallel betrieben und mit höherer Präzision kontrolliert. Ein Bioreaktor, insbesondere dessen Behandlungseinrichtung, weist insbesondere eine Rühreinrichtung zum Rühren der im Reaktorbehälter enthaltenen Probe, insbesondere des Nährmediums auf. Ein Bioreaktor, insbesondere dessen Behandlungseinrichtung, weist insbesondere eine Pumpeinrichtung zum Pumpen der vorzugsweise als Nährmedium ausgeführten Laborprobe auf. Ein Bioreaktor, insbesondere dessen Behandlungseinrichtung, weist insbesondere eine Einstelleinrichtung für die Einstellung eines Gasgehalts im Reaktorbehälter, insbesondere des Gehalts an CO₂ und/oder O₂ bzw. an Gelöstsauerstoff (DO) auf. Ein Bioreaktor, insbesondere dessen Behandlungseinrichtung, weist insbesondere eine Einstelleinrichtung für die Einstellung, insbesondere Regelung, eines pH-Werts in der Probe im Reaktorbehälter auf. Die gerätegesteuerte Behandlung der mindestens einen Laborprobe entspricht bei einem Bioreaktor insbesondere einer Nährmediumsbehandlung, der die mindestens eine vorzugsweise als Nährmedium ausgeführte Probe unterzogen wird. Mögliche Parameter, insbesondere Programmparameter, insbesondere Nutzerparameter, die zur Beeinflussung einer Nährmediumsbehandlung verwendet werden, definieren insbesondere die Temperatur des Nährmediums im Reaktorbehälter, und/oder die Geschwindigkeit der Rühreinrichtung, insbesondere Rotationsgeschwindigkeit und/oder die Pumpgeschwindigkeit bzw. Dosiergeschwindigkeit und/oder einen Gasgehalt im Nährmedium, insbesondere CO₂ und/oder O₂ bzw. Gelöstsauerstoff (DO) und/oder den pH-Wert des Nährmediums und/oder mindestens einen Ablaufparameter, der den Ablauf, insbesondere die Reihenfolge, eines aus mehreren Schritten bestehenden Nährmediumsbehandlungsprogramms beeinflusst oder definiert.

Eine Sicherheitswerkbank dient insbesondere zur sicheren Lagerung oder Aufbewahrung von Gefahrenstoffen, insbesondere zur Erfüllung einer biologischen Schutzstufe. Diese Stufen sind insbesondere in der EU-Richtlinie 2000/54/EG über den Schutz der Arbeitnehmer gegen Gefährdung durch biologische Arbeitsstoffe bei der Arbeit und in der Biostoffverordnung in Deutschland normiert. Eine Sicherheitswerkbank soll verhindern, dass im Falle der Gefahrenentstehung bei in einer Sicherheitswerkbank gelagerten Laborproben die Umwelt gefährdet wird. Die Sicherheit wird insbesondere dadurch gewährleistet, dass die im Aufnahmebereich der Sicherheitswerkbank enthaltene Atmosphäre ausgetauscht und insbesondere gefiltert wird. Dabei wird insbesondere diese Atmosphäre von einer Fördereinrichtung durch den Aufnahmebereich gefördert und durch einen Filter bewegt, der die Atmosphäre filtert, insbesondere von Gefahrstoffen reinigt. Die Sicherheitswerkbank, insbesondere deren Behandlungseinrichtung, weist insbesondere eine Fördereinrichtung zum Befördern von Atmosphärengas auf, weist insbesondere eine Zeitgebereinrichtung zur Messung einer Filterbetriebsdauer und einer Lüfterbetriebsdauer auf und/oder weist insbesondere eine Messeinrichtung zur Messung einer geförderten Menge an Atmosphärengas auf. Die gerätegesteuerte Behandlung der mindestens einen Laborprobe entspricht bei einer Sicherheitswerkbank insbesondere einer Atmosphärengasbehandlung zur Behandlung des Atmosphärengases, in dem die mindestens eine Probe gelagert ist. Mögliche Parameter, insbesondere Programmparameter, insbesondere Nutzerparameter, die zur Beeinflussung einer Atmosphärengasbehandlung verwendet werden, definieren insbesondere die Temperatur des Atmosphärengases im Aufnahmebereich, und/oder die Strömungsgeschwindigkeit des von der Fördereinrichtung beförderten Atmosphärengases, die geförderte Luftmenge, die Filterbetriebsdauer, und/oder die Lüfterbetriebsdauer.

Ein Probenplatten-Lesegerät, auch bezeichnet als "plate reader" oder "micropiate reader", ist ein Laborgerät zum Nachweis biologischer, chemischer oder physikalischer Ereignisse von Proben in Mikrotiterplatten. Sie werden in der Forschung in vielerlei Hinsicht verwendet, Wirkstoffforschung, Bioassay Validierung, Qualitätskontrolle und Fertigungsprozesse in der pharmazeutischen und biotechnologischen Industrie und akademischen Organisationen. Das Probenplatten-Lesegerät kann insbesondere mindestens eine Lichtquelle oder Strahlungsquelle aufweisen, kann mindestens einen Photodetektor aufweisen, kann eine Temperaturkontrolleinrichtung zu Temperierung der Proben bzw. der Probenplatten aufweisen, kann einen Zeitgeber aufweisen. Probenreaktionen können in 6-1536 Well-Format Mikrotiterplatten getestet werden. Das häufigste Format für Probenplatten, insbesondere Mikrotiterplatten, das in akademischen Forschungslabors oder klinisch-diagnostische Laboratorien verwendet wird, ist eine 96-well plate (eine 8 mal 12 Matrix) mit einem typischen Einzelvolumen zwischen 100 und 200 µl pro Well. Mikrotiterplatten mit höherer Dichte (384 - oder 1536-Well-Mikroplatten) werden typischerweise für Screening-Anwendungen verwendet, wenn Durchsatz (Anzahl der pro Tag verarbeiteten Proben) und Assay-Kosten pro Probe zu kritischen Parametern werden, mit einem typischen Assay-Volumen zwischen 5 und 50 µl pro Well. Die Behandlung ist insbesondere eine optische Messung der Mikrotiterplatte, insbesondere die Messung einer Absorption, Fluoreszenz-Intensität, Lumineszenz, zeitaufgelöste Fluoreszenz, und/oder Fluoreszenzpolarisation. Mögliche Parameter, insbesondere Programmparameter, insbesondere Nutzerparameter, die zur Beeinflussung einer Messung verwendet werden, definieren z.B. die Intensität der Lichtquelle, die Sensitivität eines Photodetektors, eine Zeitdauer und/oder eine Temperatur.

Ein Laborautomat zur Behandlung von fluiden Proben, insbesondere ein Pipettierautomat, dient der programmgesteuerten Behandlung dieser Proben. Ein Laborautomat kann ein Laborgerät sein oder mindestens ein Laborgerät der vorgenannten Art aufweisen und/oder kann zur Durchführung mindestens einer, mehrerer oder aller der von diesem vorgenannten Laborgerät ausführbaren Behandlungen ausgebildet sein. Ein Laborautomat weist die Behandlungseinrichtung zur automatischen, programmgesteuerten Behandlung der mindestens einen Laborprobe auf, wobei die Behandlung unter Verwendung von mehreren Programmparametern, die zumindest teilweise vom Benutzer gewählt werden, gesteuert wird. Dabei kann die Probe vom Laborautomaten bzw. einer Behandlungseinrichtung des Laborautomaten beispielsweise bewegt und/oder transportiert werden. Die Bewegung kann durch Transport in beweglichen Probenbehältern oder durch die Leitung in Schlauchsystemen, Kapillaren oder Pipettenspitzen erfolgen. Dabei werden flüssige Proben insbesondere durch Ansaugen, also Pipettieren, oder allgemeiner durch Anlegen von Druckdifferenzen transportiert. Durch eine Behandlung der Probe kann eine Probe z.B. geteilt oder verdünnt werden. Die Inhaltsstoffe einer Probe können analysiert werden oder es können, z.B. durch eine chemische Reaktion, neue Inhaltsstoffe hergestellt werden, insbesondere unter Verwendung der Probe. Insbesondere im Zusammenhang mit der Bearbeitung und Analyse von DNA oder RNA oder deren Bestandteilen sind Laborautomaten hilfreich, um eine Fülle von Informationen innerhalb einer geeigneten Zeitspanne zu gewinnen oder viele solcher Proben zu analysieren. Diese Behandlungseinrichtung eines Laborautomaten weist meist eine Arbeitsfläche mit Arbeitsstationen auf, an denen Proben in verschiedener Weise bearbeitet oder gelagert werden können. Für den Transport von z.B. flüssigen Proben zwischen verschiedenen Positionen, insbesondere Probenbehältern, weist die Behandlungseinrichtung meist eine gerätegesteuerte Bewegungsvorrichtung und eine gerätegesteuerte Fluid-Transfer-Einrichtung auf, die z.B. ein Pipettiersystem aufweisen kann. Sowohl der Transport der Proben als auch deren Behandlung an den verschiedenen Stationen lässt sich gerätegesteuert durchführen, insbesondere programmgesteuert durchführen. Die Behandlung erfolgt dann vorzugsweise zumindest teilweise oder vollständig automatisiert.

Vorzugsweise kann der Benutzer des Laborautomaten die Art der Behandlung der Probe festlegen. Eine solche Behandlungsart kann insbesondere dienen:
der Nukleinsäureaufreinigung, insbesondere
   - "MagSep Blood gDNA": Aufreinigung von genomischer DNA aus Vollblut, insbesondere unter Verwendung des Eppendorf ^{®} MagSep Blood gDNA Kits;
   - "MagSep Tissue gDNA": Aufreinigung von genomischer DNA aus lebendem Gewebe, insbesondere unter Verwendung des Eppendorf ^{®} MagSep Tissue gDNA Kits;
   - "MagSep Viral DANN/RNA": Aufreinigung von viraler RNA oder DNA aus zellfreien Körperflüssigkeiten, insbesondere unter Verwendung des Eppendorf ^{®} MagSep Viral DNA/RNA Kits;
und PCR-Anwendungen, insbesondere
   - "Compose Mastermix";
   - "Normalize Concentrations";
   - "Create Dilution Series";
   - "Setup Reactions".

Ein Laborgerät, insbesondere der Laborautomat, ist vorzugsweise so ausgebildet, dass die Steuerung der Behandlung der mindestens einen Laborprobe automatisch unter Verwendung der erfassten Programmparameter erfolgen kann. Ein Laborgerät, insbesondere der Laborautomat, insbesondere dessen Steuerungsprogramm ist vorzugsweise so ausgebildet, dass die vom Benutzer vorgenommenen Eingaben, insbesondere der mindestens eine Wert mindestens eines Programmparameters, dazu verwendet werden, um gegebenenfalls weitere, erforderliche Programmparameter automatisch zu ermitteln, insbesondere durch Berechnung oder durch Vergleich mit Daten in einer Datenbank des Laborautomaten. Insbesondere werden vorzugsweise die zur Durchführung der Behandlung im Einzelnen vorzugsweise zu verwendenden Steuerparameter automatisch bestimmt. Durch diese Maßnahmen wird die Bedienung des Laborgerätes komfortabel, der Benutzer erspart sich insbesondere das Entwerfen eines Programmcodes, da diese Schritte insbesondere automatisch vom Laborgerät durchgeführt werden. In einer bevorzugten Ausgestaltung der Erfindung werden vom Benutzer nur die Eingaben abgefordert, die direkt mit der durchzuführenden Behandlung der Proben zu tun haben. Das sind häufig dieselben Angaben, die auch für eine manuelle Durchführung der Behandlung notwendig wären und dem Benutzer geläufig sind. Dagegen müssen solche Parameter, die die Steuerung des Laborgerätes betreffen, insbesondere die Steuerparameter, nicht im Einzelnen festgelegt werden, da diese vorzugsweise automatisch festgelegt werden. Steuerparameter sind die zur Steuerung der technischen Bestandteile der Behandlungseinrichtung im Einzelnen erforderlichen Parameter. Steuerparameter können Programmparameter sein oder können daraus für die technische Umsetzung abgeleitete Parameter sein, insbesondere automatisch bestimmte Parameter sein.

Vorzugsweise wählt ein Laborgerät, insbesondere der Laborautomat, ausgehend von der vom Benutzer gewählten Behandlungsart, automatisch den passenden Satz von Programmparametern aus, dessen benutzerseitig erforderliche Programmparameter dann in den Schritten (b) und (c) vom Benutzer abgefragt wird. Der Programmparametersatz kann einerseits die benutzerseitig erforderlichen Programmparameter enthalten und kann andererseits weitere Programmparameter enthalten. Diese weiteren Programmparameter können in Abhängigkeit von der gewählten Behandlungsart automatisch festgelegt werden oder können in Abhängigkeit von mindestens einem, oder allen, vom Benutzer eingegebenen Programmparametern automatisch festgelegt werden und/oder können in der Speichereinrichtung gespeichert sein. Die gespeicherten Parametersätze sind - oder werden vom Laborautomaten - vorzugsweise für die Behandlungsart optimiert, so dass der Benutzer vorzugsweise kein Spezialwissen zur Optimierung der Parameter benötigt. Aus dem Programmparametersatz werden die Steuerparameter abgeleitet, die zur Durchführung der konkreten Behandlung mittels der Behandlungseinrichtung notwendig sind.

Für eine Behandlungsart ist vorzugsweise ein Programmparametersatz von für diese Behandlungsart spezifischen Programmparametern definiert. Die Programmparameter dieses Programmparametersatzes können insbesondere die für die Behandlung zu verwendenden Zubehörteile, z.B. Probenbehältnis, Transportbehältnis und/oder die zu verwendenden Tools und/oder weiteres Verbrauchsmaterial definieren.

Die Zuordnung von Programmparametersatz und Behandlungsart ist in der Speichereinrichtung des Laborgerätes, insbesondere des Laborautomaten, gespeichert. Vorzugsweise ist der Laborautomat dazu ausgebildet, dass der Benutzer weiterer solcher Zuordnungen im Laborgerät speichern und/oder verwenden kann. Durch diese Zuordnung, in Kombination mit der übersichtlichen und gut strukturierten Abfrage der Programmparameter wird die Bedienung der Laborautomaten besonders effizient. Diese Zuordnung erfolgt vorzugsweise durch die Verwendung eines oder mehrere Programmmodule, wobei jeweils ein Programmmodul auf eine bestimmte Anwendung zugeschnitten ist:
Vorzugsweise weist der Laborautomat mindestens ein Programmmodul auf, wobei ein vorbestimmtes Programmmodul der Steuerung einer vorbestimmten Laboraufgabe zur Behandlung von Laborproben dient.

Vorzugsweise ist der mindestens eine Programmparameter, insbesondere der benutzerseitig erforderliche Programmparameter, aus der folgenden Menge von physikalischen Größen ausgewählt, die zur Behandlung einer Laborprobe mittels der Behandlungseinrichtung relevant sind: Probenanzahl, Verdünnungsfaktor, Zielvolumen, Position der Proben in einem Probengefäßhalter oder in einer Mikrotiterplatte, Probentemperatur, Zeitpunkte und/oder Zeitdifferenzen, Temperaturen oder Temperaturdifferenzen, Änderungsraten solcher Parameter, etc.

Vorzugsweise enthält das Steuerprogramm ferner Anweisungen, um folgenden Schritt auszuführen, insbesondere ist die Steuereinrichtung des Laborgeräts zur Durchführung des folgenden Schritts eingerichtet:
Erzeugen eines Methodenprogramms unter Verwendung der vom Benutzer eingegebenen Programmparameter, und Speichern des Methodenprogramms in der Speichereinrichtung, wobei das Methodenprogramm vom Benutzer editierbar ist. Dadurch wird die Verwendung des Laborgerätes, insbesondere des Laborautomaten, noch flexibler.

Der Laborautomat kann so verändert werden, dass damit weitere Behandlungsarten durchgeführt werden können. Dies kann dadurch geschehen, dass die dafür notwendigen Dateien und/oder Programme oder Programmbestandteile, insbesondere ein der Behandlungsart zugeordnetes Programmmodul, nachträglich in den Laborautomaten, insbesondere dessen Speichereinrichtung, übertragen werden.

Eine Laborprobe ist eine Probe, die in einem Labor behandelt werden kann. Anstelle des Begriffs Laborprobe wird bei der Beschreibung der Erfindung auch der Begriff "Probe" verwendet. Die Probe kann ein Fluid sein. Die Probe kann flüssig, gelartig, pulverförmig oder ein Festkörper sein oder solche Phasen aufweisen. Die Probe kann ein Gemisch aus solchen Phasen sein, insbesondere ein Flüssigkeitsgemisch, eine Lösung, eine Suspension, z.B. eine Zellsuspension, eine Emulsion oder Dispersion. Eine Lösung ist ein homogenes Gemisch aus mindestens zwei Stoffen. Eine flüssige Probe kann eine solche sein, die üblicherweise in einem biologischen, chemischen, medizinischen Labor gehandhabt wird. Eine flüssige Probe kann eine Analysenprobe sein, ein Reagenz, ein Medium, ein Puffer etc. Eine Lösung weist einen oder mehrere gelöste feste, flüssige oder gasförmige Stoffe (Solute) auf und weist ferner ein vorzugsweise flüssiges Lösungsmittel (Solvens) auf, das insbesondere den größeren Anteil oder größten Anteil des Volumens auf, das die Lösung bildet. Das Lösungsmittel kann selbst eine Lösung sein.

Die Behandlung einer Laborprobe(n) kann einen oder mehrere der nachfolgend genannten Vorgänge, insbesondere gleichzeitig oder nacheinander, beinhalten:
- Transport der Laborprobe, insbesondere durch eine Transporteinrichtung, unter Wirkung der Gravitation und/oder einer durch den Laborautomaten bewirkten Kraft;
- berührungslose (nicht-invasive), physikalische Behandlung der Probe, insbesondere thermische Behandlung, insbesondere Erwärmen und/oder Kühlen, insbesondere geregeltes Temperieren der Probe; oder Gefrieren oder Auftauen der Probe oder sonstiges thermisches Herbeiführen einer Phasenänderung der Probe, z.B. Verdampfen, Kondensieren etc; magnetische Behandlung der Probe; optische Behandlung der Probe, insbesondere Bestrahlen der Probe mit Strahlung, insbesondere Licht, insbesondere sichtbare Licht. Infrarotlicht oder UV-Licht oder Detektion von solcher Strahlung, insbesondere Fluoreszenzlicht, aus dieser Probe; magnetische Behandlung einer Probe mit magnetischen Bestandteilen, insbesondere magnetische Separation von magnetischen Bestandteilen, insbesondere "magnetic beads", von einer fluiden Phase der Probe; Bewegen der Probe, also Durchführen einer mechanischen Behandlung der Probe, insbesondere schütteln, rotieren, oszillieren, vibrieren, zentrifugieren, akustische Behandlung, insbesondere mit Ultraschall, jeweils z.B. zum Zwecke des Mischens der Probe oder des Separierens von Bestandteilen innerhalb der Probe oder zum Transportieren der magnetischen Bestanteile aus der Probe heraus oder in die Probe hinein;
- invasive physikalische Behandlung der Probe, also Durchführen einer mechanischen Behandlung der Probe: Einbringen von Rührwerkzeug, z.B. Rührstab oder magnetischer Rührfisch in die Probe und Rühren, Einbringen einer Sonotrode für akustische oder Ultraschalbehandlung, Einbringen von Transportmitteln, insbesondere Transportbehältnissen in die Probe, z.B. Dispenserspitze oder Pipettenspitze, oder Hohlnadel oder Schlauch; Zugabe von anderen Hilfsmitteln in die Probe;
- chemische, biochemische oder biomedizinische Behandlung der Probe: Zugabe von chemischen (z.B. Reaktant, Reagenz, Solvens, Solut) biochemischen (z.B. biochemische Makromoleküle, z.B. DNA, DNA-Bestandteile; pharmazeutische Wirkstoffe), oder biomedizinischen (Blut, Serum, Zellmedium) Stoffen;
- Lagerung der Probe, insbesondere für einen programmgesteuert definierten Zeitraum, insbesondere unter bestimmten physikalische Bedingungen, z.B. bei bestimmter Temperatur, Temperaturen oder Temperaturwechseln, insbesondere wiederholten Temperaturwechseln, z.B. zyklisch und/oder periodisch wiederholten Temperaturwechselnund/oder Einstellen eines Umgebungsdrucks, z.B. Anlegen eines Überdrucks oder eines Unterdrucks, insbesondere eines Vakuums, und/oder Einstellen einer definierten Umgebungsatmosphäre, z.B. ein Schutzgas oder eine bestimmte Luftfeuchtigkeit, unter bestimmten Strahlungsbedingungen, z.B. abgeschirmt gegen sichtbares Licht, im Dunkeln oder unter definierter Bestrahlung;
- Messung oder Analyse der Probe, insbesondere Analyse durch eine nicht-invasive und/oder invasive Behandlung der Probe, insbesondere um mindestens eine oder mehrere chemische, physikalische, biochemische und/oder medizinische Eigenschaften der Probe zu messen; insbesondere Zählen von Zellen mittels Cell-Counter;
- Bearbeitung der Probe, insbesondere Änderungen mindestens einer Eigenschaft der Probe, insbesondere mittels nicht-invasiver und/oder invasiver Behandlung der Probe.

Diese Behandlung erfolgt insbesondere programmgesteuert, unter Verwendung mindestens eines Programmparameters.

Diese Behandlung erfolgt insbesondere gemäß mindestens einem Steuerparameter, der die Behandlung der Laborprobe mittels der Behandlungseinrichtung bestimmt. Ein Steuerparameter kann einen Zeitraum festlegen, einen Zeitpunkt, ein bestimmtes Probenvolumen und/oder Dosiervolumen, eine bestimmte Probentemperatur, etc.. Ein Steuerparameter kann die automatische Verwendung eines bestimmten Transportkopfes, eines bestimmten Typs eines Transportbehältnisses, eines bestimmten Typs von Probebehältnissen, einer oder mehrerer individuellen Proben oder von bestimmten Positionen dieser Komponenten im Arbeitsbereich betreffen. Ein Steuerparameter kann die Behandlung einer individuellen Probe betreffen oder die Behandlung mehrerer oder vieler Proben.

Vorzugsweise wird ein Steuerparameter in Abhängigkeit von mindestens einem Programmparameter automatisch vom Laborgerät, insbesondere Laborautomat, ausgewählt, insbesondere automatisch in Abhängigkeit von den vom Benutzer ausgewählten Programmparametern automatisch ausgewählt. Dadurch hat der Benutzer den Vorteil, dass er nicht alle Steuerparameter einzeln bestimmen muss. Der Benutzer muss keine Kenntnisse über die Programmierung des Laborgeräts besitzen. Vielmehr erfolgt die Auswahl der für die Behandlung notwendigen Steuerparameter mittels der vom Benutzer eingegebenen Programmparameter. Dadurch ist die Verwendung des Laborgeräts besonders komfortabel.

Ein Steuerparameter kann auch einem Programmparameter entsprechen.

Der Transport einer Probe kann ein Transport aus einem Probenbehältnis in ein Transportbehältnis und/oder aus dem Transportbehältnis in ein Probenbehältnis oder einen sonstigen Zielort sein. Dieser Transport erfolgt insbesondere programmgesteuert, unter Verwendung mindestens eines Programmparameters.

Das Transportbehältnis kann z.B. ein Dispenserbehältnis sein, das einen beweglichen Kolben und eine Einlass-/Auslassöffnung aufweist. Der Kolben erzeugt einen Unterdruck bzw. Überdruck im Dispenserbehältnis und saugt so die Probe in das Behältnis hinein oder gibt sie wieder ab. Dieser Vorgang folgt dem Verdrängerprinzip, d.h. die zu bewegende, meist flüssige und somit inkompressibele Probe wird zwangsbewegt, indem das zuvor von der Probe eingenommene Volumen vom Kolben bewegt wird. Der Kolben wird in der Regel von einer Bewegungseinrichtung bewegt, insbesondere programmgesteuert bewegt, die dem Laborautomaten zugeordnet ist.

Das Transportbehältnis kann ferner eine Pipettenspitze sein. Eine Pipettenspitze weist eine Einlass-/Auslassöffnung auf, und eine zweite Öffnung. Die zweite Öffnung wird an eine Ansaugeinrichtung gekoppelt, so dass durch einen Unterdruck eine flüssige Probe aus einem Probenbehältnis in das Transportbehältnis gesaugt (pipettiert) werden kann. Die Abgabe der Probe erfolgt durch Ventilieren des Ansaugbereichs, mittels Gravitation und/oder über einen Überdruck, der z. B. über die zweite Öffnung im Pipettenspitze erzeugt wird.

Das Transportbehältnis besteht vorzugsweise teilweise oder vollständig aus Kunststoff. Es ist vorzugsweise ein Verbrauchsartikel, der typischerweise nur für eine Behandlung oder eine geringe Zahl von Behandlungsschritten der Probe verwendet wird. Das Transportbehältnis kann aber auch teilweise oder vollständig aus einem anderen Material bestehen.

Der Transport einer Probe kann ein Transport der Probe von einer Ausgangsposition in eine Zielposition sein. Die Ausgangsposition kann vorliegen, wenn die Probe in einem ersten Probenbehältnis angeordnet ist und die Zielposition dieser Probe kann ihre Position in einem zweiten Probenbehältnis sein, in das die Probe übertragen wird. Diese Art des Transports wird vorliegend auch als Probentransfer oder Transfer bezeichnet. Ein Probentransfer wird in der Praxis meist durchgeführt, um eine Probe aus einem Vorratsbehältnis, in dem z.B. die Probe gelagert war und/oder das z.B. eine größere Menge der Probe enthalten kann, in ein zweites Probenbehältnis zu übertragen, in dem die Probe weiter behandelt wird. Dieser Transport erfolgt insbesondere programmgesteuert, unter Verwendung mindestens eines Programmparameters.

Das Transportbehältnis ist vorzugsweise mit einer Transporteinrichtung des Laborautomaten verbunden oder verbindbar.

Ein Probenbehältnis kann ein Einzelbehältnis sein, in dem nur eine einzige Probe enthalten ist oder kann ein Mehrfachbehältnis sein, in dem mehrere Einzelbehältnisse miteinander verbunden angeordnet sind.

Ein Einzelbehältnis kann ein offenes Behältnis oder ein verschliessbares Behältnis sein. Bei einem verschliessbaren Behältnis kann ein Deckelelement, insbesondere eine Verschlusskappe, vorgesehen sein. Das Deckelelement kann fest mit dem Behältnis verbunden sein, z.B. als Klappdeckel oder Klappverschlusskappe, oder kann als separate Komponente verwendet werden.

Vorzugsweise sind bei einem Mehrfachbehältnis die mehreren Einzelbehältnisse in festen Positionen zueinander, insbesondere entsprechend den Kreuzungspunkten eines Gittermusters angeordnet. Dies vereinfacht die automatisierte Ansteuerung der Positionen und insbesondere die individuellen Adressierung von Proben. Ein Mehrfachbehältnis kann als Plattenelement ausgebildet sein, bei dem die Einzelbehältnisse so verbunden sind, dass sie eine plattenförmige Anordnung bilden. Die Einzelbehältnisse können als Vertiefungen in einer Platte ausgebildet sein oder können über Stegelemente miteinander verbunden sein. Das Plattenelement kann ein Rahmenelement aufweisen, in dem die Einzelbehältnisse gehalten werden. Diese Verbindungen von Komponenten können integrale Verbindungen sein, d.h. stoffschlüssige Verbindungen und/oder durch einen gemeinsamen Spritzgussprozess erzeugte Verbindungen sein, oder können kraftschlüssig (englisch "force-fit") und/oder formschlüssig (englisch "form-fit") erzeugt sein. Das Plattenelement kann insbesondere eine Mikrotiterplatte sein.

Mehrfachbehältnisse können eine Mehrzahl (von 2 bis 10) Einzelbehältnissen aufweisen. Sie können ferner eine Vielzahl (größer 10) aufweisen, typischereeise 12, 16, 24, 32, 48, 64, 96, 384, 1536 Einzelbehältnisse. Das Mehrfachbehältnis kann insbesondere eine Mikrotiterplatte sein. Eine Mikrotiterplatte kann gemäß einem oder mehreren Industriestandard(s) ausgebildet sein, insbesondere der Industrienormen ANSI/SBS 1-2004, ANSI/SBS 2-2004, ANSI/SBS 3-2004, ANSI/SBS 4-2004.

Das maximale Probenvolumen, das von einem Transportbehältnis oder einem Probenbehältnis aufnehmbar ist, liegt typischer Weise zwischen 0,01 ml und 100 ml, insbesondere bei 10 - 100 µl, 100 - 500 µl, 0,5 - 5 ml, 5-25 ml, 25-50 ml, 50-100 ml, abhängig vom Typ des gewählten Transportbehältnis oder Probengefäßes.

Ein Probenbehältnis kann einen Informationsbereich aufweisen, der Informationen über das Probenbehältnis oder über dessen Inhalt aufweisen kann. Der Informationsbereich kann codierte Information aufweisen, z.B. einen Barcode oder QR-Code oder einen RFID-Chip oder eine anders codierte Information. Die Information kann Information zur Identifizierung der Probe und/oder eines Probenbehältnisses aufweisen. Der Laborautomat kann eine Informationsleseeinrichtung aufweisen, um diese Information zu lesen und dies vorzugsweise der Steuereinrichtung zur Verfügung zu stellen.

Das Probenbehältnis besteht vorzugsweise teilweise oder vollständig aus Kunststoff. Es ist vorzugsweise ein Verbrauchsartikel, der typischer Weise nur für eine Behandlung oder eine geringe Zahl von Behandlungsschritten der Probe verwendet wird. Das Probenbehältnis kann aber auch teilweise oder vollständig aus einem anderen Material bestehen.

Das Probenbehältnis ist vorzugsweise mit einer Transporteinrichtung des Laborautomaten transportierbar.

Das Laborgerät, insbesondere der Laborautomat, ist vorzugsweise dazu ausgebildet eine Vielzahl von Proben nacheinander und/oder parallel zu behandeln. Insbesondere ist ein Laborgerät, insbesondere der Laborautomat, vorzugsweise dazu ausgebildet eine Vielzahl von Probengefäßen, insbesondere Einzelbehältnisse und/oder Mehrfachbehältnisse, programmgesteuert zu behandeln, insbesondere zu transportieren, zu entleeren und/oder zu befüllen.

Vorzugsweise weist ein Laborgerät, insbesondere der Laborautomat, genau einen Arbeitsbereich auf. Ein solches Laborgerät, insbesondere Laborautomat, ist kompakt und kann sich insbesondere zur Verwendung auf einem Labortisch eignen, wobei er dann insbesondere auch als Tischgerät bezeichnet wird. Der Tisch kann z.B. die Workbench eines chemischen, biochemischen oder biomedizinischen Labors sein. Das Laborgerät, insbesondere der Laborautomat, kann auch zur Aufstellung in einem solchen Labor ausgebildet sein. Ein Laborgerät, insbesondere Laborautomat, mit einem Arbeitsbereich kann ferner als unabhängig arbeitendes Gerät eines solchen Labors ausgebildet sein oder kann in einen Geräteverbund eingebunden sein/werden.

Das Laborgerät, insbesondere der Laborautomat, kann auch als Laborstraße ausgebildet sein, bei dem mehrere Arbeitsbereiche so benachbart angeordnet sind, dass mittels eine Transportvorrichtung eine, mehrere oder eine Vielzahl von Proben nacheinander und/oder parallel zwischen den Arbeitsbereichen transportiert werden können. Ein Arbeitsbereich einer Laborstraße ist vorzugsweise dazu ausgebildet, dass eine bestimmte Laboraufgabe, betreffend meistens die parallele und/oder sequentielle Behandlung einer Vielzahl von Proben, durchgeführt wird. Durch diese Spezialisierung jedes Arbeitsbereichs wird ein hoher Arbeitsdurchsatz der Laborstrasse erreicht. Zur Durchführung einer solchen, bestimmten Aufgabe kann vorgesehen sein, dass in jedem Arbeitsbereich nur eine Art der Behandlung mindestens einer Probe, oder nur wenige Arten der Behandlung, z.B. zwei bis zehn Behandlungsarten durchgeführt werden. An jeder Arbeitsstation kann zur Durchführung einer Behandlung eine Behandlungseinrichtung angeordnet sein, die für ein bestimmtes Laborgerät, wie im Rahmen der Beschreibung der Erfindung beschrieben, charakteristisch ist. Die Transportvorrichtung kann ein Schienensystem aufweisen und/oder eine Robotereinrichtung zum programmgesteuerten Bewegen von Proben bzw. Probenbehältnissen.

Ein Laborgerät, insbesondere Laborautomat, kann mit einem LIMS verbunden oder verbindbar sein. LIMS steht für Labor-Informations- und Management-System. Ein LIMS ist in bekannter Weise ein Softwaresystem, das die Datenverarbeitung im chemischen, physikalischen, biologischen, medizinischen automatischen oder teilautomatischen Labor betrifft. Solche Daten können aus Messungen der Proben stammen, und/oder können die Steuerung der Bearbeitung der Daten betreffen. Ein LIMS dient vorzugsweise der Messwerterfassung und der Messwertauswertung. LIMS wird dazu verwendet, um den Arbeitsdurchsatz in einem Labor zu steigern und/oder die Effizienz der Behandlung von Laborproben zu optimieren.

Ein Werkzeugelement kann z.B. ein Transportkopf für den Fluidtransfer sein, insbesondere ein Pipettierkopf, der einen Verbindungsabschnitt zur Verbindung von einer Pipettenspitze (Einkanalpipettierkopf) oder zur Verbindung von mehreren Pipettenspitzen (Mehrkanalpipettierkopf) mit dem Pipettierkopf aufweisen kann. Über mindestens einen druck- und gasdichten Kanal, der mit dem Pipettierkopf verbunden ist, lässt sich Flüssigkeit in die mindestens eine Pipettenspitze saugen, wenn diese mit dem Verbindungsabschnitt verbunden ist. Dieses Pipettieren erfolgt beim Laborautomaten insbesondere programmgesteuert, insbesondere beeinflusst durch mindestens einen Programmparameter. Der Transportkopf kann auch ein Dispensierkopf sein, der mindestens eine Bewegungseinrichtung zur Bewegung eines Kolbens der Dispenserspitze aufweist. Die Bewegung der Bewegungseinrichtung erfolgt beim Laborautomaten insbesondere programmgesteuert, insbesondere beeinflusst durch mindestens einen Programmparameter. Der Transportkopf kann zur Flüssigkeitsdosierung dienen, insbesondere für die Dosierung in unterschiedlichen Bereichen; ein Transportkopf kann zur Dosierung einer flüssigen Probe mit einem Volumen ausgebildet sein, das aus einem für diesen Transportkopf spezifischen Volumenbereich ausgewählt sein kann: z.B. 1 - 50 µL, oder 20 -300 µL,oder 50 -1000 µL, ("I" und "L" stehen jeweils als Abkürzung für Liter). Ein Transportkopf kann als Einkanalkopf ausgebildet sein, bei dem nur eine Probe transportiert wird oder kann als Mehrkanal, insbesondere Achtkanal- oder 12-Kanal ausgebildet sein, bei dem mehrere Proben parallel bearbeitet bzw. transportiert werden. Es sind vorzugsweise spezifische Transportbehältnisse vorgesehen, die in Abhängigkeit vom jeweiligen Typ von Transportkopf, insbesondere gemäß dem entsprechenden Volumenbereich, eingesetzt werden können.

Ein Werkzeugelement kann z.B. ein Transportkopf für den Transport von Gegenständen sein, z.B. eine Trage- und/oder Greifwerkzeug zum Tragen und/oder Greifen eines Gegenstands. Ein Tragewerkzeug kann ein Befestigungsabschnitt zum lösbaren Befestigen des Gegenstands am Tragewerkzeug aufweisen, z.B. durch kraftschlüssiges und/oder formschlüssiges und/oder magnetisches Verbinden des Gegenstands mit dem Tragewerkzeug. Auf diese Weise können innerhalb der Arbeitsfläche oder zwischen mehreren Arbeitsbereichen und/oder Arbeitsflächen.

Ein Werkzeugelement kann ferner eine Behandlungseinheit sein, z.B. zur Durchführung einer thermischen, akustischen, optischen und/oder mechanischen Behandlung mindestens einer Probe.

Der Laborautomat kann eine Informationsleseeinrichtung aufweisen, um eine Information über eine Probe und/oder ein Probenbehältnis und/oder eine Behandlungsanweisung für diese Probe und/oder dieses Probenbehältnis zu lesen und, vorzugsweise, der Steuereinrichtung des Laborautomaten zur Verfügung zu stellen.

Der Laborautomat weist vorzugsweise mindestens eine Zeitgebereinrichtung und/oder vorzugsweise eine Zeitnehmereinrichtung auf, um die zeitabhängige Behandlung der Proben zu ermöglichen. Die zeitabhängige Behandlung erfolgt vorzugsweise programmgesteuert, und insbesondere gesteuert durch mindestens einen Programmparameter.

In einer bevorzugten Gestaltung des erfindungsgemäßen Laborautomaten ist dieser dazu ausgebildet in Abhängigkeit von der vom Benutzer gewählten Behandlungsart und der vom Benutzer eingegebenen Programmparameter automatisch eine oder mehrere der nachfolgend genannten Komponenten automatisch zur Verwendung bei der programmgesteuerten Behandlung auszuwählen:
- mindestens ein geeignetes Probenbehältnis, insbesondere geeignet zur Aufnahme mehrere Proben, die gemeinsam bearbeitet werden sollen, die z.B. gemischt werden sollen oder zwischen denen eine chemische Reaktion oder biochemische, biologische oder biomedizinische Wechselwirkung auftreten soll;
- mindestens ein geeignetes Transportbehältnis, insbesondere Pipettenspitze und/oder Dispenserspitze;
- mindestens einen geeigneten Transportkopf, mit dem das vorzugsweise automatisch gewählte Transportbehältnis verbindbar ist,
- mindestens ein geeignetes Werkzeugelement, das der Durchführung der gewünschten Behandlung dient.

Vorzugsweise ist der erfindungsgemäße Laborautomaten dazu ausgebildet in Abhängigkeit von der vom Benutzer gewählten Behandlungsart und der vom Benutzer eingegebenen Programmparameter automatisch eine oder mehrere der nachfolgend genannten Steuerparameter automatisch zur Verwendung bei der programmgesteuerten Behandlung auszuwählen:
- mindestens ein Zeitraum, zu dem ein bestimmter Arbeitsschritt der Behandlung durchgeführt wird;
- mindestens ein Probenvolumen und/oder Dosiervolumen;
- mindestens eine Arbeitsposition der mindestens einen Arbeitsfläche;
- Bewegungsparameter zur Festlegung des für die gewünschte Behandlung der Probe erforderlichen Bewegungsablaufs der Robotereinrichtung des Laborautomaten.

Durch die automatische Auswahl der genannten Komponenten und/oder der Steuerparameter in Abhängigkeit von mindestens einem Programmparameter, insbesondere in Abhängigkeit von dem mindestens einen, vom Benutzer ausgewählten Programmparameter, ergibt sich für den Benutzer der Vorteil, dass er die Auswahl der Komponenten und Steuerparameter nicht selbst einzeln bestimmen muss. Vielmehr erfolgt die Auswahl der für die Behandlung notwendigen Steuerparameter mittels der vom Benutzer eingegebenen Programmparameter. Der Benutzer muss keine Kenntnisse über die Programmierung des Automaten besitzen. Dadurch ist die Verwendung des Laborautomaten besonders komfortabel.

Durch die automatische Auswahl der genannten Komponenten und/oder der Steuerparameter in Abhängigkeit von mindestens einem Programmparameter kann z.B. erreicht werden, dass aufgrund der Anwenderangaben (z.B. 20 Proben zu verdünnen) automatisch der richtige Pipettierkopf gegriffen wird, bzw., allgemeiner, der passende Tool, z.B. Transportkopf und/oder Werkzeugkopf verwendet wird. D.h. der Anwender muss dann nicht entscheiden, was das optimale Tool ist, sondern er entscheidet nur, was seine gewünschte Behandlung ist, z.B. die Nukleinsäureaufreinigung in einer gewünschten Weise. Der Benutzer, z.B. ein Biologe, eine biologisch-technischer Assistent, ein medizinisch-technischer Assistent muss dann also lediglich die Entscheidungen treffen, die er aufgrund seiner Ausbildung einfach und schnell treffen kann, er muss aber weder eine abstrakte Programmiersprache beherrschen, noch muss er größere Berechnungen anstellen.

Die Behandlungseinrichtung des Laborautomaten weist auf: vorzugsweise mindestens einen Arbeitsbereich, vorzugsweise mindestens eine Transporteinrichtung, vorzugsweise mindestens eine Behandlungseinheit.

Vorzugsweise hat das Laborgerät, insbesondere der Laborautomat, die Eigenschaft die vom Benutzer eingegebenen Programmparameter dauerhaft zu speichern und später automatisch - oder vom Benutzer ausgelöst - wieder zu laden. Der Benutzer kann dann einzelne der Parameter ändern, um eine Probenbehandlungsart vollständig zu definieren. Dadurch wird der Bedienkomfort erhöht und die Fehleranfälligkeit verringert. Dies ist vor dem Hintergrund vorteilhaft, dass Laborgeräte besonders effizient für wiederkehrende Prozesse verwendet werden.

Das erfindungsgemäße Laborgerät weist vorzugsweise eine Kommunikationseinrichtung zur Herstellung einer Datenfernverbindung für den Datenaustausch mit einem externen Gerät auf, das ebenfalls eine geeignete Kommunikationseinrichtung zur Herstellung einer Fernverbindung für den Datenaustausch mit dem Laborautomat aufweist.

Das Laborgerät weist vorzugsweise eine Benutzerschnittstelleneinrichtung zur manuellen Eingabe von Daten durch einen Benutzer auf, und zur Anzeige von Informationen, insbesondere von in diesen Daten enthaltenen Informationen, wobei die Benutzerschnittstelleneinrichtung eine Anzeigeneinrichtung, insbesondere ein Display, insbesondere ein Touch-Screen-Display aufweist.

Das erfindungsgemäße Laborgerät kann mehrere Behandlungseinrichtungen aufweisen. Die erfindungsgemäße Zugriffssteuerung kann mehreren erfindungsgemäßen Laborgeräten zugeordnet sein, insbesondere mit diesen über eine zweite Schnittstelleneinrichtung, und insbesondere zweite Datenverbindungen, verbindbar sein oder verbunden sein. Dadurch kann mit einer Zugriffssteuerung der Zugriff der Benutzer auf mehr als ein Laborgerät oder auf ein Laborgerät mit mehr als einer Behandlungseinrichtung ermöglicht sein.

Die Erfindung betrifft ferner ein Verfahren gemäß Anspruch 13 zur Steuerung des Zugriffs auf Funktionen eines Laborgeräts mittels einer Zugriffssteuerung nach einem der Ansprüche 1 bis 12.

Weitere mögliche bevorzugte Ausgestaltungen des erfindungsgemäßen Verfahrens lassen sich aus der Beschreibung der Zugriffssteuerung und des erfindungsgemäßen Laborgeräts und von deren bevorzugten Ausgestaltungen ableiten.

Weitere bevorzugte Ausgestaltungen der Zugriffssteuerung und des erfindungsgemäßen Laborgeräts und des erfindungsgemäßen Verfahrens ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1 zeigt schematisch ein Ausführungsbeispiel der Zugriffssteuerung eines erfindungsgemäßen Laborgeräts.
Fig. 2a zeigt ein Ausführungsbeispiel des erfindungsgemäßen Laborgeräts, das eine Zugriffssteuerung aufweist.
Fig. 2b zeigt ein Ausführungsbeispiel einer externen Datenverarbeitungsanlage, mit der ein weiterer Benutzer eine Datenfernverbindung mit der Zugriffssteuerung des Laborgeräts aus Fig. 2a herstellen kann, um an der Zugriffssteuerung nach einer Anmeldung nachzufragen.
Fig. 3a zeigt ein Ausführungsbeispiel des erfindungsgemäßen Verfahrens für die Steuerung des Zugriffs eines weiteren Benutzers auf eine Zugriffssteuerung des Laborgeräts in Fig. 2a, auf dem die Sitzung eines ersten Benutzers aktiv ist.
Fig. 4 zeigt ein weiteres Ausführungsbeispiel des erfindungsgemäßen Laborgeräts, einen Thermocycler.
Fig. 5 zeigt ein weiteres Ausführungsbeispiel des erfindungsgemäßen Laborgeräts, ein Labor-Gefriergerät.

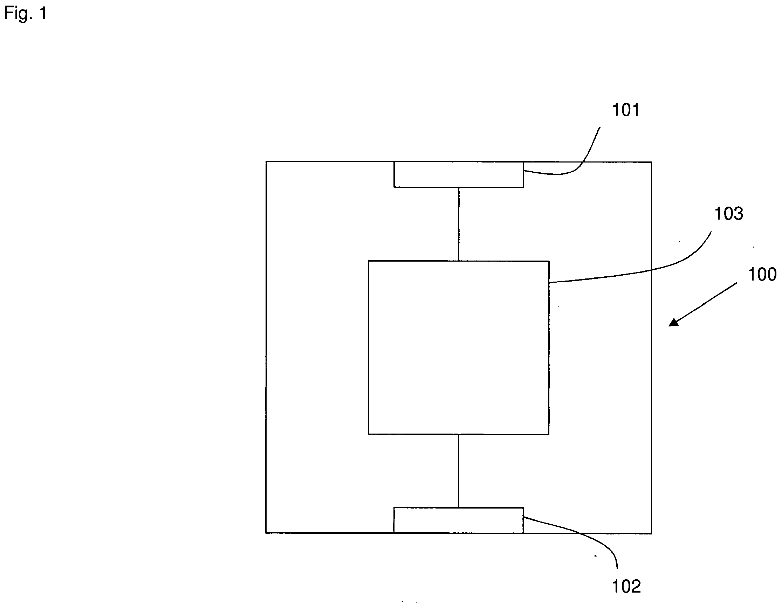

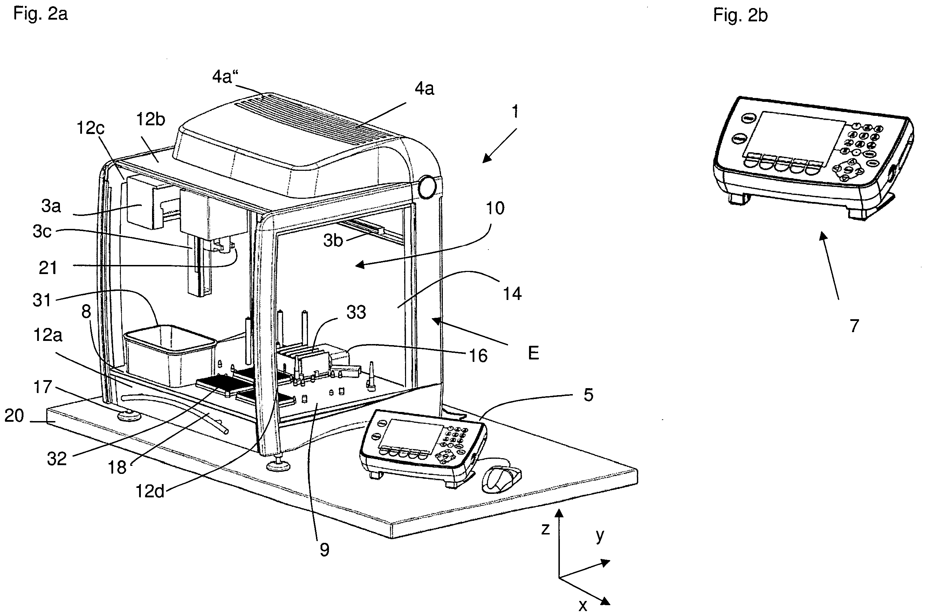

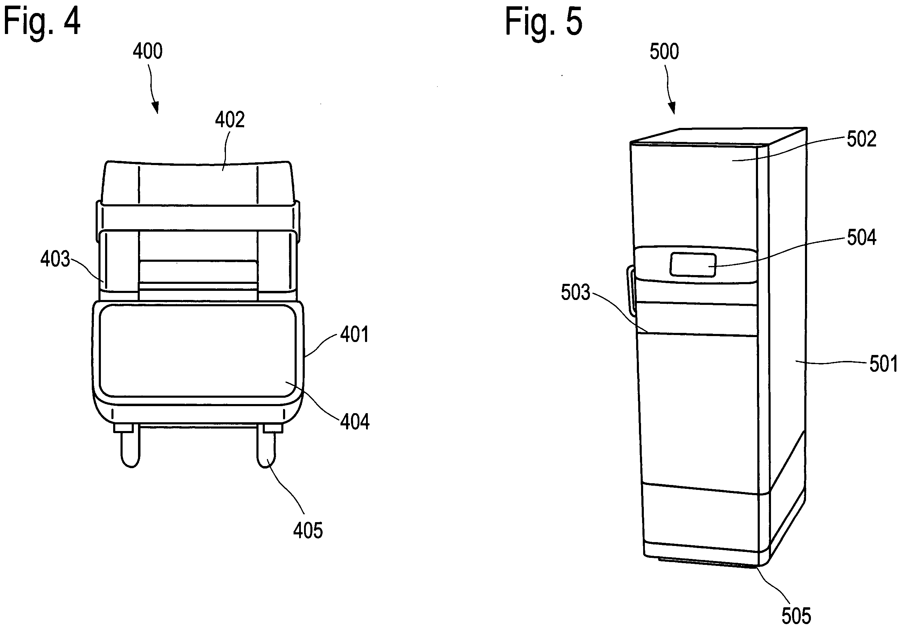

Fig. 1 zeigt die Zugriffssteuerung 100. Die Zugriffssteuerung 100 ist eingerichtet für ein Laborgerät, das der gerätegesteuerten Behandlung einer Laborprobe dient, insbesondere für Laborgeräte wie Laborgerät 1 in Fig. 2a, Laborgerät 1' in Fig. 4 und Laborgerät 1" in Fig. 5, wobei die Zugriffssteuerung 100 aufweist: eine erste Schnittstelleneinrichtung 101 und eine zweite Schnittstelleneinrichtung 102; und eine Steuereinrichtung 103. Diese ist dazu eingerichtet, a) über die erste Schnittstelleneinrichtung eine oder mehrere erste Datenverbindungen mit einer oder mehreren Benutzerschnittstelleneinrichtungen herzustellen; b) über die zweite Schnittstelleneinrichtung 102 eine zweite Datenverbindung mit dem Laborgerät 1, aber alternativ auch 1' oder 1" herzustellen; und
c) Berechtigungen und/oder Zugriffsrechte für den Zugriff von Benutzern über die ersten und zweiten Datenverbindungen auf Funktionen des Laborgeräts zu steuern. Die Steuereinrichtung 100 ist dazu eingerichtet über eine erste Datenverbindung einen anfragenden Benutzer an der Zugriffssteuerung für eine Sitzung anzumelden und ihm Berechtigungen und/oder Zugriffsrechte zuzuweisen, und während dieser Sitzung über eine weitere erste Datenverbindung die mindestens eine Anfrage mindestens eines weiteren Benutzers nach einer Anmeldung an der Zugriffssteuerung zu steuern.

Fig. 2a zeigt das Laborgerät 1, das hier als Laborautomat 1 zur Behandlung von fluiden Proben, und zwar als Pipettierautomat ausgebildet ist. Der Laborautomat 1 dient der programmgesteuerten Behandlung dieser Proben.

Fig. 2a zeigt den Laborautomaten 1 für die automatisierte Verarbeitung von flüssigen Proben, insbesondere für die programmgesteuerte Behandlung von flüssigen Proben. Der Laborautomat 1 ist ein Tischgerät und ist mit seinen vier Sockeln 17 auf dem Arbeitstisch 20 angeordnet. Es verfügt über eine elektronische Steuereinrichtung 2 (nicht gezeigt), die geeignet ist einen Programmcode für die programmgesteuerte Behandlung der flüssigen Proben zu verarbeiten. Die Steuereinrichtung 2 ist in dem Steuerraum angebracht, der durch den Pfeil E bezeichnet ist und der von dem Arbeitsraum 10 durch eine vertikale Wand 14 getrennt ist. Der Steuerraum beherbergt auch die Spannungsversorgungskomponenten, die die geeignete Versorgungsspannung für die elektrischen Komponenten des Laborautomaten liefern. In die Steuereinrichtung 2 ist die Steuereinrichtung 103 der Zugriffssteuerung 100 aus Fig. 1 integriert.

Der Laborautomat 1 weist einen Behandlungsraum 10 zur Aufnahme der flüssigen, zu behandelnden Proben auf, eine programmgesteuert steuerbare Probenbearbeitungseinrichtung 3, zum Durchführen von mindestens einem programmgesteuerten Behandlungsschritt an der mindestens einen Probe, die in dem Bearbeitungsraum angeordnet ist. Der Probenbearbeitungseinrichtung 3 sind die Komponenten 3a, 3b, 3c und 3d der Bewegungseinrichtung zugeordnet.

Der Laborautomat 1 weist ein Gehäuse 12 auf, das eine Vorderseite 12a aufweist, eine gegenüber der Vorderseite angeordnete Rückseite 12f (nicht gezeigt), eine Oberseite 12b, eine gegenüber der Oberseite angeordnete Unterseite 12e (nicht gezeigt) und gegenüberliegenden laterale Seiten 12c und 12d. Die Seiten 12a, 12b und 12c sind im Wesentlichen aus einem Material, das für sichtbares Licht transparent ist.

Die Vorderseite 12a, die im Wesentlichen wie eine Tür 12a, nämlich eine Schiebetür 12a, ausgebildet ist, kann von Hand bewegt werden und/oder programmgesteuert bewegt werden und kann und sich nach unten im Wesentlichen entlang der z- Achse des kartesischen Koordinatensystems schließen. In Fig. 2a ist die geschlossene Position der Tür 12a gezeigt.

Der Behandlungsraum 10 ist durch die Vorderseite 12a und die beiden Seitenflächen 12c und 12d sowie die Wand 14 und die Arbeitsfläche 8, welche die obere Seite der Bodenplatte 9 bildet, beschränkt. Die Arbeitsfläche 8 stellt sechs Bearbeitungsstationen zur Verfügung. Die Bearbeitungsstationen sind im Wesentlichen ebenen Flächen im Bearbeitungsbereich 8. Stifte dienen dazu Lab-Ware, also beispielsweise Thermorack 33, Mikrotiterplatten 32 und Abfallbehälter 31, an der jeweiligen Bearbeitungsstation auszurichten. Die genaue Positionierung ermöglicht eine präzise, robotergesteuerte Adressierung der Probenbehälter, insbesondere der Vertiefungen in den Mikrotiterplatten 32. Eine magnetische Trennvorrichtung 16 ist in der Nähe der Wand 14 angeordnet, wo ein Thermorack 33, d.h. eine temperaturgesteuerte Probengefäßhalterung, angeordnet ist. Die magnetische Gabel (nicht gezeigt) der magnetischen Trennvorrichtung 16 fährt von der Seite in entsprechende Aufnahmekanäle des Thermoracks ein, um seitlich an den Laborgefäßen (Probenröhrchen) ihre magnetische Wirkung zu entfalten.

Der Laborautomat 1 weist zwei Dekontaminationseinrichtungen auf, eine elektronisch steuerbare Luftreinigungsvorrichtung für die Reinigung der Luft im Behandlungsraum, die elektronisch und digital durch die Steuereinrichtung gesteuert wird und die eine Entlüftungsvorrichtung 4a, 4a" aufweist. Die Belüftungsvorrichtung weist drei Ventilatoren auf (nicht dargestellt), die einen Luftstrom von außerhalb der Vorrichtung in den Behandlungsraum transportieren.

Die Steuereinrichtung 2 weist ein Steuerprogramm auf. Der Laborautomat 1 weist eine Probenbearbeitungseinrichtung 3 auf, die eine Bewegungseinrichtung aufweist, mit drei Schienenelementen 3a, 3b, 3c, die Bewegungen entlang der y, x und z- Achse des kartesischen Koordinatensystems entsprechen. Zum Antrieb der Bewegung entlang der gewünschten Richtung sind elektronisch regelbare Linearmotoren vorgesehen. Auf diese Weise kann der Montagekopf 21 in jede gewünschte Position zugänglich in den Bearbeitungsraum 10 bewegt werden. Die Bewegungseinrichtung ist Teil eines Robotersystems der Probenbearbeitungseinrichtung 3. Mit dieser ist der Montagekopf 21 programmgesteuert transportierbar. Mit dem Montagekopf ist ein Werkzeuggerät verbindbar, z. B. ein Pipettierkopf oder ein Greifer. Die im Behandlungsraum angeordneten Bauteile, insbesondere die Probenbearbeitungseinrichtung 3, sind Bestandteil der Behandlungsvorrichtung des Laborautomaten.

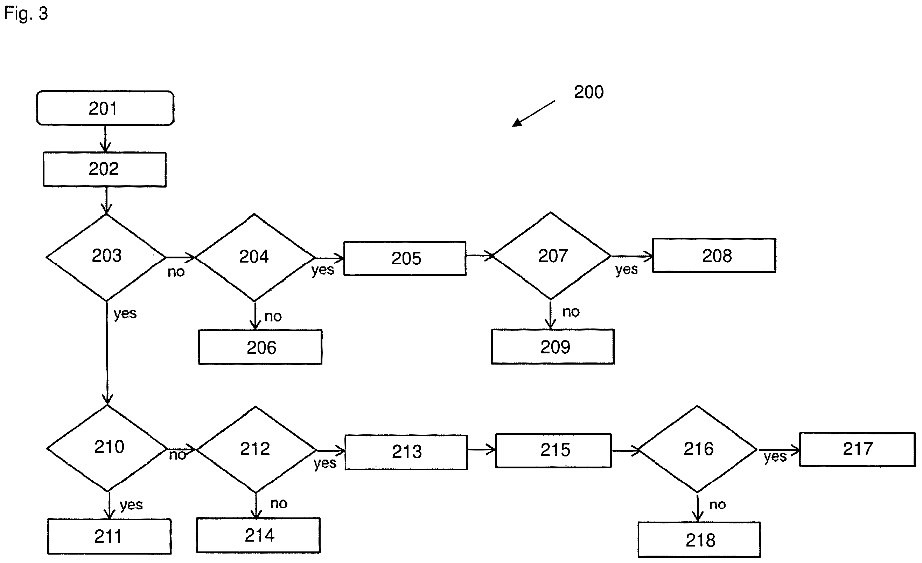

Der Laborautomat weist eine Benutzerschnittstelleneinrichtung 5 auf, mit der ein Benutzer sich lokal im Laborautomat anmelden kann. In der vorliegenden Situation in Fig. 2a wird davon ausgegangen, dass ein erster Benutzer am Laborautomaten angemeldet (eingeloggt) ist und eine Behandlung gestartet hat. Die Sitzung des ersten Benutzers ist aktiv. In dieser Situation ist es möglich, dass ein weiterer Benutzer über die in Fig. 2b gezeigte, mobile Benutzerschnittstelleneinrichtung 7 auf den Laborautomaten zugreifen will. Dazu muss er sich am Laborautomaten anmelden. Die Benutzerschnittstelleneinrichtung 7 kann z.B. über eine Mobilfunkverbindung mit der Zugriffssteuerung des Laborautomaten verbindbar sein. Fig. 3 zeigt ein Ausführungsbeispiel eines Verfahrens 200, mit dem die Zugriffssteuerung die während der Sitzung des ersten Benutzers erfolgende Anfrage des weiteren Benutzers nach einer Anmeldung an der Zugriffssteuerung bzw. dem Laborautomaten vorzugsweise steuert.

Fig. 3 bezeichnet den weiteren Benutzer 201, der eine Anfrage 202 nach einer Anmeldung an der Zugriffssteuerung des Laborautomaten 1 durchführt, die während der Sitzung des ersten Benutzers erfolgt. Die Authentifizierung erfolgt vorzugsweise im Schritt 202, insbesondere kann in diesem Schritt auch bereits eine bestimmte Autorisierung erfolgen, es können bei diesem Schritt insbesondere bereits bestimmte Zugriffsrechte und/oder Berechtigungen an den weiteren Benutzer erteilt werden oder verweigert werden. Es ist insbesondere möglich, dass dem weiteren Benutzer im Schritt 202 bereits eine zweite Sitzung eröffnet wird, die parallel zur ersten Sitzung abläuft, aber vorzugsweise andere, insbesondere beschränkte Zugriffsrechte und/oder Berechtigungen aufweist, im Vergleich mit der Sitzung des ersten Benutzers.

Die Zugriffssteuerung bzw. deren Steuereinrichtung überprüft vorzugsweise im Schritt 203, ob die Behandlungseinrichtung des Laborautomaten läuft, also aktiv ist. Alternativ wäre es auch möglich, dass erst in Schritt 203 geprüft wird, ob eine Sitzung eines ersten Benutzers läuft, also ob ein erster Benutzer angemeldet ist. Falls die Prüfung in Schritt 203 mit "ja" beantwortet wird, wird in Schritt 210 überprüft, ob der erste, bereits angemeldete Benutzer mit dem weiteren Benutzer 201 identisch ist. Dies ist möglich, da beide Benutzer über ihre Authentifizierungsdaten eindeutig ermittelt wurden und so grundsätzlich unterscheidbar sind.

Falls die Zugriffssteuerung feststellt, dass der weitere Benutzer nicht mit dem ersten Benutzer identisch ist, wird dem weiteren Benutzer vorzugsweise der Zugriff auf die Funktion der Steuerung der Behandlungseinrichtung verweigert, indem diesem Benutzer die dazu erforderlichen Zugriffsrechte und/oder Berechtigungen nicht erteilt werden (Schritt 211). Es wäre auch möglich und bevorzugt, dass dem weiteren Benutzer im Schritt 211 jegliche Zugriffsrechte und/oder Berechtigungen verweigert werden, dass also keine Autorisierung für den weiteren Benutzer erfolgt.

Die Zugriffssteuerung kann im Schritt 210 auch zum Ergebnis kommen, dass der weitere Benutzer mit dem ersten Benutzer identisch ist. Die Zugriffssteuerung kann dann im Schritt 212 prüfen, ob der Zugriff des weiteren Benutzers - dessen Identität dann mit der des ersten übereinstimmt - über eine andere Benutzerschnittstelleneinrichtung erfolgt als im Fall des Zugriffs, bzw. der Anmeldung, des ersten Benutzers. Die Information über die Identität der Benutzerschnittstelleneinrichtung kann beim Zugriff bzw. beim Authentifizieren eines Benutzers in der Zugriffssteuerung, bzw. in dem Laborgerät, gespeichert werden. Sind die Benutzer unterschiedlich, kann die Autorisierung erfolgen (Schritt 214). Sind die Benutzer identisch, kann ein Synchronisierungsvorgang eingeleitet werden.

Der Synchronisierungsvorgang kann dazu führen, dass die Benutzerschnittstelle, über die der zweite (der weitere) Zugriff erfolgt, in den Zustand versetzt wird, in dem auch die erste Benutzerschnittstelleneinrichtung war, über die der erste Zugriff bzw. die zeitlich vorhergegangene erste Autorisierung erfolgt ist. Dieser Zustand kann zumindest so sein, dass an der zweiten Benutzerschnittstelleneinrichtung in gleicher Weise wie an der ersten Benutzerschnittstelleneinrichtung weitergearbeitet werden kann, insbesondere dass die laufende Sitzung des ersten Benutzers mit der zweiten Benutzerschnittstelleneinrichtung fortgeführt werden kann.

Der Synchronisierungsvorgang kann vorsehen, dass Parameter synchronisiert werden (Schritt 213) und dass von der zweiten Benutzerschnittstelleneinrichtung eine Priorität erfragt wird (Schritt 215). Damit ist gemeint, dass die Zugriffssteuerung anhand optionaler weiterer Kriterien entscheiden kann, ob bei gegebener Identität der Benutzer tatsächlich der Zugriff über die zweite Benutzerschnittstelleneinrichtung auf den Laborautomaten bzw. dessen Behandlungseinrichtung gewährt wird (Schritt 217), oder nicht (Schritt 218).

Im Falle der Prüfung in Schritt 203 kann die Zugriffssteuerung zu dem Ergebnis kommen, dass die Behandlungseinrichtung des Laborautomaten nicht läuft, also inaktiv ist. Alternativ wäre es auch möglich, dass in Schritt 203 festgestellt wird, dass keine Sitzung eines ersten Benutzers läuft, also kein erster Benutzer angemeldet ist. Bei diesen Ergebnissen kann in Schritt 204 geprüft werden, ob ein erster Benutzer angemeldet ist und die Zugriffsrechte und/oder Berechtigung zur Steuerung der Behandlungseinrichtung besitzt. Alternativ könnte im Schritt 204 auch nur geprüft werden, ob ein erster Benutzer angemeldet ist, also irgendwelche Zugriffsrechte und/oder Berechtigung besitzt. Falls die Prüfung in Schritt 204 ein "nein" ergibt, kann die Autorisierung (zur Steuerung der Behandlungseinrichtung oder alternativ, zur Anmeldung/Autorisierung) erfolgen (Schritt 206). Falls die Prüfung in Schritt 204 ein "ja" ergibt, kann im Schritt 205 beim Anmeldevorgang des weiteren Benutzers eine Anfrage dahingehend erfolgen, dass der erste Benutzer die Rechte verliert, die der weitere Benutzer für die gewünschte Autorisierung benötigt. Falls die Prüfung dieser Anfrage in Schritt 207 ergibt, dass aufgrund irgendeines von der Zugriffssteuerung verwendeten Kriteriums der Rechteübergang nicht erlaubt werden kann, erfolgt dieser auch nicht (Schritt 209), andernfalls erfolgt dieser (Schritt 208).

Fig. 4 zeigt ein Laborgerät 400, ein Thermoycler, ausgebildet für die automatisierte Verarbeitung von flüssigen Proben, insbesondere für die programmgesteuerte Temperierung von flüssigen Proben. Das Laborgerät 400 ist ein Tischgerät. Es verfügt über eine integrierte, elektronische Steuereinrichtung (nicht gezeigt), die geeignet ist einen Programmcode für die programmgesteuerte Behandlung der flüssigen Proben zu verarbeiten. Die Steuereinrichtung ist in dem Gehäuse 401 untergebracht. Das Gehäuse beherbergt auch die Spannungsversorgungskomponenten, die die geeignete Versorgungsspannung für die elektrischen Komponenten des Thermocyclers liefern.

Das Laborgerät 400 weist einen Behandlungsraum 403 zur Aufnahme der flüssigen zu behandelnden Proben auf, der mindestens eine programmgesteuert steuerbare Behandlungseinrichtung (nicht gezeigt) aufnehmen kann, zum Durchführen von mindestens einem programmgesteuerten Behandlungsschritt an der mindestens einen Probe, die in der Behandlungseinrichtung angeordnet ist, welche in dem Bearbeitungsraum angeordnet ist. Der Behandlungsraum kann mit einem Deckel 402 verschlossen werden, um eine definierte Temperierungsumgebung herzustellen. In Fig. 4 ist das Laborgerät in geschlossenem Zustand dargestellt.

In die Steuereinrichtung ist die Steuereinrichtung 103 der Zugriffssteuerung 100 aus Fig. 1 integriert. Die Steuereinrichtung weist ein Steuerprogramm auf.

Das Laborgerät weist eine Benutzerschnittstelleneinrichtung 404 auf, mit der ein Benutzer sich lokal am Laborgerät anmelden kann. In der vorliegenden Situation in Fig. 4 wird davon ausgegangen, dass ein erster Benutzer am Laborautomaten angemeldet (eingeloggt) ist und eine Behandlung gestartet hat. Die Sitzung des ersten Benutzers ist aktiv. In dieser Situation ist es möglich, dass ein weiterer Benutzer über die in Fig. 2b gezeigte mobile Benutzerschnittstelleneinrichtung 7 auf das Laborgerät zugreifen will. Dazu muss er sich am Laborgerät anmelden. Die Benutzerschnittstelleneinrichtung 7 kann z.B. über eine Mobilfunkverbindung mit der Zugriffssteuerung des Laborgeräts verbindbar sein. Ein Ausführungsbeispiel ist in Fig. 3 dargestellt.

Fig. 5 zeigt in einem weiteren Ausführungsbeispiel der Erfindung ein Laborgerät 500, ein Labor-Gefriergerät, für die Lagerung von Laborproben, insbesondere bei Temperaturen unter -50°C. Das Laborgerät 500 ist ein Tischgerät. Es verfügt über eine integrierte elektronische Steuereinrichtung (nicht gezeigt), die geeignet ist die Temperatur in dem erforderten Bereich einzustellen, zu regeln und zu überwachen. Die Steuereinrichtung ist in dem Gehäuse 501 untergebracht. Das Gehäuse beherbergt auch die Spannungsversorgungskomponenten, die die geeignete Versorgungsspannung für die elektrischen Komponenten des Labor-Gefriergeräts liefern.

Das Laborgerät 500 weist einen Behandlungsraum 503 zur Aufnahme der aufzubewahrenden Proben auf, der mindestens eine programmgesteuert steuerbare Behandlungseinrichtung (nicht gezeigt) umfasst, der im Falle des Labor-Gefriergerätes einen abgeschlossenen Bereich mit einer definiert einstellbaren Temperatur entspricht. Der programmgesteuerte Behandlungsschritt entspricht dabei dem Einfrieren der mindestens einen Probe, die in der Behandlungseinrichtung angeordnet ist, welche in dem Bearbeitungsraum angeordnet ist. Der Behandlungsraum kann mit einer Tür 502 verschlossen werden, um eine definierte Temperierungsumgebung herzustellen. Im Falle von mehr als einer Behandlungseinrichtung sind auch mehrere Türen, ggf. angeordnet hinter der gemeinsamen Tür 502, denkbar. In Fig. 5 ist das Laborgerät in geschlossenem Zustand dargestellt.

In die Steuereinrichtung des Laborgeräts 500 ist die Steuereinrichtung 103 der Zugriffssteuerung 100 aus Fig. 1 integriert. Die Steuereinrichtung weist ein Steuerprogramm auf.

Das Laborgerät weist eine Benutzerschnittstelleneinrichtung 504 auf, mit der ein Benutzer sich lokal am Laborgerät anmelden kann. In der vorliegenden Situation in Fig. 5 wird davon ausgegangen, dass ein erster Benutzer am Laborautomaten angemeldet (eingeloggt) ist. Die Behandlung ist gestartet und läuft permanent. Die Sitzung des ersten Benutzers ist aktiv. In dieser Situation ist es möglich, dass ein weiterer Benutzer über die in Fig. 2b gezeigte mobile Benutzerschnittstelleneinrichtung 7 auf das Laborgerät zugreifen will. Dazu muss er sich am Laborgerät anmelden. Die Benutzerschnittstelleneinrichtung 7 kann z.B. über eine Mobilfunkverbindung mit der Zugriffssteuerung des Laborgeräts verbindbar sein. Ein Ausführungsbeispiel ist in Fig. 3 dargestellt.

### Anhang 1

### Mögliche Programmparameter in Abhängigkeit vom Typ des Laborgeräts

| **Gerät** | **Wichtigste Parameter** | | | | | **Abfolgeprogrammierung** |
|---|---|---|---|---|---|---|
| Centrifuge | Temperatur | Geschwindigkeit | | Zeit | | nein, Schritte denkbar |
| | | | | | | |
| Cycler | Temperatur | | | Zeit | | Schritte |
| | | | | | | |
| Biospektrometer | Temperatur (kinetic) | | | | Ergebnis | komplexe Methode |
| Plattenreader | Temperatur | Probenanzahl | | | Ergebnis | Komplexe Methdoe |
| Cellcounter | | | | | Ergebnis | komplexe Methode |
| | | | | | | |
| Inkubator | Temperatur | CO2/ O2 | rel. Luftfeuchte | Zeit | | nein, Schritte denkbar |
| | | | | | | |
| Shaker | Temperatur | Geschwindigkeit | | | | Schritte |
| | | | | | | |
| Freezer | Temperatur | Alarmwert | | | | nein |
| | | | | | | |
| Fermenter/Bioreaktor | | Rührdrehzahl | Gelöstsauerstoff (DO) | pH-Wert | Dosiergeschwindigkeit (Pumpen) | |
| Laborautomat | Probenanzahl | Probenvolumen | Pipettier-Werkzeug | Quelle/ Ziel | Transfertyp (Pipettieren/ Dispensieren) | Komplexe Methode |
| (Thermo-)Mixer | Temperatur | Geschwindigkeit | | Zeit | | Begrenzte Schritte |
| Pipettensteuerung | Probenvolumen | | Pipettierwerkzeug | | Transfertyp | Begrenzte Schritte |
| Biosafety Cabinet | | Strömungsgeschwindigkeit | Filterbetriebsdauer | Lüfterbetriebsdauer | Luftmenge | |

| | | |
|---|---|---|
| Zu betrachtende Use Cases (Beispiele): | Remote Mo-nitoring Remote Control Booking Schedule Service-Zugriff Preprogramming | |
| Zu betrachtende Rollen (Beispiele): | Admin LabUser Inexperienced Manager Service | |
| Zu betrachtende Geräte (Bei- | | |
| spiele): | Cycler | n Behandlungseinrichtungen (Thermoblöcke) |
| Annahme: Zugriffrechte sind | | |
| unabhängig vom Gerät | Centrifuge | 1 Behandlungseinrichtung (Rotor) |
| | | 1 Behandlungseinrichtung (Schüttelplatt- |
| | Shaker | form, auch mehrere denkbar) |
| | Incubator | 1 Behandlungseinrichtung |
| | Cell Counter | 1 Behandlungseinrichtung |
| | BSC | 1 Behandlungseinrichtung |
| | | n Behandlungseinrichtungen denkbar (unter- |
| | Freezer Laborautomat | schiedlich ansteuerbare Kühlebenen) |
| | Biospectro- | 1 Behandlungseinrichtung |
| | meter | 1 Behandlungseinrichtung |

| **Use Case:** | **Remote Monito-ring** | |
|---|---|---|
| **User: Admin** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | j |
| =Leerlauf? | LabUser | j |
| | Inexperienced | j |
| | Manager | j |
| Standby | - | j |

| **Use Case:** | **Remote Monito-ring** | |
|---|---|---|
| **User: LabUser** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Standby | - | j |

| **Use Case:** | **Remote Monito-ring** | |
|---|---|---|
| **User: Inexperienced** | | |
| Zustand | Rolle eingeloggt | Zugriff möglich ? |
| Leerlauf (ready) | Admin | n |
| | LabUser | n |
| | Inexperienced | n |
| | Manager | n |
| Programmiert | Admin | n |
| =Leerlauf? | LabUser | n |
| | Inexperienced | n |
| | Manager | n |
| Gestartet (running) | Admin | n |
| | LabUser | n |
| | Inexperienced | n |
| | Manager | n |
| Gestoppt (finished) | Admin | n |
| =Leerlauf? | LabUser | n |
| | Inexperienced | n |
| | Manager | n |
| Standby | - | j |
| **User: Manager** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Standby | - | j |

| **Use Case:** | **Remote Control** | |
|---|---|---|
| **User: Admin** | | |
| Zustand | Rolle eingeloggt | Zugriff möglich ? |
| Leerlauf (ready) | Admin | n |
| | LabUser | j |
| | Inexperienced | j |
| | Manager | j |
| Programmiert | Admin | n |
| =Leerlauf? | LabUser | j |
| | Inexperienced | j |
| | Manager | j |
| Gestartet (running) | Admin | n |
| | LabUser | j |
| | Inexperienced | j |
| | Manager | j |
| Gestoppt (finished) | Admin | n |
| =Leerlauf? | LabUser | j |
| | Inexperienced | j |
| | Manager | j |
| Booking | Admin | n |
| =Leerlauf? | LabUser | j |
| | Inexperienced | j |
| | Manager | j |
| Standby | - | n |

| **Use Case:** | **Remote Control** | |
|---|---|---|
| **User: LabUser** | | |
| Zustand | Rolle eingeloggt | Zugriff möglich ? |
| Leerlauf (ready) | Admin | n |
| | LabUser | n |
| | Inexperienced | j |
| | Manager | n |
| Programmiert | Admin | n |
| =Leerlauf? | LabUser | n |
| | Inexperienced | j |
| | Manager | n |
| Gestartet (running) | Admin | n |
| | LabUser | n |
| | Inexperienced | j |
| | Manager | n |
| Gestoppt (finished) | Admin | n |
| =Leerlauf? | LabUser | n |
| | Inexperienced | j |
| | Manager | n |
| Booking | Admin | n |
| =Leerlauf? | LabUser | n |
| | Inexperienced | j |
| | Manager | n |
| Standby | - | n |

| **Use Case:** | **Remote Control** | |
|---|---|---|
| **User: Inexperienced** | | |
| Zustand | Rolle eingeloggt | Zugriff möglich ? |
| Leerlauf (ready) | Admin | n |
| | LabUser | n |
| | Inexperienced | n |
| | Manager | n |
| Programmiert | Admin | n |
| =Leerlauf? | LabUser | n |
| | Inexperienced | n |
| | Manager | n |
| Gestartet (running) | Admin | n |
| | LabUser | n |
| | Inexperienced | n |
| | Manager | n |
| Gestoppt (finished) | Admin | n |
| =Leerlauf? | LabUser | n |
| | Inexperienced | n |
| | Manager | n |
| Booking | Admin | n |
| =Leerlauf? | LabUser | n |
| | Inexperienced | n |
| | Manager | n |
| Standby | - | n |

| **Use Case:** | **Remote Control** | |
|---|---|---|
| **User: Manager** | | |
| Zustand | Rolle eingeloggt | Zugriff möglich ? |
| Leerlauf (ready) | Admin | n |
| | LabUser | j |
| | Inexperienced | n |
| | Manager | n |
| Programmiert | Admin | n |
| =Leerlauf? | LabUser | j |
| | Inexperienced | n |
| | Manager | n |
| Gestartet (running) | Admin | n |
| | LabUser | j |
| | Inexperienced | n |
| | Manager | n |
| Gestoppt (finished) | Admin | n |
| =Leerlauf? | LabUser | j |
| | Inexperienced | n |
| | Manager | n |
| Booking | Admin | n |
| =Leerlauf? | LabUser | j |
| | Inexperienced | n |
| | Manager | n |
| Standby | - | n |

| **Use Case:** | **Booking Schedule** | |
|---|---|---|
| **User: Admin** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | n |
| =Leerlauf? | LabUser | n |
| | Inexperienced | n |
| | Manager | n |
| Standby | - | j |

| **Use Case:** | **Booking Schedule** | |
|---|---|---|
| **User: LabUser** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | n |
| =Leerlauf? | LabUser | n |
| | Inexperienced | n |
| | Manager | n |
| Standby | -- | j |

| **Use Case:** | **Booking Schedule** | |
|---|---|---|
| **User: Inexperienced** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | n |
| =Leerlauf? | LabUser | n |
| | Inexperienced | n |
| | Manager | n |
| Standby | -- | j |

| **Use Case:** | **Booking Schedule** | |
|---|---|---|
| **User: Manager** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | n |
| =Leerlauf? | LabUser | n |
| | Inexperienced | n |
| | Manager | n |
| Standby | -- | j |

| **Use Case:** | **Preprogramming** | |
|---|---|---|
| **User: Admin** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | j |
| =Leerlauf? | LabUser | j |
| | Inexperienced | j |
| | Manager | j |
| Standby | -- | j |

| **Use Case:** | **Preprogramming** | |
|---|---|---|
| **User: LabUser** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | j |
| =Leerlauf? | LabUser | j |
| | Inexperienced | j |
| | Manager | j |
| Standby | -- | j |

| **Use Case:** | **Preprogramming** | |
|---|---|---|
| **User: Inexperienced** | | |
| Zustand | Rolle eingeloggt | Zugriff möglich ? |
| Leerlauf (ready) | Admin | J |
| | LabUser | J |
| | Inexperienced | J |
| | Manager | J |
| Programmiert | Admin | J |
| =Leerlauf? | LabUser | J |
| | Inexperienced | J |
| | Manager | J |
| Gestartet (running) | Admin | J |
| | LabUser | J |
| | Inexperienced | J |
| | Manager | J |
| Gestoppt (finished) | Admin | J |
| =Leerlauf? | LabUser | J |
| | Inexperienced | J |
| | Manager | J |
| Booking | Admin | J |
| =Leerlauf? | LabUser | J |
| | Inexperienced | J |
| | Manager | J |
| Standby | -- | J |

| **Use Case:** | **Preprogramming** | |
|---|---|---|
| **User: Manager** | | |
| Zustand | Rolle eingeloggt | Zugriff möglich ? |
| Leerlauf (ready) | Admin | j |
| | LabUser | j |
| | Inexperienced | j |
| | Manager | j |
| Programmiert | Admin | j |
| =Leerlauf? | LabUser | j |
| | Inexperienced | j |
| | Manager | j |
| Gestartet (running) | Admin | j |
| | LabUser | j |
| | Inexperienced | j |
| | Manager | j |
| Gestoppt (finished) | Admin | j |
| =Leerlauf? | LabUser | j |
| | Inexperienced | j |
| | Manager | j |
| Booking | Admin | j |
| =Leerlauf? | LabUser | j |
| | Inexperienced | j |
| | Manager | j |
| Standby | -- | j |

| **Use Case:** | **Remote Service-Zugriff** | |
|---|---|---|
| **User: Service** | | |
| Zustand | Rolle eingeloggt | Zugriff möglich ? |
| Leerlauf (ready) | Admin | J |
| | LabUser | N |
| | Inexperienced | N |
| | Manager | J |
| Programmiert | Admin | J |
| =Leerlauf? | LabUser | N |
| | Inexperienced | N |
| | Manager | J |
| Gestartet (running) | Admin | J |
| | LabUser | N |
| | Inexperienced | N |
| | Manager | J |
| Gestoppt (finished) | Admin | J |
| =Leerlauf? | LabUser | N |
| | Inexperienced | N |
| | Manager | J |
| Booking | Admin | J |
| =Leerlauf? | LabUser | N |
| | Inexperienced | N |
| | Manager | J |
| Standby | -- | J |

## Patentansprüche

1. Laborgerät (1; 400; 500) zur gerätegesteuerten Behandlung mindestens einer Laborprobe, das aufweist:
- mindestens eine Behandlungseinrichtung (3; 403; 503) zur Durchführung der Behandlung der mindestens einen Laborprobe, und
- eine Zugriffssteuerung (100) für das Laborgerät;
- ein Gehäuse (12; 401; 501), in dem die mindestens eine Behandlungseinrichtung und die Zugriffssteuerung angeordnet sind;
wobei die Zugriffssteuerung aufweist:
- eine erste Schnittstelleneinrichtung (101) und eine zweite Schnittstelleneinrichtung (102); und
- eine Steuereinrichtung (103);
wobei die Steuereinrichtung (103) eingerichtet und dazu programmiert ist:
a) über die erste Schnittstelleneinrichtung (101) mehrere erste Datenverbindungen für den Datenaustausch mit einer datenverarbeitenden Einheit einer oder mehrerer Benutzerschnittstelleneinrichtungen (5; 7; 404; 504) herzustellen;
b) über die zweite Schnittstelleneinrichtung (102) eine zweite Datenverbindung für den Datenaustausch mit einer datenverarbeitenden Einheit des Laborgeräts herzustellen; und
c) Berechtigungen und/oder Zugriffsrechte für den Zugriff von Benutzern über die ersten und zweiten Datenverbindungen auf Funktionen des Laborgeräts zu steuern;
wobei
die Steuereinrichtung (103) dazu eingerichtet und dazu programmiert ist, einen für eine erste Sitzung an dem Laborgerät anfragenden ersten Benutzer über die erste Datenverbindung und über die erste Schnittstelleneinrichtung (101) anzumelden und ihm Berechtigungen und/oder Zugriffsrechte zuzuweisen, und
die Steuereinrichtung (103) dazu eingerichtet und dazu programmiert ist, während dieser ersten Sitzung die mindestens eine über eine weitere erste Datenverbindung und über die erste Schnittstelleneinrichtung (101) erfolgende und auf die Anmeldung gerichtete Anfrage mindestens eines weiteren Benutzers an der Zugriffssteuerung durch einen Anmeldevorgang des mindestens einen weiteren Benutzers zu steuern, und für diesen weiteren Benutzer eine Sitzung zu starten, die zumindest zeitweise parallel zu der ersten Sitzung des ersten Benutzers stattfindet.

2. Laborgerät gemäß Anspruch 1, wobei die Steuereinrichtung (103) dazu eingerichtet ist, dem mindestens einen weiteren anfragenden Benutzer jedenfalls nach dessen Anmeldung während der ersten Sitzung des ersten Benutzers Berechtigungen und/oder Zugriffsrechte zuzuweisen.

3. Laborgerät gemäß einem der vorherigen Ansprüche, wobei die Steuereinrichtung (103) dazu eingerichtet ist, dass die Berechtigungen und/oder Zugriffsrechte so gesteuert werden können, dass ein gleichzeitiger Zugriff des ersten und wenigstens des weiteren Benutzers mit jeweils getrennt zugewiesenen Berechtigungen und/oder Zugriffsrechten auf Funktionen des Laborgeräts, erfolgt.

4. Laborgerät gemäß einem der vorherigen Ansprüche, wobei die Steuereinrichtung (103) dazu eingerichtet ist, dass, wenn der erste Benutzer eine oder mehrere Funktionen des Laborgeräts aktiviert hat, die Berechtigungen und/oder Zugriffsrechte jedes weiteren angemeldeten Benutzers so festgelegt werden, dass durch eine Aktivierung einer gemäß den Berechtigungen und/oder Zugriffsrechten des weiteren Benutzers zugelassenen Funktion die Ausführung einer bereits aktivierten Funktion des Laborgeräts nicht beeinflusst werden kann.

5. Laborgerät gemäß einem der vorherigen Ansprüche, wobei die Steuereinrichtung (103) dazu eingerichtet ist, dass beim Vorliegen wenigstens einer Bedingung eine Umstellung der Berechtigungen und/oder Zugriffsrechte derart erfolgen kann, dass der weitere Benutzer anstelle des ersten Benutzers zumindest teilweise dessen Berechtigungen und/oder Zugriffsrechte erhält.

6. Laborgerät gemäß Anspruch 5, wobei die Steuereinrichtung (103) dazu eingerichtet ist, dass die Berechtigungen und/oder Zugriffsrechte, die der weitere Benutzer anstelle des ersten Benutzers erhält, das Recht zum Steuern der Behandlungseinrichtung beinhaltet.

7. Laborgerät gemäß Anspruch 6, wobei die Steuereinrichtung (103) dazu eingerichtet ist, dass die gemäß den Berechtigungen und/oder Zugriffsrechten des ersten Benutzers ausgeführte Funktion die Behandlung der wenigstens einen Probe durch eine Behandlungseinrichtung des Laborgeräts beinhaltet.

8. Laborgerät gemäß einem der vorherigen Ansprüche, wobei die Steuereinrichtung (103) so eingerichtet ist, dass die Berechtigungen und/oder Zugriffsrechte des ersten Benutzers oder jedes weiteren Benutzers in Abhängigkeit vom Betriebszustand des Laborgeräts festgelegt werden.

9. Laborgerät gemäß einem der vorherigen Ansprüche, wobei die erste Schnittstelleneinrichtung (101) das Anmelden und den Zugriff auf Funktionen des Laborgeräts über wenigstens zwei verschiedene Benutzerschnittstelleneinrichtungen (5; 7) ermöglicht und
wobei die Steuereinrichtung (103) so eingerichtet ist, dass beim Anmelden über eine zweite Benutzerschnittstelleneinrichtung (7) geprüft wird, ob der anmeldende Benutzer vorausgehend bereits über eine erste Benutzerschnittstelleneinrichtung (5)
a) eine oder mehrere gerade ausgeführte Funktionen des Laborgeräts aktiviert hatte oder
b) schon angemeldet ist, und
wenn Bedingung a) oder b) erfüllt ist, dann werden die dem Benutzer beim vorausgehenden Anmelden über die erste Benutzerschnittstelleneinrichtung durch die Zugriffsteuerung zugewiesenen Berechtigungen und/oder Zugriffsrechte für den Zugriff auf das Laborgerät über die zweite Benutzerschnittstelleneinrichtung zugewiesen.

10. Laborgerät gemäß Anspruch 9, wobei die Steuereinrichtung (103) so eingerichtet ist, dass zusätzlich geprüft wird, ob wenigstens eine weitere vorbestimmte Bedingung beim Anmelden an der zweiten Benutzerschnittstelleneinrichtung erfüllt ist, und die Zugriffsrechte für den Zugriff auf das Laborgerät über die zweite Benutzerschnittstelleneinrichtung nur dann zugewiesen werden, wenn auch die wenigstens eine weitere vorbestimmte Bedingung erfüllt ist.

11. Laborgerät gemäß Anspruch 9 oder 10, wobei die Steuereinrichtung (103) so eingerichtet ist, dass, wenn a) oder b) erfüllt ist, über die Schnittstelleneinrichtung Informationen über den Betriebszustand des Laborgeräts, Messwerte oder durch Benutzer beeinflussbare Einstellungen oder Programmierungen des Laborgeräts an die zweite Benutzerschnittstelleneinrichtung übermittelt werden.

12. Laborgerät gemäß einem der vorherigen Ansprüche, das eine Kommunikationseinrichtung zur Herstellung einer Datenfernverbindung für den Datenaustausch mit einem externen Gerät aufweist, das ebenfalls eine geeignete Kommunikationseinrichtung zur Herstellung einer Fernverbindung für den Datenaustausch mit dem Laborgerät aufweist.

13. Verfahren (200) zur Steuerung des Zugriffs auf Funktionen eines Laborgeräts (1; 400; 500) gemäß einem der Ansprüche 1 bis 13, wobei das Verfahren vorsieht
a) über die erste Schnittstelleneinrichtung (101) mehrere erste Datenverbindungen mit einer datenverarbeitenden Einheit einer oder mehrerer Benutzerschnittstelleneinrichtungen (5; 7; 404; 504) herzustellen;
b) über die zweite Schnittstelleneinrichtung (102) eine zweite Datenverbindung mit einer datenverarbeitenden Einheit des Laborgeräts herzustellen; und
c) Berechtigungen und/oder Zugriffsrechte für den Zugriff von Benutzern über die ersten und zweiten Datenverbindungen auf Funktionen des Laborgeräts zu steuern;
wobei die Steuereinrichtung (103) dazu eingerichtet und dazu programmiert ist, über eine erste Datenverbindung und über die erste Schnittstelleneinrichtung (101) einen ersten anfragenden Benutzer für eine erste Sitzung anzumelden und ihm Berechtigungen und/oder Zugriffsrechte zuzuweisen, und
die Steuereinrichtung (103) dazu eingerichtet und dazu programmiert ist, während dieser ersten Sitzung die mindestens eine über eine weitere erste Datenverbindung und über die erste Schnittstelleneinrichtung (101) erfolgende und auf die Anmeldung gerichtete Anfrage mindestens eines weiteren Benutzers (201) an der Zugriffssteuerung durch einen Anmeldevorgang des mindestens eines weiteren Benutzers zu steuern und für diesen weiteren Benutzer eine Sitzung zu starten, die zumindest zweitweise parallel zu der ersten Sitzung des ersten Benutzers stattfindet.

## Claims

1. Laboratory apparatus (1; 400; 500) for the apparatus-controlled treatment of at least one laboratory sample, comprising:
- at least one treatment device (3; 403; 503) for carrying out the treatment of the at least one laboratory sample, and
- an access control (100) for the laboratory device;
- a housing (12; 401; 501) in which the at least one treatment device and the access control (100) are arranged;
wherein the access control (100) comprises:
- a first interface device (101) and a second interface device (102); and
- a control device (103);
wherein the control device (103) is arranged and programmed to:
a) establishing, via the first interface device (101), a plurality of first data connections for data exchange with a data processing unit of one or more user interface device (5; 7; 404; 504);
b) establishing a second data connection for data exchange with a data processing unit of the laboratory instrument via the second interface device (102); and
c) controlling authorizations and/or access rights for user access to laboratory instrument functions via the first and second data connections;
wherein
the control device (103) is arranged and programmed to register a first user requesting for a first session at the laboratory apparatus via the first data connection and via the first interface device (101) and to assign authorizations and/or access rights to the user, and
the control device (103) is set up and programmed to control, during this first session, the at least one request of at least one further user to the access control, which request is made via a further first data connection and via the first interface device (101) and is directed to the login, by a login process of the at least one further user, and to start a session for this further user which takes place at least temporarily in parallel with the first session of the first user.

2. Laboratory apparatus according to claim 1, wherein the control device (103) is arranged to assign authorizations and/or access rights to the at least one further requesting user, at least after the user has logged in during the first session of the first user.

3. Laboratory apparatus according to any one of the preceding claims, wherein the control device (103) is set up in a manner that the authorizations and/or access rights can be controlled in such a way that functions of the laboratory apparatus by the first user and at least the further user, each with separately assigned authorizations and/or access rights, are accessed simultaneously.

4. Laboratory apparatus according to one of the previous claims, wherein the control device (103) is set up in such a way that, if the first user has activated one or more functions of the laboratory apparatus, the authorizations and/or access rights of each further logged-in user are defined in such a way that the execution of an already activated function of the laboratory apparatus cannot be influenced by an activation of a function permitted according to the authorizations and/or access rights of the further user.

5. Laboratory apparatus according to one of the previous claims, wherein the control device (103) is set up in such a way that, if at least one condition is present, the authorizations and/or access rights can be changed in such a way that the further user receives at least some of the authorizations and/or access rights of the first user instead of the first user.

6. Laboratory apparatus according to claim 5, wherein the control device (103) is arranged in such a way that the permissions and/or access rights given to the further user instead of the first user include the right to control the treatment means.

7. Laboratory apparatus according to claim 6, wherein the control device (103) is arranged in such a way that the function performed according to the permissions and/or access rights of the first user includes the treatment of the at least one sample by a treatment means of the laboratory apparatus.

8. Laboratory apparatus according to any one of the preceding claims, wherein the control device (103) is set up in such a way that the authorizations and/or access rights of the first user or of each further user are determined as a function of the operating state of the laboratory apparatus.

9. Laboratory apparatus according to any one of the preceding claims, wherein the first interface device (101) enables logging in and accessing functions of the laboratory apparatus via at least two different user interface device (5; 7), and
wherein the control device (103) is arranged to check, whether the user logging in via a second user interface device (7) has already via a first user interface device (5)
a) activated one or more functions of the laboratory instrument that were just being executed, or
b) already registered, and
if condition a) or b) is fulfilled, then the authorizations and/or access rights assigned to the user by the access control during the preceding login via the first user interface device are assigned for access to the laboratory device via the second user interface device.

10. Laboratory apparatus according to claim 9, wherein the control device (103) is arranged to additionally check whether at least one further predetermined condition is fulfilled when logging on to the second user interface device, and the access rights for access to the laboratory apparatus via the second user interface device are only assigned if the at least one further predetermined condition is also fulfilled.

11. Laboratory apparatus according to claim 9 or 10, wherein the control device (103) is set up in such a way that, if a) or b) is fulfilled, information about the operating state of the laboratory apparatus, measured values or settings or programming of the laboratory apparatus which can be influenced by users is transmitted to the second user interface device via the interface device.

12. Laboratory apparatus according to any one of the preceding claims, comprising communication means for establishing a remote data connection for data exchange with an external device also comprising suitable communication means for establishing a remote data connection for data exchange with the laboratory apparatus.

13. Method (200) for controlling access to functions of a laboratory instrument (1; 400; 500) according to any one of claims 1 to 12, wherein the method provides for
a) establishing a plurality of first data connections with a data processing unit of one or more user interface devices (5; 7; 404; 504) via the first interface device (101);
b) establishing a second data connection with a data processing unit of the laboratory instrument via the second interface device (102); and
c) controlling authorizations and/or access rights for user access to laboratory instrument functions via the first and second data connections;
wherein the control device (103) is arranged and programmed to register and assign permissions and/or access rights to a first requesting user for a first session via a first data connection and via the first interface device (101), and
the control device (103) is set up and programmed to control, during this first session, the at least one request of at least one further user (201) to the access control, which request is made via a further first data connection and via the first interface device (101) and is directed towards the login, by a login process of the at least one further user, and to start a session for this further user, which session takes place at least temporarily in parallel with the first session of the first user.

## Revendications

1. Appareil de laboratoire (1 ; 400 ; 500) pour un traitement commandé par un appareil d'au moins un échantillon de laboratoire, lequel appareil de laboratoire présente :
- au moins un dispositif de traitement (3 ; 403 ; 503) pour mettre en œuvre le traitement du au moins un échantillon de laboratoire, et
- une commande d'accès (100) pour l'appareil de laboratoire,
- un logement (12 ; 401 ; 501) dans lequel le au moins un dispositif de traitement et la commande d'accès sont agencés ;
dans lequel la commande d'accès présente :
- un premier dispositif d'interface (101) et un second dispositif d'interface (102) ; et
- un dispositif de commande (103) ;
dans lequel le dispositif de commande (103) est configuré et programmé pour :
a) produire plusieurs premières liaisons de données pour l'échange de données avec une unité traitant des données d'un ou plusieurs dispositifs d'interface d'utilisateur (5 ; 7 ; 404 ; 504) via le premier dispositif d'interface (101) ;
b) produire une seconde liaison de données pour l'échange de données avec une unité traitant des données de l'appareil de laboratoire via le second dispositif d'interface (102) ; et
c) commander des autorisations et/ou droits d'accès pour l'accès d'utilisateurs via les premières et secondes liaisons de données à des fonctions de l'appareil de laboratoire ;
dans lequel
le dispositif de commande (103) est configuré et programmé pour annoncer à la commande d'accès, via la première liaison de données et via le premier dispositif d'interface (101), un premier utilisateur demandeur d'une première session à l'appareil de laboratoire, et lui attribuer des autorisations et/ou droits d'accès, et
le dispositif de commande (103) est configuré et programmé pour commander pendant cette première session la au moins une demande, se produisant via une autre première liaison de données et via le premier dispositif d'interface (101) et dirigée vers l'annonce, d'au moins un autre utilisateur à la commande d'accès par un processus d'annonce du au moins un autre utilisateur, et pour démarrer une session pour cet autre utilisateur qui a lieu au moins temporairement en parallèle de la première session du premier utilisateur.

2. Appareil de laboratoire selon la revendication 1, dans lequel le dispositif de commande (103) est configuré pour attribuer des autorisations et/ou droits d'accès à au moins un autre utilisateur demandeur, dans tous les cas après son annonce pendant la première session du premier utilisateur.

3. Appareil de laboratoire selon l'une des revendications précédentes, dans lequel le dispositif de commande (103) est configuré pour que les autorisations et/ou droits d'accès puissent être commandés de telle sorte qu'un accès simultané du premier et au moins de l'autre utilisateur se produise avec des autorisations et/ou droits d'accès à des fonctions de l'appareil de laboratoire attribués respectivement séparément.

4. Appareil de laboratoire selon l'une des revendications précédentes, dans lequel le dispositif de commande (103) est configuré pour que, lorsque le premier utilisateur a activé une ou plusieurs fonctions de l'appareil de laboratoire, les autorisations et/ou droits d'accès de chaque autre utilisateur annoncé soient établis de telle sorte que par une activation d'une fonction autorisée conformément aux autorisations et/ou droits d'accès de l'autre utilisateur l'exécution d'une fonction déjà activée de l'appareil de laboratoire ne puisse pas être manipulée.

5. Appareil de laboratoire selon l'une des revendications précédentes, dans lequel le dispositif de commande (103) est configuré pour que, en présence d'au moins une condition, un changement des autorisations et/ou droits d'accès puisse se produire de telle sorte que l'autre utilisateur obtienne à la place du premier utilisateur au moins partiellement les autorisations et/ou droits d'accès de celui-ci.

6. Appareil de laboratoire selon la revendication 5, dans lequel le dispositif de commande (103) est configuré pour que les autorisations et/ou droits d'accès que l'autre utilisateur obtient à la place du premier utilisateur contient le droit de commander le dispositif de traitement.

7. Appareil de laboratoire selon la revendication 6, dans lequel le dispositif de commande (103) est configuré pour que la fonction exécutée conformément aux autorisations et/ou droits d'accès du premier utilisateur contienne le traitement du au moins un échantillon par un dispositif de traitement de l'appareil de laboratoire.

8. Appareil de laboratoire selon l'une des revendications précédentes, dans lequel le dispositif de commande (103) est configuré pour que les autorisations et/ou droits d'accès du premier utilisateur ou de chaque autre utilisateur soient établis en fonction de l'état de fonctionnement de l'appareil de laboratoire.

9. Appareil de laboratoire selon les revendications précédentes, dans lequel le premier dispositif d'interface (101) permet l'annonce et l'accès à des fonctions de l'appareil de laboratoire via au moins deux dispositifs d'interface d'utilisateur (5 ; 7) différents et
dans lequel le dispositif de commande (103) est configuré pour qu'à l'annonce via un second dispositif d'interface d'utilisateur (7) soit vérifié si au préalable déjà, via un premier dispositif d'interface d'utilisateur (5), l'utilisateur demandeur
a) avait activé une ou plusieurs fonctions en cours d'exécution de l'appareil de laboratoire ou
b) est déjà annoncé, et
lorsque la condition a) ou b) est satisfaite, les autorisations et/ou droits d'accès attribués à l'utilisateur à l'annonce préalable via le premier dispositif d'interface d'utilisateur par la commande d'accès sont alors attribués via le second dispositif d'interface d'utilisateur pour l'accès à l'appareil de laboratoire.

10. Appareil de laboratoire selon la revendication 9, dans lequel le dispositif de commande (103) est configuré pour que soit de plus vérifié si au moins une autre condition prédéterminée est satisfaite à l'annonce au second dispositif d'interface d'utilisateur, et si les droits d'accès pour l'accès à l'appareil de laboratoire via le second dispositif d'interface d'utilisateur sont attribués uniquement à partir du moment où la au moins une autre condition prédéterminée est également satisfaite.

11. Appareil de laboratoire selon la revendication 9 ou 10, dans lequel le dispositif de commande (103) est configuré pour que, lorsque a) ou b) est satisfaite, des informations sur l'état de fonctionnement de l'appareil de laboratoire, des valeurs mesurées ou des réglages ou programmations de l'appareil de laboratoire manipulables par un utilisateur soient transmis via le dispositif d'interface au second dispositif d'interface d'utilisateur.

12. Appareil de laboratoire selon l'une des revendications précédentes, qui présente un dispositif de communication pour produire une liaison à distance de données pour l'échange de données avec un appareil extérieur, qui présente également un dispositif de communication approprié pour produire une liaison à distance pour l'échange de données avec l'appareil de laboratoire.

13. Procédé (200) destiné à commander l'accès à des fonctions d'un appareil de laboratoire (1 ; 400 ; 500) selon l'une des revendications 1 à 12, dans lequel le procédé prévoit de
a) produire plusieurs premières liaisons de données avec une unité traitant des données d'un ou plusieurs dispositifs d'interface d'utilisateur (5 ; 7 ; 404 ; 504) via le premier dispositif d'interface (101) ;
b) produire une seconde liaison de données avec une unité traitant des données de l'appareil de laboratoire via le second dispositif d'interface (102) ; et
c) commander des autorisations et/ou droits d'accès pour l'accès d'utilisateurs via les premières et secondes liaisons de données à des fonctions de l'appareil de laboratoire ;
dans lequel le dispositif de commande (103) est configuré et programmé pour annoncer, via une première liaison de données et via le premier dispositif d'interface (101), un premier utilisateur demandeur d'une première session, et lui attribuer des autorisations et/ou droits d'accès, et
le dispositif de commande (103) est configuré pour et programmé pour commander pendant cette première session la au moins une demande, se produisant via une autre première liaison de données et via le premier dispositif d'interface (101) et dirigée vers l'annonce, d'au moins un autre utilisateur (201) à la commande d'accès par un processus d'annonce du au moins un autre utilisateur, et pour démarrer une session pour cet autre utilisateur qui a lieu au moins temporairement en parallèle de la première session du premier utilisateur.
